(19) Europäisches Patentamt / European Patent Office / Office européen des brevets

(11) **EP 4 269 568 A1**

(12) **EUROPEAN PATENT APPLICATION**
published in accordance with Art. 153(4) EPC

(43) Date of publication:
**01.11.2023 Bulletin 2023/44**

(21) Application number: **21909520.5**

(22) Date of filing: **23.12.2021**

(51) International Patent Classification (IPC):
*C12N 5/0783* (2010.01)    *A61K 35/17* (2015.01)
*C12N 5/09* (2010.01)    *A61P 35/00* (2006.01)

(52) Cooperative Patent Classification (CPC):
**A61K 35/17; A61P 35/00; C12N 5/06**

(86) International application number:
**PCT/CN2021/140841**

(87) International publication number:
**WO 2022/135525 (30.06.2022 Gazette 2022/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.12.2020 CN 202011556353**

(71) Applicants:
• **Suzhou Grit Biotechnology Co., Ltd.**
 **Shanghai 201315 (CN)**
• **Shanghai Grit Biotechnology Co., Ltd.**
 **Shanghai 201315 (CN)**
• **Suzhou Tuoyu Biotechnology Co., Ltd.**
 **Shanghai 201315 (CN)**

• **Zhuhai Grit Biotechnology Co., Ltd.**
 **Shanghai 201315 (CN)**

(72) Inventors:
• **LIU, Yarong**
 **Shanghai 201315 (CN)**
• **ZHAO, Peipei**
 **Shanghai 201315 (CN)**
• **SHI, Zixiao**
 **Shanghai 201315 (CN)**

(74) Representative: **Jones Day**
 **Rechtsanwälte, Attorneys-at-Law, Patentanwälte**
 **Prinzregentenstrasse 11**
 **80538 München (DE)**

Remarks:
The complete document including Reference Table(s) and the Sequence Listing(s) can be downloaded from the EPO website

(54) **PREPARATION METHOD FOR TUMOR INFILTRATING LYMPHOCYTE AND USE THEREOF**

(57) Provided are a preparation method for a tumor-infiltrating lymphocyte, and use thereof, particularly relating to a method for culturing a tumor-infiltrating lymphocyte, comprising: subjecting a TIL, which is derived from tumor tissues and not expanded in vitro, to in vitro expansion of at least one stage, and wherein the TIL is contacted with a CD28 agonist in the in vitro expansion of at least one stage. Further provided is a method for preventing and/or treating tumors by using the tumor-infiltrating lymphocyte.

EP 4 269 568 A1

**Description**

**TECHNICAL FIELD**

The present application relates to the field of biomedicine, particularly to a preparation method for a tumor-infiltrating lymphocyte and use thereof.

**BACKGROUND OF THE INVENTION**

Treating tumors by using adoptive autologous transferred tumor-infiltrating lymphocytes is an effective approach for treating patients with poor prognoses. However, treating tumors by adoptive autologous transferred tumor-infiltrating lymphocytes requires a large number of tumor-infiltrating lymphocytes. Therefore, how to provide a robust and reliable method for culturing tumor-infiltrating lymphocytes is an urgent issue to be solved.

**SUMMARY OF THE INVENTION**

The present application provides a method for culturing tumor-infiltrating lymphocytes. The tumor-infiltrating lymphocytes obtained by the culture method of the present application have one or more of the effects selected from the group consisting of an increased number of TIL cells, an enhanced cytokine secretion ability, an enhanced tumor cell killing ability, an improved proportion of T cell subpopulations, an increased proportion of central memory T cells, a reduced proportion of regulatory T cells, an increased proportion of activated T cells, an increased proportion of tumor-specific T cells, an increased proportion of stem cell-like T cells, and an improved gene editing effect.

In one aspect, the present application provides a method for culturing tumor-infiltrating lymphocytes (TILs), wherein the method comprises subjecting TILs derived from tumor tissues and not expanded in vitro to at least one stage of in vitro expansion, wherein the TILs are contacted with a CD28 agonist in the at least one stage of in vitro expansion.

In one embodiment, the TILs derived from tumor tissues and not expanded in vitro are subjected to a first stage of in vitro expansion and a second stage of in vitro expansion, and the TILs subjected to the first stage of in vitro expansion are contacted with the CD28 agonist in the second stage of in vitro expansion.

In one embodiment, the CD28 agonist includes an anti-CD28 antibody and/or an antigen-binding fragment thereof.

In one embodiment, the second stage of in vitro expansion is carried out for at most about 13 days.

In one embodiment, the second stage of in vitro expansion is carried out for about 3 days to about 13 days.

In one embodiment, the culture method of the present application further includes co-culturing the TILs with feeder cells in at least one stage of the in vitro expansion.

In one embodiment, wherein, the TILs are contacted with the CD28 agonist and co-cultured with the feeder cells in a single stage of the in vitro expansion.

In one embodiment, wherein, the TILs are contacted with the CD28 agonist for a certain period and then co-cultured with the feeder cells in a single stage of the in vitro expansion.

In one embodiment, the certain period is at least about 2 hours.

In one embodiment, the certain period is about 6 hours to about 72 hours.

In one embodiment, the certain period is about 12 hours to about 48 hours.

In one embodiment, the certain period is about 6 hours, about 12 hours, about 24 hours, about 48 hours, or about 72 hours.

In one embodiment, the feeder cells include antigen-presenting cells.

In one embodiment, the feeder cells include one or more of the cells selected from the group consisting of peripheral mononuclear cells, dendritic cells, and artificial antigen-presenting cells.

In one embodiment, the feeder cells are peripheral mononuclear cells.

In one embodiment, the feeder cells are irradiated feeder cells.

In one embodiment, the co-culture of the TILs with the feeder cells includes contacting the surface of the feeder cells with the surface of the TILs.

In one embodiment, the co-culture of the TILs with the feeder cells includes adding the feeder cells into the cell culture medium of the TILs.

In one embodiment, the feeder cells are added into the cell culture medium of the TILs at a proportion of the feeder cells to the TILs from about 40: 1 to about 400:1.

In one embodiment, the culture method of the present application further includes contacting the TILs with one or more T cell growth factors in at least one stage of the in vitro expansion.

In one embodiment, wherein, the TILs are contacted with the CD28 agonist and with the one or more T cell growth factors in a single stage of the in vitro expansion.

In one embodiment, wherein, the TILs are contacted with the CD28 agonist and with the one or more T cell growth factors substantially at the same time in a single stage of the in vitro expansion.

In one embodiment, the T cell growth factors are one or more of the factors selected from the group consisting of IL-2, IL-7, IL-12, IL-15, IL-21, interferon gamma, and functionally active fragments thereof.

In one embodiment, the T cell growth factors include IL-2 and/or functionally active fragments thereof.

In one embodiment, the contact of the TILs with the one or more T cell growth factors includes adding the T cell growth factors into the cell culture medium of the TILs.

In one embodiment, the initial concentration of the T cell growth factors in the cell culture medium of the TILs is at least about 300 IU/mL.

In one embodiment, compared to the TILs that have not been contacted with the CD28 agonist in the in vitro expansion stage, the TILs that have been contacted with the CD28 agonist in at least one stage of the in vitro expansion show improved expansion effects.

In one embodiment, the improved expansion effects include one or more of the properties selected from the group consisting of an increased number of TIL cells, an improved proportion of T cell subpopulations, an enhanced cytokine secretion ability, and an enhanced tumor cell killing ability.

In one embodiment, the improved proportion of T cell subpopulations includes one or more of the properties selected from the group consisting of an increased proportion of central memory T cells, a reduced proportion of regulatory T cells, an increased proportion of activated T cells, an increased proportion of tumor-specific T cells, and an increased proportion of stem cell-like T cells.

In one embodiment, compared to the TILs that have not been contacted with the CD28 agonist in the in vitro expansion stage, the TILs that have been contacted with the CD28 agonist in at least one stage of the in vitro expansion shows an improved gene editing effect.

In one embodiment, the improved gene editing effect includes an enhanced gene knockout efficiency.

In one embodiment, the culture method of the present application further includes contacting the TILs with one or more other T cell activators other than the CD28 agonist in at least one stage of the in vitro expansion.

In one embodiment, wherein, the TILs are contacted with the CD28 agonist and with the one or more T cell activators in a single stage of the in vitro expansion.

In one embodiment, wherein, the TILs are contacted with the CD28 agonist and with the one or more other T cell activators substantially at the same time in a single stage of the in vitro expansion.

In one embodiment, the other T cell activators include one or more of the molecules selected from the group consisting of CD80, CD86, B7-H3, 4-1BBL, CD27, CD30, CD134, B7h, CD40, LIGHT, and functionally active fragments thereof.

In one embodiment, the other T cell activators include agonists of one or more of the targets selected from the group consisting of CD3, HVEM, CD40L, OX40, and 4-1BB.

In one embodiment, the other T cell activators include a CD3 agonist.

In one embodiment, the other T cell activators include an anti-CD3 antibody and/or an antigen-binding fragment thereof.

In one embodiment, the contact of the TILs with the CD28 agonist and with the one or more other T cell activators includes one or more of the means selected from the group consisting of (1) adding the CD28 agonist and the other T cell activators into the cell culture medium of the TILs; (2) adding engineering cells expressing the CD28 agonist and the other T cell activators into the cell culture medium of the TILs; (3) adding a solid-phase medium comprising the CD28 agonist and the other T cell activators into the cell culture medium of the TILs.

In one embodiment, the initial concentration of the other T cell activators in the cell culture medium of the TILs is at least about 30 ng/mL.

In one embodiment, the initial concentration of the other T cell activators in the cell culture medium of the TILs is about 30 ng/mL to about 300 ng/mL.

In one embodiment, the diameter of the solid-phase medium is about 500 nm to about 10 μm.

In one embodiment, the diameter of the solid-phase medium is about 1 nm to about 500 nm.

In one embodiment, the diameter of the solid-phase medium is measured by a transmission electron microscope.

In one embodiment, the solid-phase medium includes a polymer.

In one embodiment, the solid-phase medium includes at least about 25 μg of the CD28 agonist and the other T cell activators per mg.

In one embodiment, the solid-phase medium comprising the CD28 agonist and the other T cell activators is added into the cell culture medium of the TILs at a proportion of the solid-phase medium to the TILs from about 2:1 to about 1:2.

In one embodiment, the solid-phase medium comprising the CD28 agonist and the other T cell activators is added into the cell culture medium of the TILs at a proportion of the solid-phase medium to the TILs from about 1: 100 to about 1:2000.

In one embodiment, the TILs derived from tumor tissues and not expanded in vitro are TILs derived from fragments of the tumor tissues.

In one embodiment, the fragments have a volume of about 1 mm$^3$ to about 27 mm$^3$.

In one aspect, the present application provides a method for culturing tumor-infiltrating lymphocytes (TILs), wherein the method comprises:

(A) contacting a first TIL population derived from tumor tissues and not expanded in vitro with one or more T cell growth factors; wherein, a second TIL cell population is obtained through the step (A);

(B) contacting the second TIL population with a CD28 agonist.

In one embodiment, the CD28 agonist includes an anti-CD28 antibody and/or an antigen-binding fragment thereof.

In one embodiment, the step (B) is carried out for at most about 13 days.

In one embodiment, the step (B) is carried out for about 3 days to about 13 days.

In one embodiment, the culture method of the present application further includes contacting the TILs with feeder cells in the step (A) and/or the step (B).

In one embodiment, wherein, the second TIL population is co-cultured with the feeder cells in the step (B).

In one embodiment, wherein, in the step (B), the second TIL population is contacted with the CD28 agonist for a certain period, and then co-cultured with the feeder cells.

In one embodiment, the certain period is at least about 2 hours.

In one embodiment, the certain period is about 6 hours to about 72 hours.

In one embodiment, the certain period is about 12 hours to about 48 hours.

In one embodiment, the certain period is about 6 hours, about 12 hours, about 24 hours, about 48 hours, or about 72 hours.

In one embodiment, the feeder cells include antigen-presenting cells.

In one embodiment, the feeder cells include one or more of the cells selected from the group consisting of peripheral mononuclear cells, dendritic cells, and artificial antigen-presenting cells.

In one embodiment, the feeder cells are peripheral mononuclear cells.

In one embodiment, the feeder cells are irradiated feeder cells.

In one embodiment, the co-culture of the TILs with the feeder cells includes contacting the surface of the feeder cells with the surface of the TILs.

In one embodiment, the co-culture of the TILs with the feeder cells includes adding the feeder cells into the cell culture medium of the TILs.

In one embodiment, the feeder cells are added into the cell culture medium of the TILs at a proportion of the feeder cells to the TILs from about 40: 1 to about 400:1.

In one embodiment, the culture method of the present application further includes contacting the TILs with one or more T cell growth factors in the step (A) and/or the step (B).

In one embodiment, wherein, the second TIL population is contacted with the one or more T cell growth factors in the step (B).

In one embodiment, wherein, the second TIL population is contacted with the CD28 agonist and with the one or more T cell growth factors substantially at the same time in the step (B).

In one embodiment, the T cell growth factors are one or more of the factors selected from the group consisting of IL-2, IL-7, IL-12, IL-15, IL-21, interferon gamma, and functionally active fragments thereof.

In one embodiment, the T cell growth factors include IL-2 and/or functionally active fragments thereof.

In one embodiment, the contact of the TILs with the one or more T cell growth factors includes adding the T cell growth factors into the cell culture medium of the TILs.

In one embodiment, the initial concentration of the T cell growth factors in the cell culture medium of the TILs is at least about 300 IU/mL.

In one embodiment, compared to the corresponding TILs that have not been contacted with the CD28 agonist in the step (B), the TILs that have been contacted with the CD28 agonist in the step (B) show improved expansion effects.

In one embodiment, the improved expansion effects include one or more of the properties selected from the group consisting of an increased number of TIL cells, an improved proportion of T cell subpopulations, an enhanced cytokine secretion ability, and an enhanced tumor cell killing ability.

In one embodiment, the improved proportion of T cell subpopulations includes one or more of the properties selected from the group consisting of an increased proportion of central memory T cells, a reduced proportion of regulatory T cells, an increased proportion of activated T cells, an increased proportion of tumor-specific T cells, and an increased proportion of stem cell-like T cells.

In one embodiment, compared to the corresponding TILs that have not been contacted with the CD28 agonist in the step (B), the TILs that have been contacted with the CD28 agonist in the step (B) show an improved gene editing effect.

In one embodiment, the improved gene editing effect includes an enhanced gene knockout efficiency.

In one embodiment, the culture method of the present application further includes contacting the TILs with one or more other T cell activators other than the CD28 agonist in the step (A) and/or the step (B).

In one embodiment, wherein, the second TIL population is contacted with the CD28 agonist and with the one or more other T cell activators in the step (B).

In one embodiment, wherein, the second TIL population is contacted with the CD28 agonist and with the one or more other T cell activators substantially at the same time in the step (B).

In one embodiment, the other T cell activators include one or more of the molecules selected from the group consisting of CD80, CD86, B7-H3, 4-1BBL, CD27, CD30, CD134, B7h, CD40, LIGHT, and functionally active fragments thereof.

In one embodiment, the other T cell activators include agonists of one or more of the targets selected from the group consisting of CD3, HVEM, CD40L, OX40, and 4-1BB.

In one embodiment, the other T cell activators include a CD3 agonist.

In one embodiment, the other T cell activators include an anti-CD3 antibody and/or an antigen-binding fragment thereof.

In one embodiment, the contact of the TILs with the CD28 agonist and with the one or more other T cell activators includes one or more of the means selected from the group consisting of (1) adding the CD28 agonist and the other T cell activators into the cell culture medium of the TILs; (2) adding engineering cells expressing the CD28 agonist and the other T cell activators into the cell culture medium of the TILs; (3) adding a solid-phase medium comprising the CD28 agonist and the other T cell activators into the cell culture medium of the TILs.

In one embodiment, the initial concentration of the other T cell activators in the cell culture medium of the TILs is at least about 30 ng/mL.

In one embodiment, the initial concentration of the other T cell activators in the cell culture medium of the TILs is about 30 ng/mL to about 300 ng/mL.

In one embodiment, the diameter of the solid-phase medium is about 500 nm to about 10 $\mu$m.

In one embodiment, the diameter of the solid-phase medium is about 1 nm to about 500 nm.

In one embodiment, the diameter of the solid-phase medium is measured by a transmission electron microscope.

In one embodiment, the solid-phase medium includes a polymer.

In one embodiment, the solid-phase medium includes at least about 25 $\mu$g of the CD28 agonist and the other T cell activators per mg.

In one embodiment, the solid-phase medium comprising the CD28 agonist and the other T cell activators is added into the cell culture medium of the TILs at a proportion of the solid-phase medium to the TILs from about 2:1 to about 1:2.

In one embodiment, the solid-phase medium comprising the CD28 agonist and the other T cell activators is added into the cell culture medium of the TILs at a proportion of the solid-phase medium to the TILs from about 1: 100 to about 1:2000.

In one embodiment, the TILs derived from tumor tissues and not expanded in vitro are TILs derived from fragments of the tumor tissues.

In one embodiment, the fragments have a volume of about 1 mm$^3$ to about 27 mm$^3$.

In one aspect, the present application provides a tumor-infiltrating lymphocyte (TIL), which is obtainable by the method of the present application.

In one aspect, the present application provides a composition comprising the TIL of the present application.

In one aspect, the present application provides a pharmaceutical composition comprising the TIL of the present application and/or the composition of the present application, and optionally a pharmaceutically acceptable carrier.

In one aspect, the present application provides a method for affecting tumor cell growth, wherein the method comprises administering to a subject the TIL of the present application and/or the pharmaceutical composition of the present application.

In one aspect, the present application provides use of the TIL of the present application and/or the pharmaceutical composition of the present application for the manufacture of a medicament for preventing and/or treating tumors.

In one embodiment, the tumors are solid tumors.

In one embodiment, the tumors are one or more of the tumors selected from the group consisting of melanoma, ovarian cancer, cervical cancer, lung cancer, bladder cancer, breast cancer, head and neck cancer, pancreatic cancer, liver cancer, stomach cancer, colorectal cancer, and kidney cancer.

Other aspects and advantages of the present application can be readily perceived by those skilled in the art from the following detailed description. In the following detailed description, only exemplary embodiments of the present application are shown and described. As will be recognized by those skilled in the art, the content of the present application enables those skilled in the art to make changes to the disclosed specific embodiments without departing from the spirit and scope of the invention involved in the present application. Correspondingly, the drawings and descriptions in the specification of the present application are merely exemplary, rather than restrictive.

## BRIEF DESCRIPTION OF THE DRAWING

The specific features of the invention involved in the present application are as shown in the appended claims. The characteristics and advantages of the invention involved in the present application can be better understood by referring to the exemplary embodiments described in detail below and the accompanying drawings. A brief description of the drawings is as below:

FIG. 1 shows the results of the proliferative ability of the TILs obtained from the second stage of in vitro expansion of a mixed antibody group with the CD28 antibody added and a control group without the CD28 antibody added for donor A.

FIG. 2 shows the results of the proportion of T cell subpopulations of the TIL cells obtained from the second stage of in vitro expansion of a mixed antibody group with the CD28 antibody added and a control group without the CD28 antibody added for donor B-1.

FIG. 3 shows the results of the proportion of T cell subpopulations of the TIL cells obtained from the second stage of in vitro expansion of a mixed antibody group with the CD28 antibody added and a control group without the CD28 antibody added for donor B-2.

FIG. 4 shows the results of the proportion of T cell subpopulations of the TIL cells obtained from the second stage of in vitro expansion of a magnetic bead group with the CD28 antibody added and a control group without the CD28 antibody added for donor C-1.

FIG. 5 shows the results of the proportion of T cell subpopulations of the TIL cells obtained from the second stage of in vitro expansion of a magnetic bead group with the CD28 antibody added and a control group without the CD28 antibody added for donor C-2.

FIG. 6 shows the results of the proportion of T cell subpopulations of the TIL cells obtained from the second stage of in vitro expansion of a nano-matrix group with the CD28 antibody added and a control group without the CD28 antibody added for donor D.

FIG. 7 shows the results of the cell killing ability of the TIL cells obtained from the second stage of in vitro expansion of a nano-matrix group with the CD28 antibody added and a control group without the CD28 antibody added for donor E.

FIG. 8 shows the test results of the intracellular factor expression of the TIL cells obtained from the culture of the second stage of in vitro expansion of a mixed antibody group with the CD28 antibody added and a control group without the CD28 antibody added for donor F.

FIG. 9 shows the test results of the intracellular factor expression of the TIL cells obtained from the culture of the second stage of in vitro expansion of a magnetic bead group with the CD28 antibody added and a control group without the CD28 antibody added for donor G-1.

FIG. 10 shows the test results of the intracellular factor expression of the TIL cells obtained from the culture of the second stage of in vitro expansion of a magnetic bead group with the CD28 antibody added and a control group without the CD28 antibody added for donor G-2.

FIG. 11 shows the test results of the intracellular factor expression of the TIL cells obtained from the culture of the second stage of in vitro expansion of a magnetic bead group with the CD28 antibody added and a control group without the CD28 antibody added for donor G-3.

FIG. 12 shows the test results of the intracellular factor expression of the TIL cells obtained from the culture of the second stage of in vitro expansion of a magnetic bead group with the CD28 antibody added and a control group without the CD28 antibody added for donor G-4.

FIG. 13 shows the test results of the intracellular factor expression of the TIL cells obtained from the culture of the second stage of in vitro expansion of a nano-matrix group with the CD28 antibody added and a control group without the CD28 antibody added for donor H.

FIG. 14 shows the test results of the cytokine secretion of the TIL cells obtained from the culture of the second stage of in vitro expansion of a nano-matrix group with the CD28 antibody added and a control group without the CD28 antibody added for donor 1-1.

FIG. 15 shows the test results of the cytokine secretion of the TIL cells obtained from the culture of the second stage of in vitro expansion of a nano-matrix group with the CD28 antibody added and a control group without the CD28 antibody added, after co-incubation with tumor cells, for donor I-2.

FIG. 16 shows the results of the gene knockout efficiency of the TIL cells obtained from the culture of the second stage of in vitro expansion of a nano-matrix group with the CD28 antibody added and a control group without the CD28 antibody added, after co-incubation with tumor cells, for donor J-1.

FIG. 17 shows the results of the gene knockout efficiency of the TIL cells obtained from the culture of the second stage of in vitro expansion of a nano-matrix group with the CD28 antibody added and a control group without the CD28 antibody added, after co-incubation with tumor cells, for donor J-2.

FIG. 18 shows the results of the proliferative ability of the TILs obtained with the CD28 agonist added in the third stage of in vitro expansion compared to that of the TILs obtained without the CD28 agonist added in the third stage of expansion for donors K-1, K-2, and K-3.

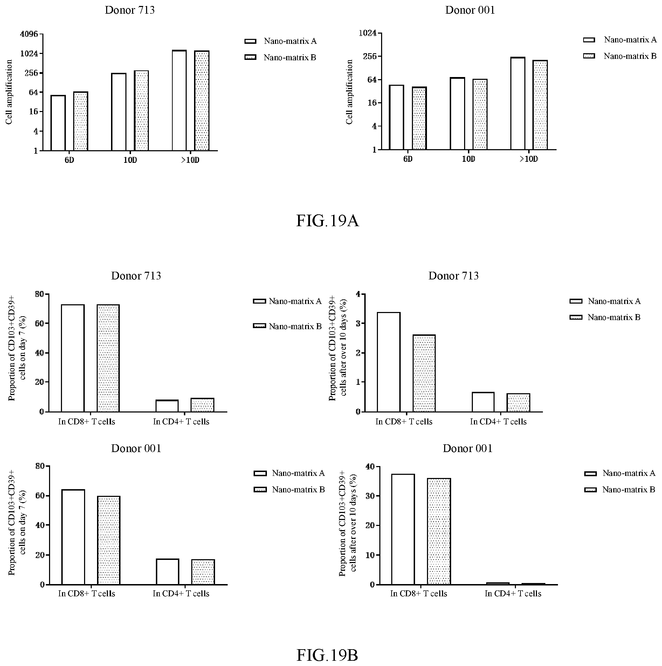

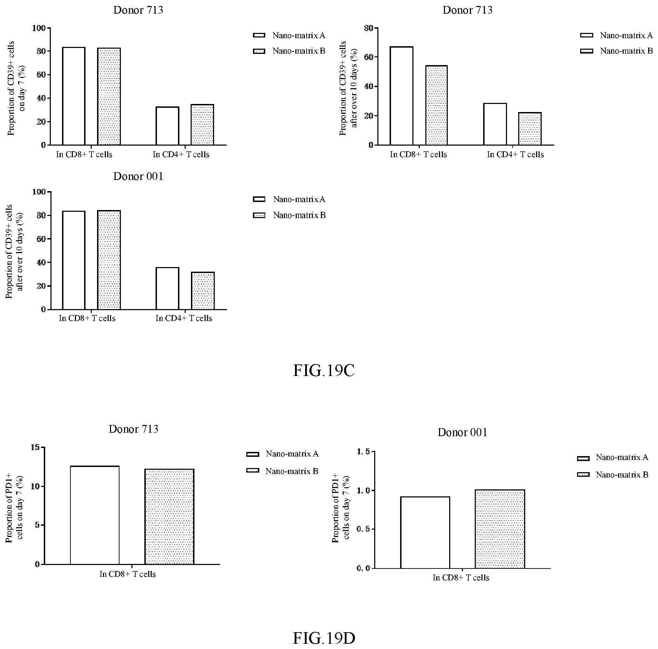

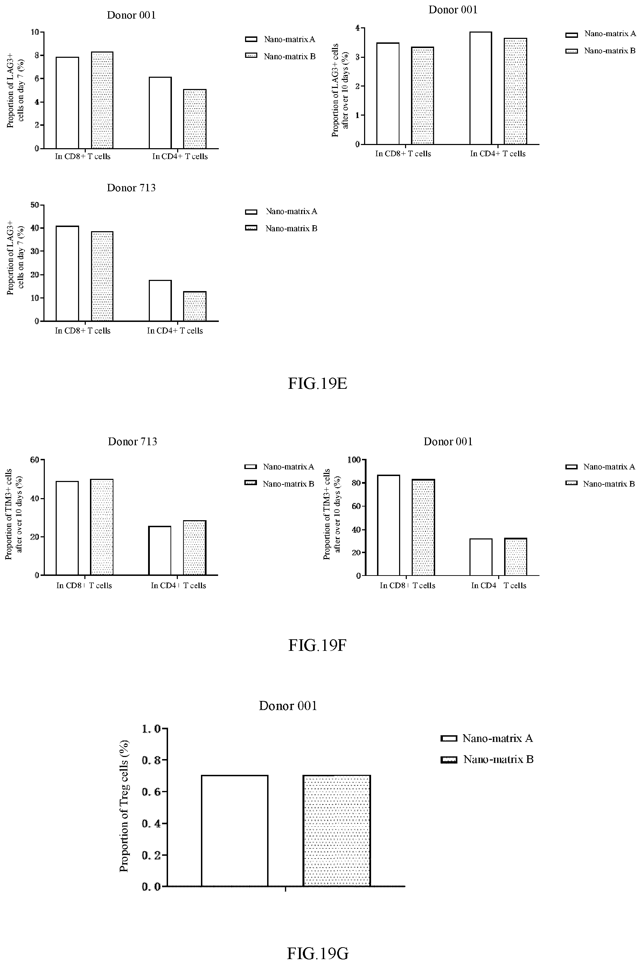

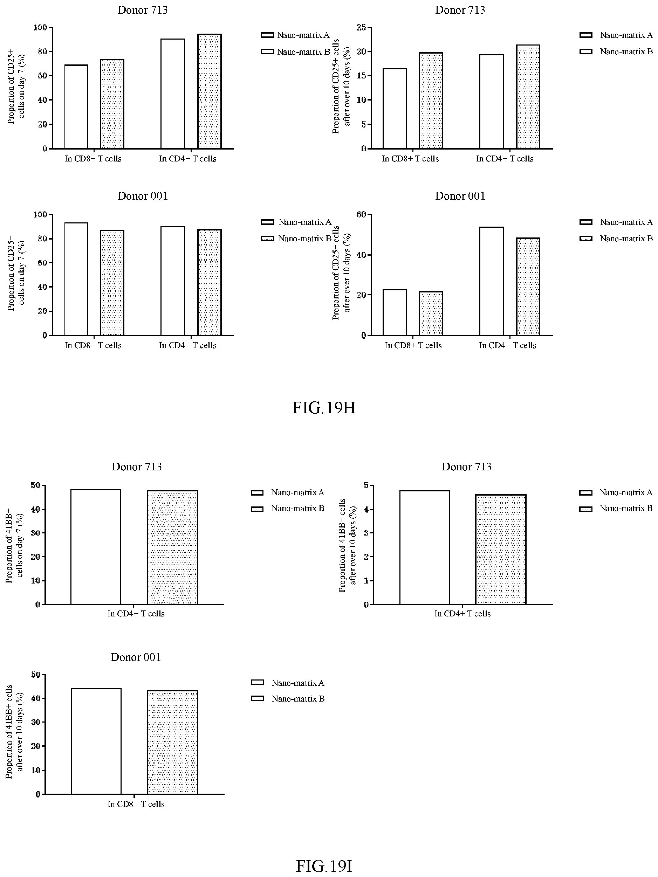

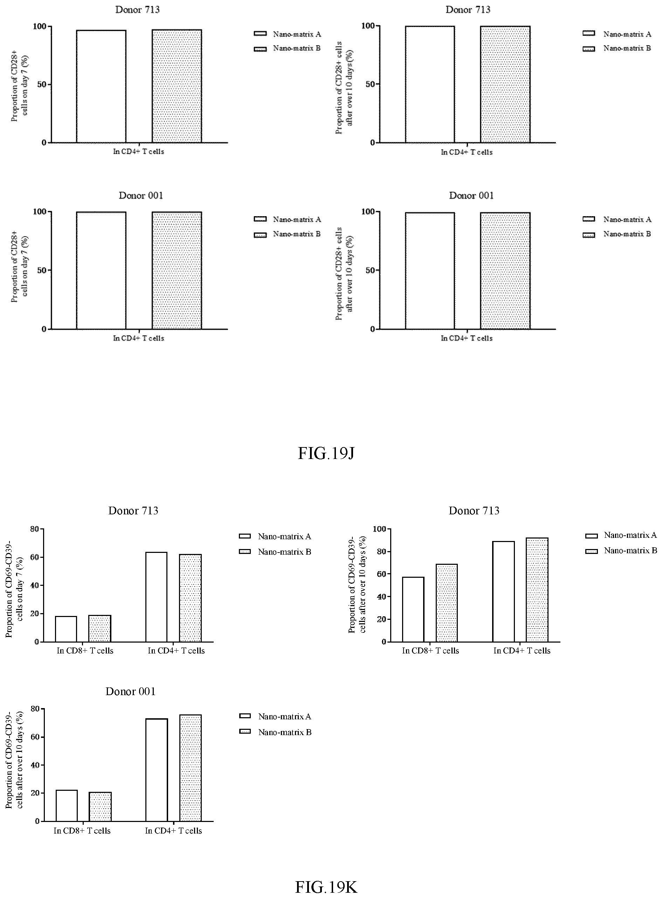

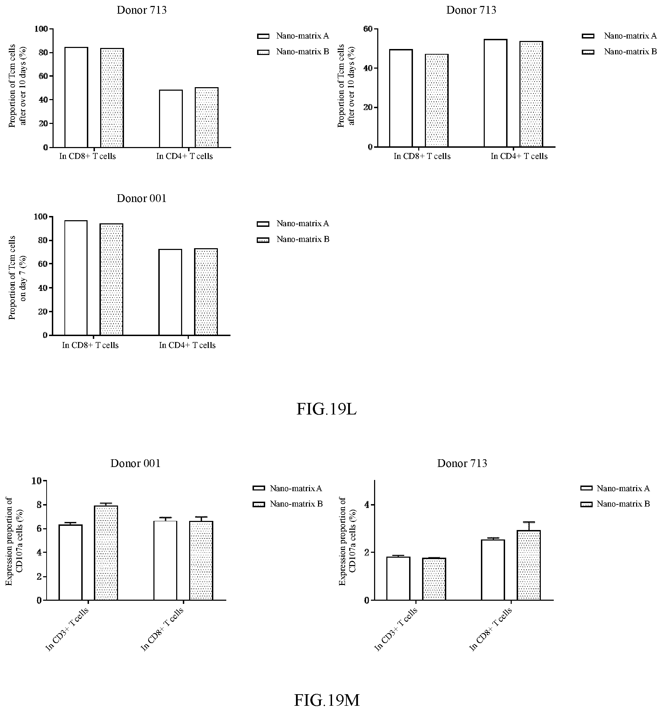

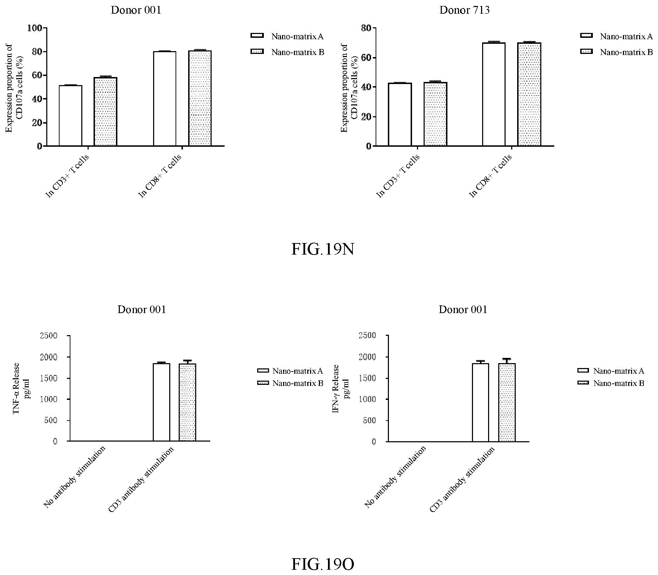

FIG. 19A shows the proliferative ability of the TILs in the experimental groups added with different forms of CD28 agonists. FIG. 19B shows the proportion of tumor-specific T cells ($CD103^+CD39^+$) in the TILs in the experimental groups added with different forms of nano-sized CD28 agonists. FIGs. 19C-19F show the proportion of exhausted T cells ($CD39^+$, $PD1^+$, $LAG3^+$, $TIM3^+$) in the TILs in the experimental groups added with different forms of nano-sized CD28 agonists. FIG. 19G shows the proportion of regulatory T cells (Treg) in the TILs in the experimental groups added with different forms of nano-sized CD28 agonists. FIGs. 19H-19J show the proportion of activated T cells ($CD25^+$, $41BB^+$, $CD28^+$) in the TILs in the experimental groups added with different forms of nano-sized CD28 agonists. FIG. 19K shows the proportion of stem cell-like T cells ($CD69^-CD39^-$) in the TILs in the experimental groups added with different forms of nano-sized CD28 agonists. FIG. 19L shows the proportion of central memory T cells (Tcm, e.g., $CD45RO^+CD62L^+$) in the TILs in the experimental groups added with different forms of nano-sized CD28 agonists. FIG. 19M shows the expression proportion of intracellular factors (CD107a) in the TILs in the experimental groups added with different forms of nano-sized CD28 agonists. FIG. 19N shows the expression proportion of intracellular factors (CD 107a) in the TILs in the experimental groups added with different forms of nano-sized CD28 agonists, in which each group of the TIL cells have been incubated with the CD3 antibody overnight before the flow cytometry. FIG. 19O shows the cytokine secretion ability of the TILs in the experimental groups added with different forms of nano-sized CD28 agonists, in which the TIL cells in the CD3 antibody stimulation group have been incubated with the CD3 antibody overnight before the cytokine detection.

## DETAILED DESCRIPTION

The implementation of the present application will be illustrated below by specific examples, and other advantages and effects of the present application will be easily known by those familiar with the art from the contents disclosed in the specification.

### Definition of Terms

In the present application, the term "expression" generally refers to the processes of transcription and/or translation of a gene encoding a polypeptide of interest occurring within a cell. The transcription level of a gene encoding a polypeptide of interest in a host cell can be determined by measuring the amount of the corresponding mRNA present in the cell. For example, quantitative measurement of an mRNA transcribed from a gene encoding a polypeptide of interest can be carried out by PCR or by RNA hybridization (see Sambrook et al., Molecular Cloning: A Laboratory Manual, Cold Spring Harbor Laboratory Press (1989)). The translation level of a gene encoding a polypeptide of interest can be measured by a variety of methods, e.g., ELISA, polypeptide biological activity assays, or, protein blotting or radioimmunoassay (see Sambrook et al., supra).

In the present application, the "stage" in the terms "one stage of in vitro expansion", "a single stage of in vitro expansion", or "the first stage of in vitro expansion", etc. generally refers to a process of expansion that TILs are subjected to in vitro. In one embodiment, each stage can be divided by the change in the number of the TIL cells. In one embodiment, when the number of the TIL cells is increased by at least about 1-fold, it can be considered that the TIL cells have entered the next stage of in vitro expansion. In some embodiments, when the number of the TIL cells is increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold, it can be considered that the TIL cells have entered the next stage of in vitro expansion. In one embodiment, each stage can also be divided by the culture conditions of the TIL cells. In one embodiment, after T cell activators and/or T cell growth factors are added or supplemented into the cell culture medium, it can be considered that the TIL cells have entered the next stage of in vitro expansion. In one embodiment, after TIL cells have been centrifuged and/or washed, it can be considered that the TIL cells have entered the next stage of in vitro expansion. In one embodiment, each stage can also be divided by the culture days of the TIL cells. In one embodiment, after the TIL cells have been cultured in vitro for about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 30 days, about 40 days, about 50 days or about 100 days, it can be considered that the TIL cells have entered the next stage of in vitro expansion.

In the present application, the term "the first stage of in vitro expansion" generally refers to a stage of expansion using T cell growth factors after primary TILs are obtained from tissues. In one embodiment, the tissues of the present application can be selected from the group consisting of tumor tissues and pleural effusion, and the pleural effusion of the present application can be pleural effusion in patients with metastatic cancers. In one embodiment, the expansion of the present application can be autologous or allogeneic in vivo expansion, or can be in vitro expansion. The first stage of in vitro expansion in the present application can also be referred to as a preREP (pre-rapid expansion protocol) stage.

In the present application, the term "the second stage of in vitro expansion" generally refers to a stage of expansion again, after the tissue has been removed from a subject and expanded. In one embodiment, compared to the TILs subjected to the first stage of in vitro expansion, the number of the TIL cells subjected to the second stage of in vitro expansion in the present application is increased, e.g., can be increased by at least about 10-fold (or at least about 20, 30, 40, 50, 60, 70, 80, or 90-fold), or in one embodiment, the cell number can be increased by at least about 100-fold. In one embodiment, the second and the first stages of the in vitro expansion can be different in culture conditions, e.g., the culture materials added can be different. The second stage of in vitro expansion in the present application can also be referred to as a REP (rapid expansion protocol) stage.

In the present application, the term "in vivo" generally refers to an event that occurs in the body of a subject.

In the present application, the term "in vitro" generally refers to an event that occurs outside the body of a subject.

In the present application, the term "ex vivo" generally refers to an event that involves treatment or surgery on cells, tissues, and/or organs that have been removed from a subject. In one embodiment, the cells, tissues, and/or organs can be returned to the subject's body by a surgery or treatment method.

In the present application, the term "secretion" generally refers to a transfer of an expressed polypeptide or protein by a cell to the extracellular environment.

In the present application, the term "secretion ability" generally refers to an ability of a cell to express a polypeptide or protein and to transfer the polypeptide or protein of the present application to the extracellular environment.

In the present application, the term "irradiation" generally refers to the process of a substance using radiation. For example, in one embodiment, irradiation can refer to irradiating a substance with X-rays, alpha rays, beta rays, or gamma rays.

In the present application, the term "engineered cells" generally refers to cells that have been genetically modified by adding additional genetic material in the form of DNA or RNA to the total genetic material of the cells. In one embodiment, the engineered cells can be TILs genetically modified to express the T cell activators and/or the T cell growth factors of the present application.

In the present application, the term "co-culture" generally refers to culturing two or more different populations of cells with some degree of contact between them. The "contact" between the two or more different populations of cells in the present application, in one embodiment, can be by direct contact, i.e., the cells of one population are directly physically contacted with the cells of another population. Alternatively, in one embodiment, the contact can be indirect contact mediated by sharing the culture medium. The shared medium in the present application can contain metabolites produced and released by at least one population of the co-cultured cells, and be used to culture the cells of another population.

In the present application, the term "contact" generally means that two or more substances of different types are contacted together in any order, in any manner, and for any length of time. In one embodiment, the contact can be direct contact, for example, one or more feeder cells, T cell activators, and/or T cell growth factors can be added into the culture medium of the TIL cells; for example, a culture medium comprising one or more feeder cells, T cell activators and/or T cell growth factors can be added into and/or replace the culture medium of the TIL cells; for example, a culture medium comprising one or more feeder cells, T cell activators and/or T cell growth factors can be used for the culture of the TIL cells. In one embodiment, the contact can be indirect contact, for example, metabolites produced and released by feeder cells can be used to culture the TIL cells.

In the present application, the term "mixture" generally refers to a combination of two or more different substances. For example, the CD28 antibody or an antigen-binding fragment thereof and the CD3 antibody or an antigen-binding fragment thereof in the present application can be added into the cell culture medium as a mixture after being mixed.

In the present application, the terms "simultaneous contact", "concurrent contact", "contacting with...at the same time", "simultaneously" and "concurrently" generally refer to the administration of two or more substances to a subject and/or a cell such that the substances are present in the subject and/or the environment in which the cell is cultured at the same time. Simultaneous contact can include administration of different compositions at the same time, administration of different compositions at different times, or administration of a composition in which two or more active pharmaceutical ingredients are present. For example, "simultaneous contact" in the present application generally refers to contact substantially at the same time.

In the present application, the term "expansion" generally refers to a several-fold increase in the number of cells over a period. In one embodiment, the cell number can be increased by at least about 3-fold (or 4, 5, 6, 7, 8, or 9-fold). In one embodiment, the cell number can be increased by at least about 10-fold (or 20, 30, 40, 50, 60, 70, 80, or 90-fold). Alternatively, in one embodiment, the cell number can be increased by at least about 100-fold. In the present application, the term "expanded" generally means that the cells of the present application have been subjected to one or more of the expansions described above.

In the present application, the term "polymer" generally refers to a molecule composed of individual chemical moieties linked together, and the polymer moieties of the present application can be the same or different. In one embodiment, the term "polymer" can refer to individual chemical moieties linked tail to tail to form a linear molecule, as well as individual chemical moieties linked together in the form of branched (e.g., "multi-arm" or "star") structures. In one embodiment, a polymer can include, for example, polysaccharide, glucan, hydrogel, polyethylene glycol, or poloxamer. Poloxamer is a nonionic triblock copolymer with a central hydrophobic chain of polyoxypropylene (polypropylene oxide)) and two pendant hydrophilic chains of polyoxyethylene (poly(ethylene oxide)). The substances encompassed in the present application can be formulated with, or administered with, any polymers described herein or known in the art.

In the present application, the term "antibody" generally refers to an immunoglobulin reactive to a specified protein or peptide or a fragment thereof. Such antibodies include, but are not limited to, human antibodies, primatized antibodies, chimeric antibodies, monoclonal antibodies, monospecific antibodies, polyclonal antibodies, multispecific antibodies, nonspecific antibodies, bispecific antibodies, multispecific antibodies, humanized antibodies, synthetic antibodies, recombinant antibodies, hybrid antibodies, mutant antibodies, graft-conjugated antibodies (i.e., antibodies conjugated or fused to other proteins, radiolabels, cytotoxins), and antibodies produced in vitro. The antibody can be antibodies from any class, including but not limited to IgG, IgA, IgM, IgD, and IgE, and antibodies from any subclass (e.g., IgG1, IgG2, IgG3, and IgG4). The antibody can have a heavy chain constant region selected from, e.g., IgG1, IgG2, IgG3, or IgG4. The antibody can also have a light chain selected from, e.g., kappa ($\kappa$) or lambda ($\lambda$). The antibody of the present application can be derived from any species, including but not limited to mice, human, camel, llama, fish, shark, goat, rabbit, chicken, and cattle. The constant region of the antibody can be altered, e.g., mutated, to modify the properties of the antibody (e.g., to enhance or reduce one or more of the following: the Fc receptor binding, the antibody glycosylation, the number of cysteine residues, the effector cell function, or the complement function). In general, the antibody specifically binds to a predetermined antigen, e.g., an antigen associated with a disorder, e.g., an inflammatory, an immune, an autoimmune, a neurodegenerative, a metabolic, and/or a malignant disorder.

In the present application, the term "chimeric antibody" generally refers to an antibody formed by fusing the variable region of a murine antibody with the constant region of a human antibody, which can reduce the immune response induced by the murine antibody. To establish a chimeric antibody, a hybridoma that secretes a murine-specific monoclonal antibody can be established, and then the variable region gene can be cloned from the murine hybridoma cell, and the constant region gene of the human antibody can be cloned as needed. The murine variable region gene is linked to the human constant region gene to form a chimeric gene, which is then inserted into an expression vector, and the chimeric antibody molecule can be expressed in a eukaryotic system or a prokaryotic system.

In the present application, the term "humanized antibody", also referred to as CDR-grafted antibody, generally refers to an antibody produced by grafting murine CDR sequences into human antibody variable region frameworks, i.e., different types of human germline antibody framework sequences. It can overcome the heterologous reaction induced by chimeric antibodies due to carrying a large number of murine protein components. Such framework sequences can be obtained from public DNA databases or published references that include germline antibody gene sequences. For example, the germline DNA sequences of human heavy chain and light chain variable region genes can be found in the "VBase" human germline sequence database.

In the present application, the term "fully humanized antibody", "full human antibody" or "completely humanized antibody", also referred to as "fully humanized monoclonal antibody", means that both the variable and constant regions of the antibody can be of human origin, with the immunogenicity and toxic side effects removed. The development of monoclonal antibodies has gone through four stages, namely: murine monoclonal antibodies, chimeric monoclonal antibodies, humanized monoclonal antibodies, and fully humanized monoclonal antibodies. The antibodies or ligands of the present application can be fully humanized monoclonal antibodies. Related technologies for the preparation of fully human antibodies can be: human hybridoma technology, EBV transformed B lymphocyte technology, phage display technology, transgenic mouse antibody preparation technology, and single B cell antibody preparation technology.

In the present application, the term "CDR" generally refers to one of the 6 hypervariable regions within the variable domain of an antibody that primarily contributes to antigen-binding. One of the most commonly used definitions of the 6 CDRs is provided by Kabat E.A. et al., (1991) Sequences of proteins of immunological interest. NIH Publication 91-3242), Chothia et al., "Canonical Structures For the Hypervariable Regions of Immunoglobulins," J. Mol. Biol. 196: 901 (1987); and MacCallum et al., "Antibody-Antigen Interactions: Contact Analysis and Binding Site Topography," J. Mol. Biol. 262: 732 (1996)). As used in the present application, the Kabat definition of CDRs can be applied to CDR1, CDR2, and CDR3 of a light chain variable domain (CDR L1, CDR L2, CDR L3 or L1, L2, L3) as well as CDR1, CDR2 and CDR3 of a heavy chain variable domain (CDR H1, CDR H2, CDR H3 or H1, H2, H3).

In the present application, the term "anti-CD3 antibody" generally refers to an antibody or variant thereof targeting CD3, e.g., a monoclonal antibody, including a human, humanized, chimeric or murine antibody, which is against the CD3 receptors in the T cell antigen receptors of mature T cells. The anti-CD3 antibody can include OKT-3. The anti-CD3 antibody can include SP34. The anti-CD3 antibody can also include other anti-CD3 antibodies including, for example, in one embodiment, otelixizumab, teplizumab, and visilizumab.

In the present application, the term "IL-2" or "IL2" generally refers to T cell growth factors known as interleukin 2 and includes all forms of IL-2, which can include, in one embodiment, human and mammalian forms, conservative amino acid substitutions, glycoform modifications or variants, or active fragments thereof. The GeneID of the gene encoding the IL-2 can be 3558.

In the present application, the term "antigen-presenting cell" or "APC" generally refers to an immune system cell that displays on its surface an exogenous antigen complexed with a major histocompatibility complex (MHC), such as a helper cell (e.g., a B cell, a dendritic cell, etc.). T cells can recognize these complexes using their T cell receptors (TCRs). APCs can process antigens and present them to T cells. In one embodiment, the antigen-presenting cells can be selected from the group consisting of peripheral mononuclear cells, dendritic cells, and artificial antigen-presenting cells.

In the present application, the term "expansion effect" generally refers to an effect that occurs after cells have been expanded. Changes in the expansion effect can include changes in the number and/or proportion of cells, changes in secretion ability, changes in killing ability, or changes in expression ability, or any combination thereof. The changes in the present application can be an enhancement or a reduction.

In the present application, the term "nanoparticle" generally refers to at least one microscopic particle having a size of less than 100 nm. In general, nanoparticles have diameters in the range of 50 nm to 500 nm (i.e., 0.05 $\mu$m to 0.5 $\mu$m); are structurally stable in physiological environments; and can accommodate smaller molecules (such as drugs or other bioactive agents), and then can deliver the molecules to desired sites. For example, the nanoparticles of the present application can include a CD28 antibody or an antigen-binding fragment thereof. For example, the nanoparticles of the present application can include the CD28 antibody or an antigen-binding fragment thereof and the CD3 antibody or an antigen-binding fragment thereof. The anti-CD28 antibody can include 15E8.

In the present application, the term "artificial antigen-presenting cell" generally refers to an artificially constructed immune cell for presenting an exogenous antigen, for example, the exogenous antigen can be presented by means of containing a complex of an exogenous antigen and a major histocompatibility complex (MHC) on the surface of the artificial antigen-presenting cell. In one embodiment, the isolated artificial antigen-presenting cells (aAPCs) can be included, which can comprise cells expressing HLA-A/B/C (the GeneID of the gene encoding it can be 3105, 3106 or 3107), CD64 (the GeneID of the gene encoding it can be 2209), CD80 (the GeneID of the gene encoding it can be 941), ICOS-L (the GeneID of the gene encoding it can be 23308) and CD58 (the GeneID of the gene encoding it can be 965), and can be modified to express more than one T cell activators, wherein the "more than" in the present application can include the numbers themselves.

In the present application, the term "fusion protein" generally refers to a polypeptide or protein containing the amino acid sequence of a first polypeptide or protein or fragment, analog or derivative thereof, as well as the amino acid sequence of a heterologous polypeptide or protein (i.e., a second polypeptide or protein or fragment, analog or derivative thereof that is different from the first polypeptide or protein or fragment, analog or derivative thereof, or a fraction that is generally not the first polypeptide or protein or fragment, analog or derivative thereof). In some cases, the fusion protein can include a prophylactic or therapeutic medicament fused to a heterologous protein, polypeptide, or peptide. Wherein, the heterologous protein, polypeptide, or peptide may or may not be different types of prophylactic or therapeutic medicaments. For example, two different proteins, polypeptides or peptides with immunomodulatory activities can be fused to form a fusion protein. In some cases, compared to the activity of the original polypeptide or protein before being fused with the heterologous protein, polypeptide, or protein, the fusion protein may have retained or enhanced activity. For example, the fusion protein of the present application may be a fusion protein fused with the CD28 antibody or an antigen-binding fragment thereof and the CD3 antibody or an antigen-binding fragment thereof.

In the present application, the term "killing ability" generally refers to an ability achieved by killing target cells through contacting the cells of the present application with an effective amount of substances. In one embodiment, the substances of the present application can be TIL cells. The killing of the present application can include killing cells by autologous CDC, apoptosis, ADCC, and/or phagocytosis, alternatively, by promoting these mechanisms of other cells or substances, or by a combination of two or more of these mechanisms.

In the present application, the term "administration" generally refers to the delivery of a substance to a subject in need thereof by any route known in the art. Pharmaceutical carriers and preparations or compositions are also well known in the art. Administration routes can include intravenous, intramuscular, intradermal, subcutaneous, transdermal, mucosal, intratumoral, and/or mucosal.

In the present application, the term "kit" generally refers to two or more components packaged together in a container, receptacle, or other containers, one of which corresponds to the substance of the present application. For example, the TIL cells of the present application are included.

In the present application, the term "subject" generally refers to a cell or an animal, which can be a mammal such as a human, a non-human primate (ape, gibbon, gorilla, chimpanzee, orangutan, and macaque), a domestic animal (dog and cat), a farm animal (poultry such as chicken and duck, horse, cattle, goat, sheep, and pig) and a laboratory animal (mouse, rat, rabbit, and guinea pig). The human subject includes a fetal, a neonatal, an infant, an adolescent, and an adult subject. The subject includes an animal disease model, e.g., a tumor animal model, and other animal models known to those skilled in the art.

In the present application, the term "feeder" generally refers to a cultured cell that grows in vitro and secretes at least one factor into the culture medium and can be used to support the growth of another cell of interest in culture. In one embodiment, the feeder cells can include antigen-presenting cells.

In the present application, the term "specific binding" generally refers to an antibody that recognizes a specific antigen but does not substantially recognize or bind to other molecules in a sample. For example, if an antibody can specifically bind to the specific antigen of the present application derived from one species, the antibody of the present application can also specifically bind to the antigen of the present application or its homologous antigen derived from one or more other species. Such interspecies reactivities themselves may not alter the classification of the antibody as specific. In some cases, an antibody that specifically binds to an antigen can also bind to different allelic forms of the antigen.

In the present application, the term "entire culture process" generally refers to an entire process starting from the isolation of cells from tumor tissues isolated from a patient, through one or more expansions, and finally obtaining cells that can be administered to a subject.

In the present application, the term "cell culture medium" generally refers to a nutrient solution in which cells, for example, mammalian cells, are grown. The formulation of the cell culture medium is well known in the art. Typically, the cell culture medium includes buffers, salts, carbohydrates, amino acids, vitamins, and essential trace elements. The cell culture medium might or might not contain serum, peptone, and/or protein. The cell culture medium can be supplemented with additional components or increased concentration of components such as amino acids, salts, sugars, vitamins, hormones, growth factors, buffers, antibiotics, lipids, trace elements, etc., depending on the requirements of cells to be cultured and/or desired cell culture parameters.

In the present application, the term "pharmaceutical composition" or "pharmaceutical preparation" generally refers to a kind of preparation, and the preparation of the present application may allow the biological activity of the active ingredients to be effective and can be free of additional components that are unacceptably toxic to the subject to whom the preparation will be administered. Such preparations are sterile. "Pharmaceutically acceptable" excipients (carriers, additives) are those that can be reasonably administered to a subject mammal to provide an effective dose of the active ingredient used.

In the present application, the term "tumor-infiltrating lymphocyte" or "TIL" generally refers to a population of cells initially obtained as leukocytes, and the cells of the present application have left the bloodstream of a subject and migrated into a tumor. The TILs can include, but are not limited to, CD8$^+$ cytotoxic T cells (lymphocytes), Th1 and Th17CD4$^+$T cells, natural killer cells, dendritic cells, and M1 macrophages. The TILs can include primary TILs and secondary TILs. The "primary TILs" can be those TIL cells obtained from a tissue sample of a subject. The "secondary TILs" can be any TIL cell population that has been expanded or expanded in the present application. In some embodiments, the tumor-infiltrating lymphocytes of the present application may not be isolated or purified or may be infiltrated with tumor cells. In one embodiment, the TILs of the present application can refer to a TIL cell population.

In the present application, the term "central memory T cell" generally refers to T cells that have long-term memory and can be re-stimulated by antigens. The central memory T cells can have phenotypes of CD45RA$^-$CCR7$^+$ and/or CD45RO$^+$CD62L$^+$. For example, the central memory T cells can be identified by CD45RA$^-$ and CCR7$^+$. For example, the central memory T cells can be identified by CD45RO$^+$ and CD62L$^+$. The central memory T cells can have stronger anti-tumor growth ability than ordinary T cells.

In the present application, the term "regulatory T cell" generally refers to a subpopulation of T cells that control autoimmune reactivities in the body. Regulatory T cells can have phenotypes of CD4$^+$CD25$^+$Foxp3$^+$, for example, the regulatory T cells can be identified by CD4$^+$, CD25$^+$, and Foxp3$^+$. The regulatory T cells can have an ability to suppress the anti-tumor growth of T cells.

In the present application, the term "activated T cell" generally refers to T cells that have been activated to have an ability to resist tumor growth. The activated T cells can have phenotypes of PD1$^+$, LAG3$^+$, or CD28$^+$, for example, the activated T cells can be identified by PD1$^+$, LAG3$^+$, or CD28$^+$. The activated T cells can have an ability to resist tumor growth.

In the present application, the term "tumor-specific T cell" generally refers to T cells that can specifically resist tumor growth. The tumor-specific T cells can have phenotypes of CD103$^+$CD39$^+$. For example, the tumor-specific T cells can be identified by CD103$^+$ and CD39$^+$. The tumor-specific T cells can have a more specific anti-tumor growth ability than ordinary T cells.

In the present application, the term "stem cell-like T cells" generally refers to a class of T cells that can have the potential to self-proliferate and/or differentiate. The stem cell-like T cells can have a phenotype of TCF1$^+$, for example, the stem cell-like T cells can be identified by TCF1$^+$. The tumor-specific T cells can have a stronger and/or longer-term anti-tumor growth ability than ordinary T cells.

In the present application, the term "tumor fragment" generally refers to tumor fragments that can be formed by mechanical disruption, enzymatic hydrolysis, and/or other disruption methods after a tumor tissue has been removed from a subject.

In the present application, the term "composition" or "pharmaceutical composition" generally refers to a mixture of at least one cell and at least one and optionally more than one other pharmaceutically acceptable chemical components such as carriers, stabilizers, diluents, dispersing agents, suspending agents, thickening agents and/or excipients.

In the present application, the term "pharmaceutically acceptable carrier" generally refers to one or more non-toxic materials that do not interfere with the active ingredients. For example, the pharmaceutically acceptable carriers may not interfere with the biological activity of the active ingredients; for example, the pharmaceutically acceptable carriers may not interfere with the effectiveness of the biological activity of the active ingredients. Such preparations can conventionally contain salts, buffers, preservatives, compatible carriers, and optionally other therapeutic agents. Such pharmaceutically acceptable preparations can also contain compatible solid or liquid fillers, diluents, or encapsulating substances suitable for administration to humans. Other contemplated carriers, excipients, and/or additives that can be used in the formulations described herein can include, for example, flavoring agents, antimicrobial agents, sweeteners, antioxidants, antistatic agents, lipids, protein excipients (such as serum albumin, gelatin, and casein), salt-forming counterions (such as sodium), and the like. These and other known pharmaceutical carriers, excipients, and/or additives suitable for use in the formulations described herein are known in the art.

In the present application, the term "functionally active fragment" generally refers to a fragment that has a partial region of a full-length protein or nucleic acid, but retains or partially retains the biological activity or function of the full-length protein or nucleic acid. For example, functionally active fragments can retain or partially retain the ability of the full-length protein to bind another molecule. For example, the functionally active fragments of the growth factor IL-2 can retain or partially retain the biologically active function of the full-length IL-2 that causes cell proliferation.

In the present application, the term "T cell activator" generally refers to a substance that binds to the corresponding binding receptor on a T cell and mediates a T cell co-stimulatory response. T cell activators can be substances other than antigen receptors that are required by T cells to generate an effective immune response. T cell activators can refer to T cell co-stimulatory molecules. T cell activators can include, but are not limited to, MHC class I molecules, TNF receptor proteins, immunoglobulin-like proteins, cytokine receptors, integrins, signaling lymphocyte activation molecules (SLAM proteins), NK cell activation receptors, BTLA (the GeneID of the gene encoding it can be 151888), Toll ligand receptors, OX40 (the GeneID of the gene encoding it can be 7293), CD2 (the GeneID of the gene encoding it can be 914), CD7 (the GeneID of the gene encoding it can be 924), CD27 (the GeneID of the gene encoding it can be 939), CD28 (the GeneID of the gene encoding it can be 940), CD30 (the GeneID of the gene encoding it can be 943), CD40 (the GeneID of the gene encoding it can be 958), CDS, ICAM-1 (the GeneID of the gene encoding it can be 3383), LFA-1 (CD11a/CD18) (the GeneID of the gene encoding it can be 3689), 4-1BB (CD137) (the GeneID of the gene encoding it can be 3604), B7-H3 (the GeneID of the gene encoding it can be 80381), ICOS (CD278) (the GeneID of the gene encoding it can be 29851), GITR (the GeneID of the gene encoding it can be 8784), BAFFR (the GeneID of the gene encoding it can be 115650), LIGHT (the GeneID of the gene encoding it can be 8740), HVEM (LIGHTR) (the GeneID of the gene encoding it can be 8764), KIRDS2, SLAMF7 (the GeneID of the gene encoding it can be 57823), NKp80 (KLRF1) (the GeneID of the gene encoding it can be 51348), NKp44 (the GeneID of the gene encoding it can be 9436), NKp30 (the GeneID of the gene encoding it can be 259197), NKp46 (the GeneID of the gene encoding it can be 9437), CD19 (the GeneID of the gene encoding it can be 930), CD4 (the GeneID of the gene encoding it can be 920), CD8$\alpha$ (the GeneID of the gene encoding it can be 925), CD8$\beta$ (the GeneID of the gene encoding it can be 926), IL-2R$\beta$, IL-2Ry, IL7R$\alpha$ (the GeneID of the gene encoding it can be ), ITGA4 (the GeneID of the gene encoding it can be 3676), VLA1 (the GeneID of the gene encoding it can be 3672), CD49a (the GeneID of the gene encoding it can be 3672), IA4 (the GeneID of the gene encoding it can be 3732), CD49D (the GeneID of the gene encoding it can be 3676), ITGA6 (the GeneID of the gene encoding it can be 3655), VLA-6 (the GeneID of the gene encoding it can be 3655), CD49f (the GeneID of the gene encoding it can be 3655), ITGAD (the GeneID of the gene encoding it can be 3681), CD11d (the GeneID of the gene encoding it can be 3681), ITGAE (the GeneID of the gene encoding it can be 3682), CD103 (the GeneID of the gene encoding it can be 3682), ITGAL (the GeneID of the gene encoding it can be 3683), CD11a (the GeneID of the gene encoding it can be 3683), LFA-1 (the GeneID of the gene encoding it can be 3683), ITGAM (the GeneID of the gene encoding it can be 3684), CD11b (the GeneID of the gene encoding it can be 3684), ITGAX (the GeneID of the gene encoding it can be 3687), CD11c (the GeneID of the gene encoding it can be 3687), ITGB1 (the GeneID of the gene encoding it can be 3688), CD29 (the GeneID of the gene encoding it can be 3688), ITGB2 (the GeneID of the gene encoding it can be 3689), CD18 (the GeneID of the gene encoding it can be 3689), LFA-1 (the GeneID of the gene encoding it can be 3689), ITGB7 (the GeneID of the gene encoding it can be 3695), NKG2D (the GeneID of the gene encoding it can be 22914), NKG2C (the GeneID of the gene encoding it can be 3822), TNFR2 (the GeneID of the gene encoding it can be 7133), TRANCE/RANKL (the GeneID of the gene encoding it can be 8600), DNAM1(CD226) (the GeneID of the gene encoding it can be 10666), SLAMF4 (CD244, 2B4) (the GeneID of the gene encoding it can be 51744), CD84 (the GeneID of the gene encoding it can be 8832), CD96 (Tactile) (the GeneID of the gene encoding it can be 10225), CEACAM1 (the GeneID of the gene encoding it can be 634), CRTAM (the GeneID of the gene encoding it can be 56253), Ly9 (CD229) (the GeneID of the gene encoding it can be 4063), CD160 (BY55) (the GeneID of the gene encoding it can be 11126), PSGL1 (the GeneID of the gene encoding it can be 6404), CD100 (SEMA4D) (the GeneID of the gene encoding it can be 10507), CD69 (the GeneID of the gene encoding it can be 969), SLAMF6 (NTB-A, Ly108) (the GeneID of the gene encoding it can be 114836), SLAM (SLAMF1, CD150, IPO-3) (the GeneID of the gene encoding it can be 6504), BLAME (SLAMF8) (the GeneID of the gene encoding it can be 56833), SELPLG (CD162) (the GeneID of the gene encoding it can be 6404), LTBR (the GeneID of the gene encoding it can be 4055), LAT (the GeneID of the gene encoding it can be 27040), GADS (the GeneID of the gene encoding it can be 9402), SLP-76 (the GeneID of the gene encoding it can be 3937), PAG/Cbp (the GeneID of the gene encoding it can be 55824), CD19a, and ligands that specifically bind to CD3, ligands that specifically bind to CD28, ligands that specifically bind to HVEM, ligands that specifically bind to CD40L, ligands that specifically bind to OX40, and ligands that specifically bind to 4-1BB. A co-stimulatory intracellular signaling domain can refer to the intracellular portion of a T cell activator. The intracellular signaling domain can include the entire intracellular portion of a molecule from which it is derived or the entire native intracellular signaling domain or a functional fragment thereof.

In the present application, the term "T cell growth factor" generally refers to a biologically active polypeptide or a small molecule compound that causes cell proliferation. In one embodiment, the T cell growth factor can be one or more of the factors selected from the group consisting of IL-2 (the GeneID of the gene encoding it can be 3558), IL-4 (the GeneID of the gene encoding it can be 3565), IL-7 (the GeneID of the gene encoding it can be 3574), IL-10 (the GeneID of the gene encoding it can be 3586), IL-12 (the GeneID of the gene encoding it can be 3592 or 3593), IL-15 (the GeneID of the gene encoding it can be 3600), and interferon gamma (the GeneID of the gene encoding it can be 3458).

In the present application, the term "substantially at the same time" generally means that TILs can be in contact with two or more substances simultaneously within a period during the contact process, but may not be limited to the fact that the TILs are always in contact with the two or more substances simultaneously during the entire contact process. In one embodiment, substantially at the same time can mean that the TILs can be in contact with at least 10%, 20%, 30%, 40%, 50%, 60%, 70%, 75%, 80%, 85%, 90%, and 95% of each of the two or more substances simultaneously within a period.

In the present application, the term "solid-phase medium" or "medium" generally refers to a solid material with a binding function. For example, the solid-phase medium of the present application may refer to a material that binds one or more substances within and/or on the surface of the medium through covalent and/or non-covalent binding. For example, the solid-phase medium of the present application can bind to one or more T cell activators. For example, the solid-phase medium of the present application may refer to a material that binds the CD28 antibody or an antigen-binding fragment thereof and the CD3 antibody or an antigen-binding fragment thereof within and/or on the surface of the medium through covalent and/or non-covalent binding. For example, the solid-phase medium of the present application can be microspheres comprising the OKT3 antibody and the 15E8 antibody with a diameter of about 500 nm to about 10 μm. For example, the solid-phase medium of the present application can be polymer materials. For example, the solid-phase medium of the present application can be microspheres with a diameter of at least about 500 nm. For example, the solid-phase medium of the present application can be nano-matrix. For example, the solid-phase medium of the present application can be nano-matrix comprising the OKT3 antibody and the 15E8 antibody with a diameter of about 1 nm to about 500 nm.

In the present application, the term "nano-matrix" generally refers to a material with a diameter of about 1 nm to about 500 nm. In the present application, the nano-matrix can have a binding function. For example, the nano-matrix of the present application can bind to one or more T cell activators. In the present application, the nano-matrix can include polymers. For example, the nano-matrix of the present application can include degradable polymers. In the present application, the nano-matrix can include polysaccharides and/or glucans.

In the present application, the term "gene editing" generally refers to a form of genetic engineering which inserts, replaces, or removes DNA from a target DNA (e.g., the TCRβ genome of a cell) using one or more nucleases and/or nickases.

In the present application, the term "gene knockout" generally refers to a genetic engineering means to silence a gene and/or make a gene unable to express the protein it encodes. For example, gene knockout can refer to a targeted disruption of a gene within a cell or in vivo that results in the complete loss of its function. For example, the gene knockout of the present application can be performed using site-specific nucleases. For example, the gene knockout of the present application can be performed using Zinc-finger nucleases (ZFNs), Transcription activator-like effector nucleases (TALENs), and/or a CRISPR/Cas-based system. For example, the gene knockout of the present application can be performed using a CRISPR/Cas9 system.

In the present application, the term "dendritic cell" generally refers to antigen-presenting cells that are present in vivo, in vitro, ex vivo, or within a host or subject, or can be derived from hematopoietic stem cells or monocytes. Dendritic cells and their precursors can be isolated from various lymphoid organs such as spleen and lymph nodes, as well as bone marrow and peripheral blood. The dendritic cells of the present application may have characteristic morphology, such as lamellae extending in multiple directions of the dendritic cell body (lamellipodia). In general, dendritic cells can express high levels of MHC and co-stimulatory (e.g., B7-1 and B7-2) molecules. Dendritic cells can induce antigen-specific differentiation of T cells in vitro and can trigger primary T cell responses in vitro and in vivo.

In the present application, the term "in vitro expansion" generally refers to culturing to produce changes in the number of cells, and the expanded cells can also produce changes in the number and/or proportion of cells, changes in the secretion ability, changes in the killing ability, or changes in the expression ability, or any combination thereof. The changes in the present application can be enhancement or reduction. In the present application, the in vitro expansion can be for expansion; the operation steps performed on TIL cells to detect the function of the TIL cells, for example, to detect the ability of the TIL cells to release cytokines (for example, adding one or more substances into the culture medium of the TIL cells to detect the ability of the TIL cells to release cytokines) may not belong to the in vitro expansion of the present application.

In the present application, the term "peripheral mononuclear cell" or "peripheral blood mononuclear cell" generally refers to cells with a single nucleus in peripheral blood. For example, in the present application, the peripheral blood mononuclear cells of the present application can include lymphocytes, monocytes, and/or dendritic cells.

In the present application, the term "cytokine" generally refers to proteins released by one cell population that act as intercellular regulators for another cell. The cytokines of the present application can be lymphokines, monokines, and polypeptide hormones. The cytokines of the present application can include interleukins (ILs) such as IL-1, IL-1α, IL-2, IL-3, IL-4, IL-5, IL-6, IL-7, IL-8, IL-9, IL-10, IL-11, IL-21, and/or IL-12. In the present application, the term "cytokine" can include proteins from natural sources or recombinant cell cultures, bioactive equivalents of natural sequence cytokines, and functionally active fragments thereof.

In the present application, the term "diameter" generally refers to the diameter of the cross-section of the substance of the present application. For example, when the substance of the present application is not spherical, the term "diameter" generally refers to the maximum diameter and/or average diameter of the largest cross-section of the substance of the present application. The method for determining the diameter of the substance may be a method commonly used in the art, such as transmission electron microscopy.

In the present application, the term "tumor" generally refers to any new pathological tissue proliferation. The tumor of the present application can be benign or malignant. The tumor of the present application can be solid or hematologic. The term "tumor" can be one or more of the tumors selected from the group consisting of melanoma, ovarian cancer, cervical cancer, lung cancer, bladder cancer, breast cancer, head and neck cancer, pancreatic cancer, liver cancer, stomach cancer, colorectal cancer, and kidney cancer.

In the present application, the term "tumor tissue" generally refers to a sample from any tissue of a tumor in a subject, including any solid tumor and/or non-solid tumor in the subject.

In the present application, the term "CD28 agonist" generally refers to a compound that binds to the CD28 protein on the cell surface and elicits a response in the cell. For example, the CD28 agonist of the present application can be a small molecule preparation that binds to CD28. For example, the CD28 agonist of the present application can be an antibody that binds to CD28 or an antigen-binding fragment thereof.

In the present application, the term "proportion of T cell subpopulation" generally refers to the proportions of different T cell subpopulations in TIL cells or TIL cell populations. For example, the different T cell subpopulations of the present application have different immunoreactivities and/or differentiation abilities. For example, the T cell subpopulations of the present application can be distinguished based on T cell surface markers. For example, central memory T cells can have phenotypes of CD45RA$^-$CCR7$^+$ and/or CD45RO$^+$CD62L$^+$ For example, regulatory T cells can have phenotypes of CD4$^+$CD25$^+$Foxp3$^+$. For example, activated T cells can have phenotypes of CD25$^+$, CD28$^+$, PD1$^+$ or 41BB$^+$. For example, tumor-specific T cells can have phenotypes of CD103$^+$CD39$^+$. For example, stem cell-like T cells can have a phenotype of TCF1$^+$.

In the present application, the term "TIL cell number" generally refers to the number of cells in the TIL cells of the present application. In the present application, the TIL cell number can refer to the number of cells in the TIL population obtained in any one stage of the present application. For example, the TIL cell number can refer to the number of cells in the first TIL population that is derived from tumor tissues and not expanded in vitro. For example, the TIL cell number can refer to the number of cells in the second TIL population that has been expanded in the first stage of in vitro expansion. For example, the TIL cell number can refer to the number of cells in the third TIL population that has been expanded in the second stage of in vitro expansion. For example, the TIL cell number can refer to the TIL cells finally obtained by any one culture method of the present application. In the present application, the TIL cell number can be measured by methods commonly used in the art, for example, including, but not limited to, manual cell counting with cytometer plates and/or counting with an automated cell counter.

In the present application, the terms "about" and "approximately" generally refer to within a statistically significant numerical range. Such a range can be within an order of magnitude of a given value or range, can be within 50%, can be within 20%, can be within 10%, can be within 5%. The permissible variation encompassed by the term "about" or "approximately" may depend on the particular system under study, and can be readily understood by one of ordinary skill in the art. The terms "more than", "less than", "at most", and "at least" can include the numbers themselves.

## Detailed Description

In one aspect, the present application provides a method for culturing tumor-infiltrating lymphocytes (TILs), which can include subjecting TILs derived from tumor tissues and not expanded in vitro to at least one stage of in vitro expansion, and wherein the TILs are contacted with a CD28 agonist in at least one stage of the in vitro expansion.

In another aspect, the present application provides a method for culturing tumor-infiltrating lymphocytes (TILs). The method can include (A) contacting a first TIL population derived from tumor tissues and not expanded in vitro with one or more T cell growth factors; wherein, a second TIL cell population is obtained through step (A); (B) contacting the second TIL population of the present application with a CD28 agonist.

In another aspect, the present application provides a method for culturing tumor-infiltrating lymphocytes (TILs). The method can include (A) contacting a first TIL population derived from tumor tissues and not expanded in vitro with one or more T cell growth factors; wherein, a second TIL cell population is obtained through step (A); (B) contacting the second TIL population of the present application with a CD28 agonist and co-culturing the second TIL population with feeder cells.

In another aspect, the present application provides a method for culturing tumor-infiltrating lymphocytes (TILs). The method can include (A) contacting a first TIL population derived from tumor tissues and not expanded in vitro with one or more T cell growth factors; wherein, a second TIL cell population is obtained through step (A); (B) contacting the second TIL population of the present application with a CD28 agonist and one or more T cell growth factors.

In another aspect, the present application provides a method for culturing tumor-infiltrating lymphocytes (TILs). The method can include (A) contacting a first TIL population derived from tumor tissues and not expanded in vitro with one or more T cell growth factors; wherein, a second TIL cell population is obtained through step (A); (B) contacting the second TIL population of the present application with a CD28 agonist and one or more T cell growth factors for a certain period, and then co-culturing with the feeder cells of the present application.

In the terms used in one embodiment, the first stage of in vitro expansion in the present application can be used optionally in place of step (A) in the method of the above aspects. In the terms used in one embodiment, the second stage of in vitro expansion in the present application can be used optionally in place of step (B) in the method of the above aspects. In the terms used in one embodiment, the TILs subjected to the first stage of in vitro expansion in the present application can be used optionally in place of the second TIL population obtained from step (A) in the method of the above aspects. In the terms used in one embodiment, the TILs subjected to the second stage of in vitro expansion in the present application can be used optionally in place of the third TIL population obtained from step (B) in the method of the above aspects. In the terms used in one embodiment, if necessary, the third stage of in vitro expansion in the present application can be used optionally in place of step (C) optionally added in the method of the above aspects. In the terms used in one embodiment, if necessary, the TILs subjected to the third stage of in vitro expansion in the present application can be used optionally in place of the fourth TIL population obtained from step (C) optionally added in the method of the above aspects.

In one embodiment, the TILs of the present application derived from tumor tissues and not expanded in vitro can be subjected to the first stage of in vitro expansion and the second stage of in vitro expansion, and in the second stage of in vitro expansion of the present application, the TILs subjected to the first stage of in vitro expansion of the present application are contacted with the CD28 agonist of the present application.

In one embodiment, the TILs of the present application derived from tumor tissues and not expanded in vitro are subjected to the first stage of in vitro expansion and the second stage of in vitro expansion, and in the first stage of in vitro expansion of the present application, the TILs of the present application derived from tumor tissues and not expanded in vitro are contacted with the CD28 agonist of the present application.

In one embodiment, the TILs of the present application derived from tumor tissues and not expanded in vitro are subjected to the first stage of in vitro expansion and the second stage of in vitro expansion, and in the first stage of in vitro expansion of the present application, the TILs of the present application derived from tumor tissues and not expanded in vitro are contacted with the CD28 agonist of the present application, and in the second stage of in vitro expansion of the present application, the TILs subjected to the first stage of in vitro expansion of the present application are contacted with the CD28 agonist of the present application.

In one embodiment, the TILs of the present application derived from tumor tissues and not expanded in vitro are subjected to the first stage of in vitro expansion, the second stage of in vitro expansion and the third stage of in vitro expansion, and in the first stage of in vitro expansion of the present application, the TILs of the present application derived from tumor tissues and not expanded in vitro are contacted with the CD28 agonist of the present application.

In one embodiment, the TILs of the present application derived from tumor tissues and not expanded in vitro can be subjected to the first stage of in vitro expansion, the second stage of in vitro expansion and the third stage of in vitro expansion, and in the second stage of in vitro expansion of the present application, the TILs subjected to the first stage of in vitro expansion of the present application are contacted with the CD28 agonist of the present application.

In one embodiment, the TILs of the present application derived from tumor tissues and not expanded in vitro can be subjected to the first stage of in vitro expansion, the second stage of in vitro expansion and the third stage of in vitro expansion, and in the third stage of in vitro expansion of the present application, the TILs subjected to the second stage of in vitro expansion of the present application are contacted with the CD28 agonist of the present application.

In one embodiment, the TILs of the present application derived from tumor tissues and not expanded in vitro are subjected to the first stage of in vitro expansion, the second stage of in vitro expansion and the third stage of in vitro expansion, and in the first stage of in vitro expansion of the present application, the TILs of the present application derived from tumor tissues and not expanded in vitro are contacted with the CD28 agonist of the present application, and in the second stage of in vitro expansion of the present application, the TILs subjected to the first stage of in vitro expansion of the present application are contacted with the CD28 agonist of the present application.

In one embodiment, the TILs of the present application derived from tumor tissues and not expanded in vitro are subjected to the first stage of in vitro expansion, the second stage of in vitro expansion and the third stage of in vitro expansion, and in the first stage of in vitro expansion of the present application, the TILs of the present application derived from tumor tissues and not expanded in vitro are contacted with the CD28 agonist of the present application, and in the third stage of in vitro expansion of the present application, the TILs subjected to the second stage of in vitro expansion of the present application are contacted with the CD28 agonist of the present application.

In one embodiment, the TILs of the present application derived from tumor tissues and not expanded in vitro are subjected to the first stage of in vitro expansion, the second stage of in vitro expansion and the third stage of in vitro expansion, and in the second stage of in vitro expansion of the present application, the TILs subjected to the first stage of in vitro expansion of the present application are contacted with the CD28 agonist of the present application, and in the third stage of in vitro expansion of the present application, the TILs subjected to the second stage of in vitro expansion of the present application are contacted with the CD28 agonist of the present application.

In one embodiment, the TILs of the present application derived from tumor tissues and not expanded in vitro are subjected to the first stage of in vitro expansion, the second stage of in vitro expansion and the third stage of in vitro expansion, and in the first stage of in vitro expansion of the present application, the TILs of the present application derived from tumor tissues and not expanded in vitro are contacted with the CD28 agonist of the present application, and in the second stage of in vitro expansion of the present application, the TILs subjected to the first stage of in vitro expansion of the present application are contacted with the CD28 agonist of the present application, and in the third stage of in vitro expansion of the present application, the TILs subjected to the second stage of in vitro expansion of the present application are contacted with the CD28 agonist of the present application.

In one embodiment, each stage of in vitro expansion can be divided by the change in the number of the TIL cells. In one embodiment, when the number of the TIL cells is increased by at least about 1-fold, it can be considered that the TIL cells have entered the next stage of in vitro expansion. In some embodiments, when the number of the TIL cells is increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, at least about 50-fold, at least about 100-fold, at least about 200-fold, at least about 500-fold, or at least about 1000-fold, it can be considered that the TIL cells have entered the next stage of in vitro expansion. In one embodiment, each stage of in vitro expansion can also be divided by the change in the culture conditions of the TIL cells. In one embodiment, after T cell activators and/or T cell growth factors are added or supplemented into the cell culture medium, it can be considered that the TIL cells have entered the next stage of in vitro expansion. For example, after IL-2 is added or supplemented into the cell culture medium, it can be considered that the TIL cells have entered the next stage of in vitro expansion. For example, after the CD28 agonist is added or supplemented into the cell culture medium, it can be considered that the TIL cells have entered the next stage of in vitro expansion. For example, after feeder cells are added or supplemented into the cell culture medium, it can be considered that the TIL cells have entered the next stage of in vitro expansion. In one embodiment, after TIL cells have been centrifuged and/or washed, it can be considered that the TIL cells have entered the next stage of the in vitro expansion. In one embodiment, each stage can also be divided by the culture days of the TIL cells. In one embodiment, after the TIL cells have been cultured in vitro for about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 30 days, about 40 days, about 50 days or about 100 days, it can be considered that the TIL cells have entered the next stage of in vitro expansion.

In one embodiment, the in vitro expansion of the present application can only include the complete culture process. In one embodiment, the in vitro expansion of the present application can refer to culturing to produce changes in the number of cells, and the expanded cells can also produce changes in the number and/or proportion of cells, changes in the secretion ability, changes in the killing ability, or changes in the expression ability, or any combination thereof. The changes here can be enhancement or reduction. In one embodiment, the in vitro expansion of the present application can be for the purpose of expansion; the operation steps performed on TIL cells to detect the function of the TIL cells, for example, to detect the ability of the TIL cells to release cytokines (for example, adding one or more substances into the culture medium of the TIL cells to detect the ability of the TIL cells to release cytokines) may not belong to the in vitro expansion of the present application. For example, in a single stage of the in vitro expansion of the present application, the TILs of the present application are contacted only with the CD 28 agonist, but not contact with TIL growth factors, which may not belong to the in vitro expansion of the present application. For example, in a single stage of the in vitro expansion of the present application, the TILs of the present application are contacted only with the CD 28 agonist, but not contact with IL-2, which may not belong to the in vitro expansion of the present application.

In one embodiment, the CD28 agonist of the present application may include an anti-CD28 antibody or an antigen-binding fragment thereof.

In one embodiment, the CD28 agonist of the present application may include an anti-CD28 antibody selected from the group consisting of a murine antibody, a humanized antibody, a fully humanized antibody, and a chimeric antibody.

In one embodiment, the CD28 agonist of the present application may include an antigen-binding fragment of the anti-CD28 antibody selected from the group consisting of Fab, Fab', $F(ab)_2$, and Fv fragments.

In one embodiment, the second stage of in vitro expansion of the present application can be carried out for at most about 13 days. In one embodiment, the number of days for the second stage of in vitro expansion of the present application can be calculated from the beginning moment of the second stage of in vitro expansion. For example, the moment when the second stage of in vitro expansion starts can be considered that the second stage of in vitro expansion has been carried out for about 0 days. For example, about 24 hours after the start of the second stage of in vitro expansion, it can be considered that the second stage of in vitro expansion has been carried out for about 1 day. For example, the day on which the second stage of in vitro expansion starts can be considered that the second stage of in vitro expansion has been carried out for about 0 days. In one embodiment, the number of days for the second stage of in vitro expansion of the present application can be calculated by the number of days for the second stage of in vitro expansion. For example, the next day after the start of the second stage of in vitro expansion can be considered that the second stage of in vitro expansion has been carried out for about 1 day. For example, the second stage of in vitro expansion of the present application can be carried out for at most about 13 days, at most about 12 days, at most about 11 days, at most about 10 days, at most about 9 days, at most about 8 days, at most about 7 days, at most about 6 days, at most about 5 days, at most about 4 days, at most about 3 days, at most about 2 days, or at most about 1 day. In one embodiment, the second stage of in vitro expansion of the present application can be carried out for about 3 days to about 13 days. In one embodiment, the second stage of in vitro expansion of the present application can be carried out for about 1 day to about 13 days. For example, the second stage of in vitro expansion of the present application can be carried out for about 2 days to about 13 days, about 3 days to about 13 days, about 4 days to about 13 days, about 5 days to about 13 days, about 6 days to about 13 days, about 7 days to about 13 days, about 8 days to about 13 days, about 9 days to about 13 days, about 10 days to about 13 days, about 11 days to about 13 days, or about 12 days to about 13 days. For example, the second stage of in vitro expansion of the present application can be carried out for about 2 days to about 3 days, about 2 days to about 4 days, about 2 days to about 5 days, about 2 days to about 6 days, about 2 days to about 7 days, about 2 days to about 8 days, about 2 days to about 9 days, about 2 days to about 10 days, about 2 days to about 11 days, about 2 days to about 12 days, or about 2 days to about 13 days. For example, the second stage of in vitro expansion of the present application can be carried out for about 3 days to about 4 days, about 3 days to about 5 days, about 3 days to about 6 days, about 3 days to about 7 days, about 3 days to about 8 days, about 3 days to about 9 days, about 3 days to about 10 days, about 3 days to about 11 days, about 3 days to about 12 days, or about 3 days to about 13 days. For example, the second stage of in vitro expansion of the present application can be carried out for about 13 days, about 12 days, about 11 days, about 10 days, about 9 days, about 8 days, about 7 days, about 6 days, about 5 days, about 4 days, about 3 days, about 2 days or about 1 day. In one embodiment, the second stage of in vitro expansion of the present application can be taken as the REP (rapid expansion protocol) stage. In one embodiment, the first stage of in vitro expansion in the present application can be taken as the preREP (pre-rapid expansion protocol) stage.

In one embodiment, the third stage of in vitro expansion of the present application can be carried out for at most about 13 days. In one embodiment, the number of days for the third stage of in vitro expansion of the present application can be calculated from the beginning of the third stage of in vitro expansion. For example, the moment when the third stage of in vitro expansion starts can be considered that the third stage of in vitro expansion has been carried out for about 0 days. For example, about 24 hours after the start of the third stage of in vitro expansion, it can be considered that the third stage of in vitro expansion has been carried out for about 1 day. For example, the day on which the third stage of in vitro expansion starts can be considered that the third stage of in vitro expansion has been carried out for about 0 days. For example, the next day after the start of the third stage of in vitro expansion can be considered that the third stage of in vitro expansion has been carried out for about 1 day. For example, the third stage of in vitro expansion of the present application can be carried out for at most about 13 days, at most about 12 days, at most about 11 days, at most about 10 days, at most about 9 days, at most about 8 days, at most about 7 days, at most about 6 days, at most about 5 days, at most about 4 days, at most about 3 days, at most about 2 days, or at most about 1 day. In one embodiment, the third stage of in vitro expansion of the present application can be carried out for about 3 days to about 13 days. In one embodiment, the third stage of in vitro expansion of the present application can be carried out for about 1 day to about 13 days. For example, the third stage of in vitro expansion of the present application can be carried out for about 2 days to about 13 days, about 3 days to about 13 days, about 4 days to about 13 days, about 5 days to about 13 days, about 6 days to about 13 days, about 7 days to about 13 days, about 8 days to about 13 days, about 9 days to about 13 days, about 10 days to about 13 days, about 11 days to about 13 days, or about 12 days to about 13 days. For example, the third stage of in vitro expansion of the present application can be carried out for about 2 days to about 3 days, about 2 days to about 4 days, about 2 days to about 5 days, about 2 days to about 6 days, about 2 days to about 7 days, about 2 days to about 8 days, about 2 days to about 9 days, about 2 days to about 10 days, about 2 days to about 11 days, about 2 days to about 12 days, or about 2 days to about 13 days. For example, the third stage of in vitro expansion of the present application can be carried out for about 3 days to about 4 days, about 3 days to about 5 days, about 3 days to about 6 days, about 3 days to about 7 days, about 3 days to about 8 days, about 3 days to about 9 days, about 3 days to about 10 days, about 3 days to about 11 days, about 3 days to about 12 days, or about 3 days to about 13 days. For example, the third stage of in vitro expansion of the present application can be carried out for about 13 days, about 12 days, about 11 days, about 10 days, about 9 days, about 8 days, about 7 days, about 6 days, about 5 days, about 4 days, about 3 days, about 2 days or about 1 day. In one embodiment, the second stage of in vitro expansion of the present application can be taken as the REP (rapid expansion protocol) stage. In one embodiment, the third stage of in vitro expansion in the present application can be taken as the reREP (re-rapid expansion protocol) stage.

In one embodiment, the method of the present application can further include coculturing the TILs of the present application with feeder cells in at least one stage of the in vitro expansion of the present application.

In one embodiment, in a single stage of in vitro expansion of the present application, the TILs of the present application can be contacted with the CD28 agonist of the present application and co-cultured with the feeder cells of the present application; in one embodiment, the single stage of in vitro expansion of the present application can refer to the same stage of in vitro expansion of the present application, for example, all in the first stage of in vitro expansion of the present application, all in the second stage of in vitro expansion of the present application, or all in the third stage of in vitro expansion of the present application, etc.

In one embodiment, in the first stage of in vitro expansion of the present application, the TILs of the present application can be contacted with the CD28 agonist of the present application and co-cultured with the feeder cells of the present application. In one embodiment, in the second stage of in vitro expansion of the present application, the TILs of the present application can be contacted with the CD28 agonist of the present application and co-cultured with the feeder cells of the present application. In one embodiment, in the third stage of in vitro expansion of the present application, the TILs of the present application can be contacted with the CD28 agonist of the present application and co-cultured with the feeder cells of the present application.

In one embodiment, in a single stage of in vitro expansion of the present application, the TILs of the present application can be contacted with the CD28 agonist of the present application for a certain period and then co-cultured with the feeder cells of the present application. In one embodiment, in the first stage of in vitro expansion of the present application, the TILs of the present application can be contacted with the CD28 agonist of the present application for a certain period and then co-cultured with the feeder cells of the present application. In one embodiment, in the second stage of in vitro expansion of the present application, the TILs of the present application can be contacted with the CD28 agonist of the present application for a certain period and then co-cultured with the feeder cells of the present application. In one embodiment, in the third stage of in vitro expansion of the present application, the TILs of the present application can be contacted with the CD28 agonist of the present application for a certain period and then co-cultured with the feeder cells of the present application.

In one embodiment, the certain period in the present application can be at least about 2 hours. In one embodiment, the certain period in the present application can be at least about 1 hour, at least about 2 hours, at least about 3 hours, at least about 4 hours, at least about 5 hours, at least about 6 hours, at least about 7 hours, at least about 8 hours, at least about 9 hours, at least about 10 hours, at least about 11 hours, at least about 12 hours, at least about 13 hours, at least about 14 hours, at least about 15 hours, at least about 16 hours, at least about 17 hours, at least about 18 hours, at least about 19 hours, at least about 20 hours, at least about 21 hours, at least about 22 hours, at least about 23 hours, at least about 24 hours, at least about 36 hours, at least about 48 hours, at least about 60 hours or at least about 72 hours. In one embodiment, the certain period in the present application can be about 6 hours to about 72 hours. In one embodiment, the certain period in the present application can be about 6 hours to about 7 hours, about 6 hours to about 8 hours, about 6 hours to about 9 hours, about 6 hours to about 10 hours, about 6 hours to about 11 hours, about 6 hours to about 12 hours, about 6 hours to about 13 hours, about 6 hours to about 14 hours, about 6 hours to about 15 hours, about 6 hours to about 16 hours, about 6 hours to about 17 hours, about 6 hours to about 18 hours, about 6 hours to about 19 hours, about 6 hours to about 20 hours, about 6 hours to about 21 hours, about 6 hours to about 22 hours, about 6 hours to about 23 hours, about 6 hours to about 24 hours, about 6 hours to about 36 hours, about 6 hours to about 48 hours, about 6 hours to about 60 hours, or about 6 hours to about 72 hours. In one embodiment, the certain period in the present application can be about 12 hours to about 13 hours, about 12 hours to about 14 hours, about 12 hours to about 15 hours, about 12 hours to about 16 hours, about 12 hours to about 17 hours, about 12 hours to about 18 hours, about 12 hours to about 19 hours, about 12 hours to about 20 hours, about 12 hours to about 21 hours, about 12 hours to about 22 hours, about 12 hours to about 23 hours, about 12 hours to about 24 hours, about 12 hours to about 36 hours, about 12 hours to about 48 hours, about 12 hours to about 60 hours, or about 12 hours to about 72 hours. In one embodiment, the certain period in the present application can be about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about 24 hours, about 36 hours, about 48 hours, about 60 hours, or about 72 hours.

In one embodiment, the feeder cells of the present application can include antigen-presenting cells. In one embodiment, the feeder cells of the present application can include one or more of the cells selected from the group consisting of peripheral mononuclear cells, dendritic cells, and artificial antigen-presenting cells. In one embodiment, the feeder cells of the present application can be peripheral mononuclear cells. In one embodiment, the feeder cells of the present application can be irradiated feeder cells. For example, the feeder cells of the present application can be isolated artificial antigen-presenting cells (aAPCs). The artificial antigen-presenting cells of the present application can include cells expressing HLA-A/B/C, CD64, CD80, ICOS-L, and/or CD58, and can be modified to express more than one T cell activators of the present application. In one embodiment, the feeder cells of the present application can be irradiated, for example, they can be irradiated by gamma rays or X rays.

In one embodiment, the co-culture of the TILs of the present application with the feeder cells of the present application can include contacting the surfaces of the feeder cells of the present application with the surfaces of the TILs of the present application. In one embodiment, the co-culture of the TILs of the present application with the feeder cells of the present application includes adding the feeder cells of the present application into the cell culture medium of the TILs of the present application.

In one embodiment, the feeder cells of the present application can be added into the cell culture medium of the TILs of the present application at a proportion of the feeder cells of the present application to the TILs of the present application from about 40:1 to about 400:1. In one embodiment, the feeder cells of the present application can be added into the cell culture medium of the TILs of the present application at a proportion of the feeder cells of the present application to the TILs of the present application from about 40:1 to about 400:1, from about 40:1 to about 300:1, from about 40:1 to about 200:1, from about 40:1 to about 100:1, from about 40:1 to about 90:1, from about 40:1 to about 80:1, from about 40:1 to about 70:1, from about 40:1 to about 60:1, from about 40:1 to about 50:1, from about 50:1 to about 400:1, from about 60:1 to about 400:1, from about 70:1 to about 400:1, from about 80:1 to about 400:1, from about 90:1 to about 400:1, from about 100:1 to about 400:1, from about 200:1 to about 400:1, or from about 300:1 to about 400:1.

In one embodiment, the method of the present application can further include contacting the TILs of the present application with one or more T cell growth factors in at least one stage of in vitro expansion of the present application.

In one embodiment, in a single stage of in vitro expansion of the present application, the TILs of the present application can be contacted with the CD28 agonist of the present application and with one or more T cell growth factors of the present application. For example, in the first stage of in vitro expansion of the present application, the TILs of the present application can be contacted with the CD28 agonist of the present application and contact with the one or more T cell growth factors of the present application. For example, in the second stage of in vitro expansion of the present application, the TILs of the present application can be contacted with the CD28 agonist of the present application and contact with the one or more T cell growth factors of the present application. For example, in the third stage of in vitro expansion of the present application, the TILs of the present application can be contacted with the CD28 agonist of the present application and contact with the one or more T cell growth factors of the present application.

In one embodiment, in a single stage of in vitro expansion of the present application, the TILs of the present application are contacted with the CD28 agonist of the present application and the one or more T cell growth factors of the present application substantially at the same time. For example, in the first stage of in vitro expansion of the present application, the TILs of the present application are contacted with the CD28 agonist of the present application and the one or more T cell growth factors of the present application substantially at the same time. For example, in the second stage of in vitro expansion of the present application, the TILs of the present application are contacted with the CD28 agonist of the present application and the one or more T cell growth factors of the present application substantially at the same time. For example, in the third stage of in vitro expansion of the present application, the TILs of the present application are contacted with the CD28 agonist of the present application and the one or more T cell growth factors of the present application substantially at the same time.

In one embodiment, the T cell growth factors of the present application can be one or more of the factors selected from the group consisting of IL-2, IL-7, IL-12, IL-15, IL-21, interferon gamma, and functionally active fragments thereof. In one embodiment, the T cell growth factors of the present application can include IL-2 and/or functionally active fragments thereof. For example, the functionally active fragments of IL-2 can include fragments of IL-2 known in the art that can bind to the IL-2 receptors of T cells.

In one embodiment, the contact of the TILs of the present application with the one or more T cell growth factors of the present application can include adding the T cell growth factors of the present application into the cell culture medium of the TILs of the present application. In one embodiment, the initial concentration of the T cell growth factors of the present application in the cell culture medium of the TILs of the present application can be at least about 300 IU/mL. In one embodiment, the initial concentration of the IL-2 of the present application in the cell culture medium of the TILs of the present application can be at least about 350 IU/mL, at least about 400 IU/mL, at least about 500 IU/mL, at least about 600 IU/mL, at least about 700 IU/mL, at least about 800 IU/mL, at least about 900 IU/mL, at least about 1000 IU/mL, at least about 1100 IU/mL, at least about 1200 IU/mL, at least about 1300 IU/mL, at least about 1400 IU/mL, at least about 1500 IU/mL, at least about 2000 IU/mL, at least about 2500 IU/mL, at least about 2600 IU/mL, at least about 2700 IU/mL, at least about 2800 IU/mL, at least about 2900 IU/mL, at least about 3000 IU/mL, at least about 3100 IU/mL, at least about 3200 IU/mL, at least about 3300 IU/mL, at least about 3400 IU/mL, at least about 3500 IU/mL, at least about 4000 IU/mL, at least about 4500 IU/mL, at least about 5000 IU/mL, at least about 5500 IU/mL, at least about 6000 IU/mL, at least about 6500 IU/mL, at least about 7000 IU/mL, at least about 7500 IU/mL, at least about 8000 IU/mL, at least about 8500 IU/mL, or at least about 9000 IU/mL.

In one embodiment, compared to the corresponding TILs that have not been contacted with the CD28 agonist of the present application in the in vitro expansion stage, the TILs of the present application that have been contacted with the CD28 agonist of the present application in at least one stage of the in vitro expansion can show improved expansion effects. In one embodiment, the corresponding TILs that have not been contacted with the CD28 agonist of the present application can refer to TIL cells derived from the same donor that have not been contacted with the CD28 agonist of the present application. In one embodiment, the corresponding TILs that have not been contacted with the CD28 agonist of the present application can refer to TIL cells derived from the same donor that have been isolated in the same manner and that have not been contacted with the CD28 agonist of the present application. In one embodiment, the corresponding TILs that have not been contacted with the CD28 agonist of the present application can refer to TIL cells derived from the same tumor source of the same donor that have not been contacted with the CD28 agonist of the present application. In one embodiment, the corresponding TILs that have not been contacted with the CD28 agonist of the present application can refer to TIL cells derived from the same tumor source of the same donor that have been isolated in the same manner and that have not been contacted with the CD28 agonist of the present application. In one embodiment, the corresponding TILs that have not been contacted with the CD28 agonist of the present application can mean that the TIL cells derived from the same donor are divided into two groups, wherein one group of the TIL cells that have not been contacted with the CD28 agonist of the present application can be the corresponding TILs that have not been contacted with the CD28 agonist of the present application. In one embodiment, the corresponding TILs that have not been contacted with the CD28 agonist of the present application can mean that, the TIL cells derived from the same donor that are isolated in the same manner are divided into two groups, wherein one group of the TIL cells that have not been contacted with the CD28 agonist of the present application can be the corresponding TILs that have not been contacted with the CD28 agonist of the present application. In one embodiment, the corresponding TILs that have not been contacted with the CD28 agonist of the present application can mean that, the TIL cells derived from the same tumor source of the same donor are divided into two groups, wherein one group of the TIL cells that have not been contacted with the CD28 agonist of the present application can be the corresponding TILs that have not been contacted with the CD28 agonist of the present application. In one embodiment, the corresponding TILs that have not been contacted with the CD28 agonist of the present application can mean that, the TIL cells derived from the same tumor source of the same donor that are isolated in the same manner are divided into two groups, wherein one group of the TIL cells that have not been contacted with the CD28 agonist of the present application can be the corresponding TILs that have not been contacted with the CD28 agonist of the present application.

In one embodiment, the improved expansion effects of the present application can include one or more of the properties selected from the group consisting of an increased number of TIL cells, an improved proportion of T cell subpopulations, an enhanced cytokine secretion ability, and an enhanced tumor cell killing ability.

In one embodiment, the increased number of TIL cells in the present application can mean that, compared to the corresponding TILs that have not been contacted with the CD28 agonist of the present application in the in vitro expansion stage, the number of the TIL cells of the present application that have been contacted with the CD28 agonist of the present application in at least one in vitro expansion stage can be increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. In one embodiment, the increased number of TIL cells in the present application can mean that, compared to the corresponding TILs that have not been contacted with the CD28 agonist of the present application in the in vitro expansion stage, the number of the TIL cells of the present application that have been contacted with the CD28 agonist of the present application in at least one in vitro expansion stage can be increased by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%.

In one embodiment, the enhanced cytokine secretion ability in the present application can refer to the enhancement of the cytokine, selected from the group consisting of CD107a, GZMB, IL-2, TNF, and IFNγ, secretion ability of the TIL cells. In one embodiment, the enhanced cytokine secretion ability in the present application can mean that, compared to the corresponding TILs that have not been contacted with the CD28 agonist of the present application in the in vitro expansion stage, the cytokine secretion ability of the TILs of the present application that have been contacted with the CD28 agonist of the present application in at least one in vitro expansion stage can be increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. In one embodiment, the enhanced cytokine secretion ability in the present application can mean that, compared to the corresponding TILs that have not been contacted with the CD28 agonist of the present application in the in vitro expansion stage, the cytokine secretion ability of the TILs of the present application that have been contacted with the CD28 agonist of the present application in at least one in vitro expansion stage can be increased by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%. In one embodiment, the enhanced cytokine secretion ability in the present application can mean that, compared to the corresponding TILs that have not been contacted with the CD28 agonist of the present application in the in vitro expansion stage, the CD107a secretion ability of the TILs of the present application that have been contacted with the CD28 agonist of the present application in at least one in vitro expansion stage can be increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. In one embodiment, the enhanced cytokine secretion ability in the present application can mean that, compared to the corresponding TILs that have not been contacted with the CD28 agonist of the present application in the in vitro expansion stage, the CD 107a secretion ability of the TILs of the present application that have been contacted with the CD28 agonist of the present application in at least one in vitro expansion stage can be increased by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%. In one embodiment, the enhanced cytokine secretion ability in the present application can mean that, compared to the corresponding TILs that have not been contacted with the CD28 agonist of the present application in the in vitro expansion stage, the GZMB secretion ability of the TILs of the present application that have been contacted with the CD28 agonist of the present application in at least one in vitro expansion stage can be increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. In one embodiment, the enhanced cytokine secretion ability in the present application can mean that, compared to the corresponding TILs that have not been contacted with the CD28 agonist of the present application in the in vitro expansion stage, the GZMB secretion ability of the TILs of the present application that have been contacted with the CD28 agonist of the present application in at least one in vitro expansion stage can be increased by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%. In one embodiment, the enhanced cytokine secretion ability in the present application can mean that, compared to the corresponding TILs that have not been contacted with the CD28 agonist of the present application in the in vitro expansion stage, the IL-2 secretion ability of the TILs of the present application that have been contacted with the CD28 agonist of the present application in at least one in vitro expansion stage can be increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. In one embodiment, the enhanced cytokine secretion ability in the present application can mean that, compared to the corresponding TILs that have not been contacted with the CD28 agonist of the present application in the in vitro expansion stage, the IL-2 secretion ability of the TILs of the present application that have been contacted with the CD28 agonist of the present application in at least one in vitro expansion stage can be increased by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%. In one embodiment, the enhanced cytokine secretion ability in the present application can mean that, compared to the corresponding TILs that have not been contacted with the CD28 agonist of the present application in the in vitro expansion stage, the TNF secretion ability of the TILs of the present application that have been contacted with the CD28 agonist of the present application in at least one in vitro expansion stage can be increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. In one embodiment, the enhanced cytokine secretion ability in the present application can mean that, compared to the corresponding TILs that have not been contacted with the CD28 agonist of the present application in the in vitro expansion stage, the TNF secretion ability of the TILs of the present application that have been contacted with the CD28 agonist of the present application in at least one in vitro expansion stage can be increased by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%. In one embodiment, the enhanced cytokine secretion ability in the present application can mean that, compared to the corresponding TILs that have not been contacted with the CD28 agonist of the present application in the in vitro expansion stage, the IFNγ secretion ability of the TILs of the present application that have been contacted with the CD28 agonist of the present application in at least one in vitro expansion stage can be increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. In one embodiment, the enhanced cytokine secretion ability in the present application can mean that, compared to the corresponding TILs that have not been contacted with the CD28 agonist of the present application in the in vitro expansion stage, the IFNγ secretion ability of the TILs of the present application that have been contacted with the CD28 agonist of the present application in at least one in vitro expansion stage can be increased by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%. In one embodiment, the cytokine secretion ability of the TILs of the present application can be determined by measuring the cytokine expression ability of the TIL cells. In one embodiment, the cytokine secretion ability of the TILs of the present application is determined by measuring the cytokine release ability of the TIL cells. In one embodiment, the cytokine secretion ability of the TILs of the present application is determined by cytometric bead array (CBA).

In one embodiment, the enhanced tumor cell killing ability in the present application can mean that, compared to the corresponding TILs that have not been contacted with the CD28 agonist of the present application in the in vitro expansion stage, the tumor cell killing rate of the TILs of the present application that have been contacted with the CD28 agonist of the present application in at least one in vitro expansion stage can be increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. In one embodiment, the enhanced tumor cell killing ability in the present application can mean that, compared to the corresponding TILs that have not been contacted with the CD28 agonist of the present application in the in vitro expansion stage, the tumor cell killing rate of the TILs of the present application that have been contacted with the CD28 agonist of the present application in at least one in vitro expansion stage can be increased by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%. In one embodiment, the tumor cell killing rate of the TILs of the present application can be measured by CFSE and DAPI staining method. In one embodiment, the tumor cell killing of the TILs of the present application can refer to the ability of the TILs to kill solid tumor cells. In one embodiment, the tumor cell killing of the TILs of the present application can refer to the ability of the TILs to kill cervical cancer cells. In one embodiment, the tumor cell killing of the TILs of the present application can refer to the ability of the TILs to kill Hela cells.

In one embodiment, the improved proportion of T cell subpopulations in the present application can include one or more of the properties selected from the group consisting of an increased proportion of central memory T cells, a reduced proportion of regulatory T cells, an increased proportion of activated T cells, an increased proportion of tumor-specific T cells, and an increased proportion of stem cell-like T cells.

In one embodiment, the increased proportion of central memory T cells in the present application can be the increasement in the proportion of CD45RA⁻CCR7⁺ and/or CD45R0⁺CD62L⁺ cells in the TIL cells. For example, the proportion of central memory T cells in the TIL cells can be increased by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%.

In one embodiment, the reduced proportion of regulatory T cells in the present application can be the reduction in the proportion of $CD4^+CD25^+Foxp3^+$ cells in the TIL cells. For example, the proportion of regulatory T cells in the TIL cells can be reduced by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%.

In one embodiment, the increased proportion of activated T cells in the present application can be the increasement in the proportion of $CD25^+$, $CD28^+$, $PD1^+$ or $41BB^+$ cells in the TIL cells. For example, the proportion of activated T cells in the TIL cells can be increased by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%, or can be increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. For example, the proportion of $CD25^+$ cells in the TIL cells can be increased by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%, or can be increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. For example, the proportion of $CD28^+$ cells in the TIL cells can be increased by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%, or can be increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. For example, the proportion of $PD1^+$ cells in the TIL cells can be increased by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%, or can be increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold. For example, the proportion of $41BB^+$ cells in the TIL cells can be increased by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%, or can be increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold.

In one embodiment, compared to the corresponding TILs that have not been contacted with the CD28 agonist of the present application in the in vitro expansion stage, the TILs of the present application that have been contacted with the CD28 agonist of the present application in at least one stage of the in vitro expansion can show an improved gene editing effect. In one embodiment, the improved gene editing effect of the present application can include an enhanced gene knockout efficiency. In one embodiment, compared to the corresponding TILs that have not been contacted with the CD28 agonist of the present application in the in vitro expansion stage, the gene knockout efficiency of the TILs of the present application that have been contacted with the CD28 agonist of the present application in at least one in vitro expansion stage can be increased by at least about 100%, at least about 90%, at least about 80%, at least about 70%, at least about 60%, at least about 50%, at least about 40%, at least about 30%, at least about 20%, at least about 19%, at least about 18%, at least about 17%, at least about 16%, at least about 15%, at least about 14%, at least about 13%, at least about 12%, at least about 11%, at least about 10%, at least about 9%, at least about 8%, at least about 7%, at least about 6%, at least about 5%, at least about 4%, at least about 3%, at least about 2%, at least about 1%, at least about 0.5%, at least about 0.4%, at least about 0.3%, at least about 0.2%, or at least about 0.1%, or can be increased by at least about 1-fold, at least about 2-fold, at least about 3-fold, at least about 4-fold, at least about 5-fold, at least about 6-fold, at least about 7-fold, at least about 8-fold, at least about 9-fold, at least about 10-fold, at least about 11-fold, at least about 12-fold, at least about 13-fold, at least about 14-fold, at least about 15-fold, at least about 20-fold, at least about 30-fold, at least about 40-fold, or at least about 50-fold.

In one embodiment, the method of the present application can further include contacting the TILs of the present application with one or more other T cell activators other than the CD28 agonist of the present application in at least one stage of the in vitro expansion of the present application.

In one embodiment, in a single stage of the in vitro expansion of the present application, the other T cell activators can include agonists of one or more of the targets selected from the group consisting of CD3, HVEM, CD40L, OX40, and 4-1BB. The TILs of the present application are contacted with the CD28 agonist of the present application and with one or more other T cell activators of the present application. In one embodiment, in the first stage of in vitro expansion of the present application, the TILs of the present application can be contacted with the CD28 agonist of the present application and contact with the one or more other T cell activators of the present application. In one embodiment, in the second stage of in vitro expansion of the present application, the TILs of the present application can be contacted with the CD28 agonist of the present application and contact with the one or more other T cell activators of the present application. In one embodiment, in the third stage of in vitro expansion of the present application, the TILs of the present application can be contacted with the CD28 agonist of the present application and contact with the one or more other T cell activators of the present application.

In one embodiment, in a single stage of in vitro expansion of the present application, the TILs of the present application can be contacted with the CD28 agonist of the present application and the one or more other T cell activators of the present application substantially at the same time. In one embodiment, in the first stage of in vitro expansion of the present application, the TILs of the present application can be contacted with the CD28 agonist of the present application and the one or more other T cell activators of the present application substantially at the same time. In one embodiment, in the second stage of in vitro expansion of the present application, the TILs of the present application can be contacted with the CD28 agonist of the present application and the one or more other T cell activators of the present application substantially at the same time. In one embodiment, in the third stage of in vitro expansion of the present application, the TILs of the present application can be contacted with the CD28 agonist of the present application and the one or more other T cell activators of the present application substantially at the same time.

In one embodiment, the other T cell activators of the present application can include one or more of the molecules selected from the group consisting of CD80, CD86, B7-H3, 4-1BBL, CD27, CD30, CD134, B7h, CD40, LIGHT, and functionally active fragments thereof. In one embodiment, the other T cell activators of the present application can include agonists of one or more of the targets selected from the group consisting of CD3, HVEM, CD40L, OX40, and 4-1BB. In one embodiment, the other T cell activators of the present application can include those selected from the group consisting of antibodies against CD3, HVEM, CD40L, OX40, and 4-1BB as well as antigen-binding fragments thereof. In one embodiment, the other T cell activators of the present application can include a CD3 agonist. In one embodiment, the other T cell activators of the present application can include an anti-CD3 antibody and/or an antigen-binding fragment thereof; for example, it can be OKT3 from Miltenyi Biotech.

In one embodiment, the contact of the TILs of the present application with the CD28 agonist of the present application and the one or more other T cell activators of the present application can include one or more of the means selected from the group consisting of (1) adding the CD28 agonist of the present application and the other T cell activators of the present application into the cell culture medium of the TILs of the present application; (2) adding engineering cells expressing the CD28 agonist of the present application and the other T cell activators of the present application into the cell culture medium of the TILs of the present application; (3) adding a solid-phase medium comprising the CD28 agonist of the present application and the other T cell activators of the present application into the cell culture medium of the TILs of the present application. In one embodiment, the contact of the TILs of the present application with the CD28 agonist of the present application and the one or more other T cell activators of the present application can include adding a solid-phase medium comprising the CD28 agonist of the present application and the other T cell activators of the present application into the cell culture medium of the TILs of the present application. In one embodiment, the contact of the TILs of the present application with the CD28 agonist of the present application and the one or more other T cell activators of the present application can include adding a solid-phase medium comprising the CD28 antibody and the CD3 antibody of the present application into the cell culture medium of the TILs of the present application.

In one embodiment, the initial concentration of the other T cell activators in the cell culture medium of the TILs of the present application can be at least about 30 ng/mL. For example, the initial concentration of the CD28 antibody of the present application in the cell culture medium of the TILs of the present application can be at least about 30 ng/mL; for example, the initial concentration of the CD3 antibody of the present application in the cell culture medium of the TILs of the present application can be at least about 30 ng/mL. For example, the selection of the initial concentration of the CD28 antibody of the present application can be independent of the selection of the initial concentration of the CD3 antibody of the present application; for example, the initial concentrations of the CD28 antibody of the present application and the CD3 antibody of the present application in the cell culture medium of the TILs of the present application can be combined optionally. For example, the initial concentration of the CD28 antibody of the present application in the cell culture medium of the TILs of the present application can be optionally selected from about 30 ng/mL to about 300 ng/mL. For example, the initial concentration of the CD3 antibody of the present application in the cell culture medium of the TILs of the present application can be optionally selected from about 30 ng/mL to about 300 ng/mL. For example, the initial concentration of the CD28 antibody of the present application in the cell culture medium of the TILs of the present application can be optionally selected from about 30 ng/mL to about 300 ng/mL, and the initial concentration of the CD3 antibody of the present application in the cell culture medium of the TILs of the present application can be optionally selected from about 30 ng/mL to about 300 ng/mL, and the selection of the initial concentration of the CD28 antibody of the present application can be independent of the selection of the initial concentration of the CD3 antibody of the present application. In one embodiment, the diameter of the solid-phase medium of the present application can be about 500 nm to about 10 $\mu$m. In one embodiment, the diameter of the solid-phase medium of the present application can be measured by a transmission electron microscope. In one embodiment, the diameter of the solid-phase medium of the present application can be about 1 nm to about 500 nm. In one embodiment, the diameter of the solid-phase medium of the present application can be about 100 nm to about 500 nm. In one embodiment, the diameter of the solid-phase medium of the present application can be about 200 nm to about 500 nm. In one embodiment, the diameter of the solid-phase medium of the present application can be measured by a transmission electron microscope.

In one embodiment, the solid-phase medium of the present application can include a polymer. In one embodiment, the solid-phase medium of the present application can include glucan.

In one embodiment, the solid-phase medium of the present application includes at least about 25 $\mu$g of the CD28 agonist of the present application and other T cell activators of the present application per mg.

In one embodiment, a solid-phase medium comprising the CD28 agonist of the present application and other T cell activators of the present application is added into the cell culture medium of the TILs of the present application at a proportion of the solid-phase medium of the present application to the TILs of the present application from about 1:100 to about 1:2000. In one embodiment, a solid-phase medium comprising the CD28 agonist of the present application and other T cell activators of the present application is added into the cell culture medium of the TILs of the present application at a proportion of the solid-phase medium of the present application to the TILs of the present application from about 2:1 to about 1:2.

For example, when the diameter of the solid-phase medium of the present application is about 100 nm to about 500 nm, a solid-phase medium comprising the CD28 agonist of the present application and other T cell activators of the present application can be added into the cell culture medium of the TILs of the present application at a proportion of the solid-phase medium of the present application to the TILs of the present application from about 2:1 to about 1:2. For example, when the diameter of the solid-phase medium of the present application is about 100 nm to about 500 nm, a solid-phase medium comprising the CD28 agonist of the present application and other T cell activators of the present application, e.g., the CD3 agonist, can be added into the cell culture medium of the TILs of the present application at a proportion of the solid-phase medium of the present application to the TILs of the present application from about 2:1 to about 1:2, from about 2:1 to about 1:1, or from about 1:1 to about 1:2.

For example, when the diameter of the solid-phase medium of the present application is about 100 nm to about 500 nm, a solid-phase medium comprising the CD28 agonist of the present application and other T cell activators of the present application can be added into the cell culture medium of the TILs of the present application at a proportion of the solid-phase medium of the present application to the TILs of the present application from about 1:100 to about 1:2000. For example, when the diameter of the solid-phase medium of the present application is about 100 nm to about 500 nm, a solid-phase medium comprising the CD28 agonist of the present application and other T cell activators of the present application, e.g., the CD3 agonist, can be added into the cell culture medium of the TILs of the present application at a proportion of the solid-phase medium of the present application to the TILs of the present application from about 1:100 to about 1:2000, from about 1:200 to about 1:2000, from about 1:300 to about 1:2000, from about 1:400 to about 1:2000, from about 1:500 to about 1:2000, from about 1:600 to about 1:2000, from about 1:700 to about 1:2000, from about 1:800 to about 1:2000, from about 1:900 to about 1:2000, from about 1:1000 to about 1:2000, from about 1:1200 to about 1:2000, from about 1:1400 to about 1:2000, from about 1:1600 to about 1:2000, or from about 1:1800 to about 1:2000.

In one embodiment, the CD3 agonist of the present application can be a CD3 antibody or an antigen-binding protein thereof.

In the present application, the antibody of the present application or the antigen-binding protein thereof includes at least one CDR in the heavy chain variable region VH of the antibody. The CDRs of the present application can be defined according to IMGT nomenclature, Chothia, or Kabat.

For example, the antibody of the present application or the antigen-binding protein thereof can include HCDR1, and the HCDR1 of the present application can include an amino acid sequence as set forth in any one of SEQ ID NOs: 1 and 11; the CDRs of the present application can be defined according to IMGT nomenclature; the CDRs of the present application can be defined according to Kabat; for example, the antigen-binding protein of the present application can have the ability of binding CD3.

For example, the antibody of the present application or the antigen-binding protein thereof can include HCDR2, and the HCDR2 of the present application can include an amino acid sequence as set forth in any one of SEQ ID NOs: 2 and 12; the CDRs of the present application can be defined according to IMGT nomenclature; the CDRs of the present application can be defined according to Kabat; for example, the antigen-binding protein of the present application can have the ability of binding CD3.

For example, the antibody of the present application or the antigen-binding protein thereof can include HCDR3, and the HCDR3 of the present application can include an amino acid sequence as set forth in any one of SEQ ID NOs: 3 and 13; the CDRs of the present application can be defined according to IMGT nomenclature; the CDRs of the present application can be defined according to Kabat; for example, the antigen-binding protein of the present application can have the ability of binding CD3.

For example, the antibody of the present application or the antigen-binding protein thereof can include HCDR1-3, wherein the HCDR1 of the present application can include an amino acid sequence as set forth in any one of SEQ ID NOs: 1 and 11, the HCDR2 of the present application can include an amino acid sequence as set forth in any one of SEQ ID NOs: 2 and 12, and the HCDR3 of the present application can include an amino acid sequence as set forth in any one of SEQ ID NOs: 3 and 13; the CDRs of the present application can be defined according to IMGT nomenclature; the CDRs of the present application can be defined according to Kabat; for example, the antigen-binding protein of the present application can have the ability of binding CD3.

For example, the antibody of the present application or the antigen-binding protein thereof can include HCDR1-3 that are the same as those of OKT3, wherein the HCDR1 of the present application can include an amino acid sequence as set forth in SEQ ID NO: 1, the HCDR2 of the present application can include an amino acid sequence as set forth in SEQ ID NO: 2, and the HCDR3 of the present application can include an amino acid sequence as set forth in SEQ ID NO: 3; the CDRs of the present application can be defined according to IMGT nomenclature; for example, the antigen-binding protein of the present application can have the ability of binding CD3.

For example, the antibody of the present application or the antigen-binding protein thereof can include HCDR1-3 that are the same as those of SP34, wherein the HCDR1 of the present application can include an amino acid sequence as set forth in SEQ ID NO: 11, the HCDR2 of the present application can include an amino acid sequence as set forth in SEQ ID NO: 12, and the HCDR3 of the present application can include an amino acid sequence as set forth in SEQ ID NO: 13; the CDRs of the present application can be defined according to Kabat; for example, the antigen-binding protein of the present application can have the ability of binding CD3.

In the present application, the antibody of the present application or the antigen-binding protein thereof includes at least one CDR in the light chain variable region VL of the antibody. The CDRs of the present application can be defined according to IMGT nomenclature, or Kabat.

For example, the antibody of the present application or the antigen-binding protein thereof can include LCDR1, and the LCDR1 of the present application can include an amino acid sequence as set forth in any one of SEQ ID NOs: 4 and 14; the CDRs of the present application can be defined according to IMGT nomenclature; the CDRs of the present application can be defined according to Kabat; for example, the antigen-binding protein of the present application can have the ability of binding CD3.

For example, the antibody of the present application or the antigen-binding protein thereof can include LCDR2, and the LCDR2 of the present application can include an amino acid sequence as set forth in any one of SEQ ID NOs:

5 and 15; the CDRs of the present application can be defined according to IMGT nomenclature; the CDRs of the present application can be defined according to Kabat; for example, the antigen-binding protein of the present application can have the ability of binding CD3.

For example, the antibody of the present application or the antigen-binding protein thereof can include LCDR3, and the LCDR3 of the present application can include an amino acid sequence as set forth in any one of SEQ ID NOs: 6 and 16; the CDRs of the present application can be defined according to IMGT nomenclature; the CDRs of the present application can be defined according to Kabat; for example, the antigen-binding protein of the present application can have the ability of binding CD3.

For example, the antibody of the present application or the antigen-binding protein thereof can include LCDR1-3, wherein the LCDR1 of the present application can include an amino acid sequence as set forth in any one of SEQ ID NOs: 4 and 14, the LCDR2 of the present application can include an amino acid sequence as set forth in any one of SEQ ID NOs: 5 and 15, and the LCDR3 of the present application can include an amino acid sequence as set forth in any one of SEQ ID NOs: 6 and 16; the CDRs of the present application can be defined according to IMGT nomenclature; the CDRs of the present application can be defined according to Kabat; for example, the antigen-binding protein of the present application can have the ability of binding CD3.

For example, the antibody of the present application or the antigen-binding protein thereof can include LCDR1-3 that are the same as those of OKT3, wherein the LCDR1 of the present application can include an amino acid sequence as set forth in SEQ ID NO: 4, the LCDR2 of the present application can include an amino acid sequence as set forth in SEQ ID NO: 5, and the LCDR3 of the present application can include an amino acid sequence as set forth in SEQ ID NO: 6; the CDRs of the present application can be defined according to IMGT nomenclature; for example, the antigen-binding protein of the present application can have the ability of binding CD3.

For example, the antibody of the present application or the antigen-binding protein thereof can include LCDR1-3 that are the same as those of SP34, wherein the LCDR1 of the present application can include an amino acid sequence as set forth in SEQ ID NO: 14, the LCDR2 of the present application can include an amino acid sequence as set forth in SEQ ID NO: 15, and the LCDR3 of the present application can include an amino acid sequence as set forth in SEQ ID NO: 16; the CDRs of the present application can be defined according to Kabat; for example, the antigen-binding protein of the present application can have the ability of binding CD3.

For example, the antibody of the present application or the antigen-binding protein thereof can include HCDR1-3 and LCDR1-3, wherein the HCDR1 of the present application can include an amino acid sequence as set forth in any one of SEQ ID NOs: 1 and 11, the HCDR2 of the present application can include an amino acid sequence as set forth in any one of SEQ ID NOs: 2 and 12, the HCDR3 of the present application can include an amino acid sequence as set forth in any one of SEQ ID NOs: 3 and 13, the LCDR1 of the present application can include an amino acid sequence as set forth in any one of SEQ ID NOs: 4 and 14, the LCDR2 of the present application can include an amino acid sequence as set forth in any one of SEQ ID NOs: 5 and 15, and the LCDR3 of the present application can include an amino acid sequence as set forth in any one of SEQ ID NOs: 6 and 16; the CDRs of the present application can be defined according to IMGT nomenclature; the CDRs of the present application can be defined according to Kabat; for example, the antigen-binding protein of the present application can have the ability of binding CD3.

For example, the antibody of the present application or the antigen-binding protein thereof can include HCDR1-3 and LCDR1-3 that are the same as those of OKT3, wherein the HCDR1 of the present application can include an amino acid sequence as set forth in SEQ ID NO: 1, the HCDR2 of the present application can include an amino acid sequence as set forth in SEQ ID NO: 2, the HCDR3 of the present application can include an amino acid sequence as set forth in SEQ ID NO: 3, the LCDR1 of the present application can include an amino acid sequence as set forth in SEQ ID NO: 4, the LCDR2 of the present application can include an amino acid sequence as set forth in SEQ ID NO: 5, and the LCDR3 of the present application can include an amino acid sequence as set forth in SEQ ID NO: 6; the CDRs of the present application can be defined according to IMGT nomenclature; for example, the antigen-binding protein of the present application can have the ability of binding CD3.

For example, the antibody of the present application or the antigen-binding protein thereof can include HCDR1-3 that are the same as those of SP34, wherein the HCDR1 of the present application can include an amino acid sequence as set forth in SEQ ID NO: 11, the HCDR2 of the present application can include an amino acid sequence as set forth in SEQ ID NO: 12, the HCDR3 of the present application can include an amino acid sequence as set forth in SEQ ID NO: 13, the LCDR1 of the present application can include an amino acid sequence as set forth in SEQ ID NO: 14, the LCDR2 of the present application can include an amino acid sequence as set forth in SEQ ID NO: 15, and the LCDR3 of the present application can include an amino acid sequence as set forth in SEQ ID NO: 16; the CDRs of the present application can be defined according to Kabat; for example, the antigen-binding protein of the present application can have the ability of binding CD3.

In one embodiment, the antibody of the present application or the antigen-binding protein thereof can include a heavy chain variable region VH, and the VH of the present application can include an amino acid sequence as set forth in any one of SEQ ID NOs: 7 and 17; for example, the antigen-binding protein of the present application can have the ability of binding CD3.

For example, the antibody of the present application or the antigen-binding protein thereof can include a VH that is the same as that of OKT3, and the VH of the present application can include an amino acid sequence as set forth in SEQ ID NO: 7; for example, the antigen-binding protein of the present application can have the ability of binding CD3.

For example, the antibody of the present application or the antigen-binding protein thereof can include a VH that is the same as that of SP34, and the VH of the present application can include an amino acid sequence as set forth in SEQ ID NO: 17; for example, the antigen-binding protein of the present application can have the ability of binding CD3.

In one embodiment, the antibody of the present application or the antigen-binding protein thereof can include a light chain variable region VL, and the VL of the present application can include an amino acid sequence as set forth in any one of SEQ ID NOs: 8 and 18; for example, the antigen-binding protein of the present application can have the ability of binding CD3.

For example, the antibody of the present application or the antigen-binding protein thereof can include a VL that is the same as that of OKT3, and the VL of the present application can include an amino acid sequence as set forth in SEQ ID NO: 8; for example, the antigen-binding protein of the present application can have the ability of binding CD3.

For example, the antibody of the present application or the antigen-binding protein thereof can include a VL that is the same as that of SP34, and the VL of the present application can include an amino acid sequence as set forth in SEQ ID NO: 18; for example, the antigen-binding protein of the present application can have the ability of binding CD3.

In one embodiment, the antibody of the present application or the antigen-binding protein thereof can include a heavy chain variable region VH and a light chain variable region VL, and the VH of the present application can include an amino acid sequence as set forth in any one of SEQ ID NOs: 7 and 17, the VL of the present application can include an amino acid sequence as set forth in any one of SEQ ID NOs: 8 and 18; for example, the antigen-binding protein of the present application can have the ability of binding CD3.

For example, the antibody of the present application or the antigen-binding protein thereof can include a VH and a VL that are the same as those of OKT3, and the VH of the present application can include an amino acid sequence as set forth in SEQ ID NO: 7, the VL of the present application can include an amino acid sequence as set forth in SEQ ID NO: 8; for example, the antigen-binding protein of the present application can have the ability of binding CD3.

For example, the antibody of the present application or the antigen-binding protein thereof can include a VH and a VL that are the same as those of SP34, and the VH of the present application can include an amino acid sequence as set forth in SEQ ID NO: 17, the VL of the present application can include an amino acid sequence as set forth in SEQ ID NO: 18; for example, the antigen-binding protein of the present application can have the ability of binding CD3.

In one embodiment, the antibody of the present application or the antigen-binding protein thereof can include a heavy chain, and the heavy chain of the present application can include an amino acid sequence as set forth in any one of SEQ ID NOs: 9 and 19; for example, the antigen-binding protein of the present application can have the ability of binding CD3.

For example, the antibody of the present application or the antigen-binding protein thereof can include a heavy chain that is the same as that of OKT3, and the heavy chain of the present application can include an amino acid sequence as set forth in SEQ ID NO: 9; for example, the antigen-binding protein of the present application can have the ability of binding CD3 .

For example, the antibody of the present application or the antigen-binding protein thereof can include a heavy chain that is the same as that of SP34, and the heavy chain of the present application can include an amino acid sequence as set forth in SEQ ID NO: 19; for example, the antigen-binding protein of the present application can have the ability of binding CD3 .

In one embodiment, the antibody of the present application or the antigen-binding protein thereof can include a light chain, and the light chain of the present application can include an amino acid sequence as set forth in any one of SEQ ID NOs: 10 and 20; for example, the antigen-binding protein of the present application can have the ability of binding CD3.

For example, the antibody of the present application or the antigen-binding protein thereof can include a light chain that is the same as that of OKT3, and the light chain of the present application can include an amino acid sequence as set forth in SEQ ID NO: 10; for example, the antigen-binding protein of the present application can have the ability of binding CD3 .

For example, the antibody of the present application or the antigen-binding protein thereof can include a light chain that is the same as that of SP34, and the light chain of the present application can include an amino acid sequence as set forth in SEQ ID NO: 20; for example, the antigen-binding protein of the present application can have the ability of binding CD3 .

In one embodiment, the antibody of the present application or the antigen-binding protein thereof can include a heavy chain and a light chain, and the heavy chain of the present application can include an amino acid sequence as set forth in any one of SEQ ID NOs: 9 and 19, the light chain of the present application can include an amino acid sequence as set forth in any one of SEQ ID NOs: 10 and 20; for example, the antigen-binding protein of the present application can have the ability of binding CD3.

For example, the antibody of the present application or the antigen-binding protein thereof can include a heavy chain and a light chain that are the same as those of OKT3, and the heavy chain of the present application can include an amino acid sequence as set forth in SEQ ID NO: 9, the light chain of the present application can include an amino acid sequence as set forth in SEQ ID NO: 10; for example, the antigen-binding protein of the present application can have the ability of binding CD3.

For example, the antibody of the present application or the antigen-binding protein thereof can include a heavy chain and a light chain that are the same as those of SP34, and the heavy chain of the present application can include an amino acid sequence as set forth in SEQ ID NO: 19, the light chain of the present application can include an amino acid sequence as set forth in SEQ ID NO: 20; for example, the antigen-binding protein of the present application can have the ability of binding CD3.

In one embodiment, the CD28 agonist of the present application can be a CD28 antibody or an antigen-binding protein thereof.

In the present application, the antibody of the present application or the antigen-binding protein thereof includes at least one CDR in the heavy chain variable region VH of the antibody. The CDRs of the present application can be defined according to IMGT nomenclature, or Kabat.

For example, the antibody of the present application or the antigen-binding protein thereof can include HCDR1, and the HCDR1 of the present application can include an amino acid sequence as set forth in SEQ ID NO: 21; the CDRs of the present application can be defined according to IMGT nomenclature; the CDRs of the present application can be defined according to Kabat; for example, the antigen-binding protein of the present application can have the ability of binding CD28.

For example, the antibody of the present application or the antigen-binding protein thereof can include HCDR2, and the HCDR2 of the present application can include an amino acid sequence as set forth in SEQ ID NO: 22; the CDRs of the present application can be defined according to IMGT nomenclature; the CDRs of the present application can be defined according to Kabat; for example, the antigen-binding protein of the present application can have the ability of binding CD28.

For example, the antibody of the present application or the antigen-binding protein thereof can include HCDR3, and the HCDR3 of the present application can include an amino acid sequence as set forth in SEQ ID NO: 23; the CDRs of the present application can be defined according to IMGT nomenclature; the CDRs of the present application can be defined according to Kabat; for example, the antigen-binding protein of the present application can have the ability of binding CD28.

For example, the antibody of the present application or the antigen-binding protein thereof can include HCDR1-3, wherein the HCDR1 of the present application can include an amino acid sequence as set forth in SEQ ID NO: 21, the HCDR2 of the present application can include an amino acid sequence as set forth in SEQ ID NO: 22, and the HCDR3 of the present application can include an amino acid sequence as set forth in SEQ ID NO: 23; the CDRs of the present application can be defined according to IMGT nomenclature; the CDRs of the present application can be defined according to Kabat; for example, the antigen-binding protein of the present application can have the ability of binding CD28.

For example, the antibody of the present application or the antigen-binding protein thereof can include HCDR1-3 that are the same as those of 15E8, wherein the HCDR1 of the present application can include an amino acid sequence as set forth in SEQ ID NO: 21, the HCDR2 of the present application can include an amino acid sequence as set forth in SEQ ID NO: 22, and the HCDR3 of the present application can include an amino acid sequence as set forth in SEQ ID NO: 23; the CDRs of the present application can be defined according to IMGT nomenclature; for example, the antigen-binding protein of the present application can have the ability of binding CD28.

In the present application, the antibody of the present application or the antigen-binding protein thereof includes at least one CDR in the light chain variable region VL of the antibody. The CDRs of the present application can be defined according to IMGT nomenclature, or Kabat.

For example, the antibody of the present application or the antigen-binding protein thereof can include LCDR1, and the LCDR1 of the present application can include an amino acid sequence as set forth in SEQ ID NO: 24; the CDRs of the present application can be defined according to IMGT nomenclature; the CDRs of the present application can be defined according to Kabat; for example, the antigen-binding protein of the present application can have the ability of binding CD28.

For example, the antibody of the present application or the antigen-binding protein thereof can include LCDR2, and the LCDR2 of the present application can include an amino acid sequence as set forth in SEQ ID NO: 25; the CDRs of the present application can be defined according to IMGT nomenclature; the CDRs of the present application can be defined according to Kabat; for example, the antigen-binding protein of the present application can have the ability of binding CD28.

For example, the antibody of the present application or the antigen-binding protein thereof can include LCDR3, and the LCDR3 of the present application can include an amino acid sequence as set forth in SEQ ID NO: 26; the CDRs of the present application can be defined according to IMGT nomenclature; the CDRs of the present application can be defined according to Kabat; for example, the antigen-binding protein of the present application can have the ability of binding CD28.

For example, the antibody of the present application or the antigen-binding protein thereof can include LCDR1-3, wherein the LCDR1 of the present application can include an amino acid sequence as set forth in SEQ ID NO: 24, the LCDR2 of the present application can include an amino acid sequence as set forth in SEQ ID NO: 25, and the LCDR3 of the present application can include an amino acid sequence as set forth in SEQ ID NO: 26; the CDRs of the present application can be defined according to IMGT nomenclature; the CDRs of the present application can be defined according to Kabat; for example, the antigen-binding protein of the present application can have the ability of binding CD28.

For example, the antibody of the present application or the antigen-binding protein thereof can include LCDR1-3 that are the same as those of 15E8, wherein the LCDR1 of the present application can include an amino acid sequence as set forth in SEQ ID NO: 24, the LCDR2 of the present application can include an amino acid sequence as set forth in SEQ ID NO: 25, and the LCDR3 of the present application can include an amino acid sequence as set forth in SEQ ID NO: 26; the CDRs of the present application can be defined according to IMGT nomenclature; for example, the antigen-binding protein of the present application can have the ability of binding CD28.

For example, the antibody of the present application or the antigen-binding protein thereof can include HCDR1-3 and LCDR1-3, wherein the HCDR1 of the present application can include an amino acid sequence as set forth in SEQ ID NO: 21, the HCDR2 of the present application can include an amino acid sequence as set forth in SEQ ID NO: 22, the HCDR3 of the present application can include an amino acid sequence as set forth in SEQ ID NO: 23, the LCDR1 of the present application can include an amino acid sequence as set forth in SEQ ID NO: 24, the LCDR2 of the present application can include an amino acid sequence as set forth in SEQ ID NO: 25, and the LCDR3 of the present application can include an amino acid sequence as set forth in SEQ ID NO: 26; the CDRs of the present application can be defined according to IMGT nomenclature; the CDRs of the present application can be defined according to Kabat; for example, the antigen-binding protein of the present application can have the ability of binding CD28.

For example, the antibody of the present application or the antigen-binding protein thereof can include HCDR1-3 and LCDR1-3 that are the same as those of 15E8, wherein the HCDR1 of the present application can include an amino acid sequence as set forth in SEQ ID NO: 21, the HCDR2 of the present application can include an amino acid sequence as set forth in SEQ ID NO: 22, the HCDR3 of the present application can include an amino acid sequence as set forth in SEQ ID NO: 23, the LCDR1 of the present application can include an amino acid sequence as set forth in SEQ ID NO: 24, the LCDR2 of the present application can include an amino acid sequence as set forth in SEQ ID NO: 25, and the LCDR3 of the present application can include an amino acid sequence as set forth in SEQ ID NO: 26; the CDRs of the present application can be defined according to IMGT nomenclature; for example, the antigen-binding protein of the present application can have the ability of binding CD28.

In one embodiment, the antibody of the present application or the antigen-binding protein thereof can include a heavy chain variable region VH, and the VH of the present application can include an amino acid sequence as set forth in any one of SEQ ID NOs: 27 and 28; for example, the antigen-binding protein of the present application can have the ability of binding CD28.

For example, the antibody of the present application or the antigen-binding protein thereof can include a VH that is the same as that of 15E8, and the VH of the present application can include an amino acid sequence as set forth in SEQ ID NO: 27; for example, the antigen-binding protein of the present application can have the ability of binding CD28.

For example, the antibody of the present application or the antigen-binding protein thereof can include a VH that is the same as that of 15E8, and the VH of the present application can include an amino acid sequence as set forth in SEQ ID NO: 28; for example, the antigen-binding protein of the present application can have the ability of binding CD28.

In one embodiment, the antibody of the present application or the antigen-binding protein thereof can include a light chain variable region VL, and the VL of the present application can include an amino acid sequence as set forth in SEQ ID NO: 29; for example, the antigen-binding protein of the present application can have the ability of binding CD28.

For example, the antibody of the present application or the antigen-binding protein thereof can include a VL that is the same as that of 15E8, and the VL of the present application can include an amino acid sequence as set forth in SEQ ID NO: 29; for example, the antigen-binding protein of the present application can have the ability of binding CD28.

In one embodiment, the antibody of the present application or the antigen-binding protein thereof can include a heavy chain variable region VH and a light chain variable region VL, and the VH of the present application can include an amino acid sequence as set forth in any one of SEQ ID NOs: 27 and 28, the VL of the present application can include an amino acid sequence as set forth in SEQ ID NO: 29; for example, the antigen-binding protein of the present application can have the ability of binding CD28.

For example, the antibody of the present application or the antigen-binding protein thereof can include a VH and a VL that are the same as those of one 15E8, and the VH of the present application can include an amino acid sequence as set forth in SEQ ID NO: 27, the VL of the present application can include an amino acid sequence as set forth in SEQ ID NO: 29; for example, the antigen-binding protein of the present application can have the ability of binding CD28.

For example, the antibody of the present application or the antigen-binding protein thereof can include a VH and a VL that are the same as those of another 15E8, and the VH of the present application can include an amino acid sequence as set forth in SEQ ID NO: 28, the VL of the present application can include an amino acid sequence as set forth in SEQ ID NO: 29; for example, the antigen-binding protein of the present application can have the ability of binding CD28.

In one embodiment, the antibody of the present application or the antigen-binding protein thereof can include a heavy chain, and the heavy chain of the present application can include an amino acid sequence as set forth in any one of SEQ ID NOs: 30 and 31; for example, the antigen-binding protein of the present application can have the ability of binding CD28.

For example, the antibody of the present application or the antigen-binding protein thereof can include a heavy chain that is the same as that of one 15E8, and the heavy chain of the present application can include an amino acid sequence as set forth in SEQ ID NO: 30; for example, the antigen-binding protein of the present application can have the ability of binding CD28.

For example, the antibody of the present application or the antigen-binding protein thereof can include a heavy chain that is the same as that of another 15E8, and the heavy chain of the present application can include an amino acid sequence as set forth in SEQ ID NO: 31; for example, the antigen-binding protein of the present application can have the ability of binding CD28.

In one embodiment, the antibody of the present application or the antigen-binding protein thereof can include a light chain, and the light chain of the present application can include an amino acid sequence as set forth in SEQ ID NO: 32; for example, the antigen-binding protein of the present application can have the ability of binding CD28.

For example, the antibody of the present application or the antigen-binding protein thereof can include a light chain that is the same as that of 15E8, and the light chain of the present application can include an amino acid sequence as set forth in SEQ ID NO: 32; for example, the antigen-binding protein of the present application can have the ability of binding CD28.

In one embodiment, the antibody of the present application or the antigen-binding protein thereof can include a heavy chain and a light chain, and the heavy chain of the present application can include an amino acid sequence as set forth in any one of SEQ ID NOs: 30 and 31, the light chain of the present application can include an amino acid sequence as set forth in any one of SEQ ID NOs: 32 and 20; for example, the antigen-binding protein of the present application can have the ability of binding CD28.

For example, the antibody of the present application or the antigen-binding protein thereof can include a heavy chain and a light chain that are the same as those of one 15E8, and the heavy chain of the present application can include an amino acid sequence as set forth in SEQ ID NO: 30, the light chain of the present application can include an amino acid sequence as set forth in SEQ ID NO: 32; for example, the antigen-binding protein of the present application can have the ability of binding CD28.

For example, the antibody of the present application or the antigen-binding protein thereof can include a heavy chain and a light chain that are the same as those of another 15E8, and the heavy chain of the present application can include an amino acid sequence as set forth in SEQ ID NO: 31, the light chain of the present application can include an amino acid sequence as set forth in SEQ ID NO: 32; for example, the antigen-binding protein of the present application can have the ability of binding CD28.

In one embodiment, the TILs of the present application can be TILs derived from the fragments of tumor tissues of the present application. In one embodiment, the TILs of the present application can be obtained by processing the tumor tissues into tumor fragments. In one embodiment, the tumor fragments of the present application have a volume of about 1 to 27 mm$^3$. In one embodiment, the tumor fragments of the present application have a volume of about 1 mm$^3$, about 2 mm$^3$, about 3 mm$^3$, about 4 mm$^3$, about 5 mm$^3$, about 6 mm$^3$, about 7 mm$^3$, about 8 mm$^3$, about 9 mm$^3$, about 10 mm$^3$, about 11 mm$^3$, about 12 mm$^3$, about 13 mm$^3$, about 15 mm$^3$, about 17 mm$^3$, about 19 mm$^3$, about 20 mm$^3$, about 21 mm$^3$, about 23 mm$^3$, about 24 mm$^3$, about 25 mm$^3$, about 26 mm$^3$, or 27 mm$^3$.

In one aspect, the present application provides a method for culturing tumor-infiltrating lymphocytes (TILs), which can include (A) contacting a first TIL population derived from tumor tissues and not expanded in vitro with IL-2, wherein, a second TIL cell population is obtained through step (A); (B) contacting the second TIL population of the present application with the CD28 agonist and IL-2 substantially at the same time.

In one aspect, the present application provides a method for culturing tumor-infiltrating lymphocytes (TILs), which can include (A) contacting a first TIL population derived from tumor tissues and not expanded in vitro with IL-2, wherein, a second TIL cell population is obtained through step (A); (B) contacting the second TIL population of the present application with the CD28 agonist and IL-2 substantially at the same time for a certain period, and then co-culturing with feeder cells.

In one aspect, the present application provides a method for culturing tumor-infiltrating lymphocytes (TILs), which can include (A) contacting a first TIL population derived from tumor tissues and not expanded in vitro with IL-2 at a concentration of 300-9000 IU/mL, wherein, a second TIL cell population is obtained through step (A); (B) contacting the second TIL population of the present application with the CD28 agonist, the CD3 antibody and IL-2 at a concentration of 300-9000 IU/mL substantially at the same time for a certain period, and then co-culturing with PBMC cells at a proportion of the feeder cells to the TILs from about 40:1 to about 400:1.

In one aspect, the present application provides a method for culturing tumor-infiltrating lymphocytes (TILs). The method for obtaining TIL cells from a tissue sample of a subject can be obtaining an in-situ tumor sample or a metastatic tumor sample the weight of which may be at least about 1 g through surgery on the patient or combining a plurality of tissues. The tumor tissues are transported at about 2-8°C in a sample transport solution, e.g. a commercially commonly used tumor tissue transport solution, tumor tissue preservation solution, or tumor tissue transfer solution, and processed within 48 hours. The tissue pieces can be mechanically disrupted to pieces with a size of about 1-27 mm$^3$, transferred into a gas-permeable culture bag or Grex, into which are added T cell serum-free medium and IL-2 at a concentration of 300-9000 IU/mL (e.g., the concentration can be 1000-9000 IU/mL, for example, can be 6000 IU/mL), and cultured for about 3-14 days. The cells in the culture medium are collected and can be transferred together with the tissue pieces into a gas-permeable culture bag, Grex, or Xuri equipment. The T cell serum-free medium can be supplemented with the CD28 antibody of the present application, the CD3 antibody and CD28 antibody, magnetic beads containing the CD3 antibody and CD28 antibody (e.g., Dynabeads), and/or nano-matrix containing the CD3 antibody and CD28 antibody (e.g., transACT) and IL-2 at a concentration of 300-9000 IU/mL (e.g., the concentration can be 1000-9000 IU/mL, e.g., it can be 6000 IU/mL). After activation for a certain period of the present application, irradiated PBMCs (TILs and PBMCs are in a proportion of about 1:40 to about 1:400) are added, and the expansion and culture last about 3-14 days. After filtering the tissue pieces, the cells in the culture medium can be collected, washed, cryopreserved, and detected using a cell processing system. The proportion of CD3 in the final product can be greater than 80%, the cell viability rate can be greater than 70%, and more than 80% of the T cells can be memory effector T cells and effector T cells. After stimulation, the final product can secrete IPNγ, and/or can be characterized by an up-regulated proportion of activated T cells.

In one aspect, the present application provides a tumor-infiltrating lymphocyte (TIL), which can be obtained by culturing with the culture method of the present application. In one embodiment, the TILs provided in the present application can include one or a batch of the TILs obtained by culturing with the culture method of the present application. In one embodiment, the TILs provided in the present application can include various or multiple batches of the TILs obtained by culturing with the culture method of the present application and combined in any proportion.

In some embodiments, the TILs expanded by the method of the present application can be administered to a patient as a pharmaceutical composition. In some embodiments, the pharmaceutical composition can be a suspension of the TILs in a sterile buffer. The TILs expanded using the PBMCs of the present application can be administered by any suitable route known in the art. In some embodiments, T cells can be administered as a single intra-arterial or intravenous infusion, which can last about 30 to 60 minutes. Other suitable routes of administration can include intraperitoneal, intrathecal, and intralymphatic administration.

In some embodiments, any suitable dose of the TILs can be administered. In some embodiments, for example, when the tumor is melanoma, from about $2.3\times10^9$ to about $13.7\times10^{10}$ TILs can be administered. In some embodiments, from about $1\times10^9$ to about $12\times10^{10}$ TILs can be administered. In some embodiments, from about $1.2\times10^{10}$ to about $4.3\times10^{10}$ TILs can be administered. In some embodiments, from about $3\times10^{10}$ to about $12\times10^{10}$ TILs can be administered. In some embodiments, from about $4\times10^{10}$ to about $10\times10^{10}$ TILs can be administered. In some embodiments, from about $5\times10^{10}$ to about $8\times10^{10}$ TILs can be administered. In some embodiments, from about $6\times10^{10}$ to about $8\times10^{10}$ TILs can be administered. In some embodiments, from about $7\times10^{10}$ to about $8\times10^{10}$ TILs can be administered. In some embodiments, the therapeutically effective dose can be about $2.3\times10^9$ to about $13.7\times10^{10}$. In some embodiments, the therapeutically effective dose can be about $1\times10^9$ to about $12\times10^{10}$ TILs. In some embodiments, the therapeutically effective dose can be about $1.2\times10^{10}$ to about $4.3\times10^{10}$ TILs. In some embodiments, the therapeutically effective dose can be about $3\times10^{10}$ to about $12\times10^{10}$ TILs. In some embodiments, the therapeutically effective dose can be about $4\times10^{10}$ to about $10\times10^{10}$ TILs. In some embodiments, the therapeutically effective dose can be about $5\times10^{10}$ to about $8\times10^{10}$ TILs. In some embodiments, the therapeutically effective dose can be about $6\times10^{10}$ to about $8\times10^{10}$ TILs. In some embodiments, the therapeutically effective dose can be about $7\times10^{10}$ to about $8\times10^{10}$ TILs.

In some embodiments, the number of the TILs provided in the composition of the present application can be about $1\times10^6$, about $2\times10^6$, about $3\times10^6$, about $4\times10^6$, about $5\times10^6$, about $6\times10^6$, about $7\times10^6$, about $8\times10^6$, about $9\times10^6$, about $1\times10^7$, about $2\times10^7$, about $3\times10^7$, about $4\times10^7$, about $5\times10^7$, about $6\times10^7$, about $7\times10^7$, about $8\times10^7$, about $9\times10^7$, about $1\times10^8$, about $2\times10^8$, about $3\times10^8$, about $4\times10^8$, about $5\times10^8$, about $6\times10^8$, about $7\times10^8$, about $8\times10^8$, about $9\times10^8$, about $1\times10^9$, about $2\times10^9$, about $3\times10^9$, about $4\times10^9$, about $5\times10^9$, about $6\times10^9$, about $7\times10^9$, about $8\times10^9$, about $9\times10^9$, about $1\times10^{10}$, about $2\times10^{10}$, about $3\times10^{10}$, about $4\times10^{10}$, about $5\times10^{10}$, about $6\times10^{10}$, about $7\times10^{10}$, about $8\times10^{10}$, about $9\times10^{10}$, about $1\times10^{11}$, about $2\times10^{11}$, about $3\times10^{11}$, about $4\times10^{11}$, about $5\times10^{11}$, about $6\times10^{11}$, about $7\times10^{11}$, about $8\times10^{11}$, about $9\times10^{11}$, about $1\times10^{12}$, about $2\times10^{12}$, about $3\times10^{12}$, about $4\times10^{12}$, about $5\times10^{12}$, about $6\times10^{12}$, about $7\times10^{12}$, about $8\times10^{12}$, about $9\times10^{12}$, about $1\times10^{13}$, about $2\times10^{13}$, about

$3\times10^{13}$, about $4\times10^{13}$, about $5\times10^{13}$, about $6\times10^{13}$, about $7\times10^{13}$, about $8\times10^{13}$, or about $9\times10^{13}$. In some embodiments, the number of the TILs provided in the composition of the present application can range from about $1\times10^{6}$ to $5\times10^{6}$, about $5\times10^{6}$ to $1\times10^{7}$, about $1\times10^{7}$ to $5\times10^{7}$, about $5\times10^{7}$ to $1\times10^{8}$, about $1\times10^{8}$ to $5\times10^{8}$, about $5\times10^{8}$ to $1\times10^{9}$, about $1\times10^{9}$ to $5\times10^{9}$, about $5\times10^{9}$ to $1\times10^{10}$, about $1\times10^{10}$ to $5\times10^{10}$, about $5\times10^{10}$ to $1\times10^{11}$, about $5\times10^{11}$ to $1\times10^{12}$, about $1\times10^{12}$ to $5\times10^{12}$, or about $5\times10^{12}$ to $1\times10^{13}$.

In some embodiments, the concentration of the TILs provided in the composition of the present application can be less than, e.g., about 100%, about 90%, about 80%, about 70%, about 60%, about 50%, about 40%, about 30%, about 20%, about 19%, about 18%, about 17%, about 16%, about 15%, about 14%, about 13%, about 12%, about 11%, about 10%, about 9%, about 8%, about 7%, about 6%, about 5%, about 4%, about 3%, about 2%, about 1%, about 0.5%, about 0.4%, about 0.3%, about 0.2%, about 0.1%, about 0.09%, about 0.08%, about 0.07%, about 0.06%, about 0.05%, about 0.04%, about 0.03%, about 0.02%, about 0.01%, about 0.009%, about 0.008%, about 0.007%, about 0.006%, about 0.005%, about 0.004%, about 0.003%, about 0.002%, about 0.001%, about 0.0009%, about 0.0008%, about 0.0007%, about 0.0006%, about 0.0005%, about 0.0004%, about 0.0003%, about 0.0002%, or about 0.0001% w/w, w/v or v/v of the composition.

In some embodiments, the concentration of the TILs provided in the composition of the present application can be greater than about 90%, about 80%, about 70%, about 60%, about 50%, about 40%, about 30%, about 20%, about 19.75%, about 19.50%, about 19.25%, about 19%, about 18.75%, about 18.50%, about 18.25%, about 18%, about 17.75%, about 17.50%, about 17.25%, about 17%, about 16.75%, about 16.50%, about 16.25%, about 16%, about 15.75%, about 15.50%, about 15.25%, about 15%, about 14.75%, about 14.50%, about 14.25%, about 14%, about 13.75%, about 13.50%, about 13.25%, about 13%, about 12.75%, about 12.50%, about 12.25%, about 12%, about 11.75%, about 11.50%, about 11.25%, about 11%, about 10.75%, about 10.50%, about 10.25%, about 10%, about 9.75%, about 9.50%, about 9.25%, about 9%, about 8.75%, about 8.50%, about 8.25%, about 8%, about 7.75%, about 7.50%, about 7.25%, about 7%, about 6.75%, about 6.50%, about 6.25%, about 6%, about 5.75%, about 5.50%, about 5.25%, about 5%, about 4.75%, about 4.50%, about 4.25%, about 4%, about 3.75%, about 3.50%, about 3.25%, about 3%, about 2.75%, about 2.50%, about 2.25%, about 2%, about 1.75%, about 1.50%, about 125%, about 1%, about 0.5%, about 0.4%, about 0.3%, about 0.2%, about 0.1%, about 0.09%, about 0.08%, about 0.07%, about 0.06%, about 0.05%, about 0.04%, about 0.03%, about 0.02%, about 0.01%, about 0.009%, about 0.008%, about 0.007%, about 0.006%, about 0.005%, about 0.004%, about 0.003%, about 0.002%, about 0.001%, about 0.0009%, about 0.0008%, about 0.0007%, about 0.0006%, about 0.0005%, about 0.0004%, about 0.0003%, or about 0.0002%, or about 0.0001% w/w, w/v or v/v of the composition.

In some embodiments, the concentration of the TILs provided in the composition of the present application can range from about 0.0001% to about 50%, about 0.001% to about 40%, about 0.01% to about 30%, about 0.02% to about 29%, about 0.03% to about 28%, about 0.04% to about 27%, about 0.05% to about 26%, about 0.06% to about 25%, about 0.07% to about 24%, about 0.08% to about 23%, about 0.09% to about 22%, about 0.1% to about 21%, about 0.2% to about 20%, about 0.3% to about 19%, about 0.4% to about 18%, about 0.5% to about 17%, about 0.6% to about 16%, about 0.7% to about 15%, about 0.8% to about 14%, about 0.9% to about 12%, or about 1% to about 10% w/w, w/v or v/v of the composition.

In some embodiments, the concentration of the TILs provided in the composition of the present application can range from about 0.001% to about 10%, about 0.01% to about 5%, about 0.02% to about 4.5%, about 0.03% to about 4%, about 0.04% to about 3.5%, about 0.05% to about 3%, about 0.06% to about 2.5%, about 0.07% to about 2%, about 0.08% to about 1.5%, about 0.09% to about 1%, or about 0.1% to about 0.9% w/w, w/v or v/v of the composition.

In some embodiments, the amount of the TILs provided in the composition of the present application can be equal to or less than about 10 g, about 9.5 g, about 9.0 g, about 8.5 g, about 8.0 g, about 7.5 g, about 7.0 g, about 6.5 g, about 6.0 g, about 5.5 g, about 5.0 g, about 4.5 g, about 4.0 g, about 3.5 g, about 3.0 g, about 2.5 g, about 2.0 g, about 1.5 g, about 1.0 g, about 0.95 g, about 0.9 g, about 0.85 g, about 0.8 g, about 0.75 g, about 0.7 g, about 0.65 g, about 0.6 g, about 0.55 g, about 0.5 g, about 0.45 g, about 0.4 g, about 0.35 g, about 0.3 g, about 0.25 g, about 0.2 g, about 0.15 g, about 0.1 g, about 0.09 g, about 0.08 g, about 0.07 g, about 0.06 g, about 0.05 g, about 0.04 g, about 0.03 g, about 0.02 g, about 0.01 g, about 0.009 g, about 0.008 g, about 0.007 g, about 0.006 g, about 0.005 g, about 0.004 g, about 0.003 g, about 0.002 g, about 0.001 g, about 0.0009 g, about 0.0008 g, about 0.0007 g, about 0.0006 g, about 0.0005 g, about 0.0004 g, about 0.0003 g, about 0.0002 g, or about 0.0001 g.

In some embodiments, the amount of the TILs provided in the composition of the present application can be greater than about 0.0001 g, about 0.0002 g, about 0.0003 g, about 0.0004 g, about 0.0005 g, about 0.0006 g, about 0.0007 g, about 0.0008 g, about 0.0009 g, about 0.001 g, about 0.0015 g, about 0.002 g, about 0.0025 g, about 0.003 g, about 0.0035 g, about 0.004 g, about 0.0045 g, about 0.005 g, about 0.0055 g, about 0.006 g, about 0.0065 g, about 0.007 g, about 0.0075 g, about 0.008 g, about 0.0085 g, about 0.009 g, about 0.0095 g, about 0.01 g, about 0.015 g, about 0.02 g, about 0.025 g, about 0.03 g, about 0.035 g, about 0.04 g, about 0.045 g, about 0.05 g, about 0.055 g, about 0.06 g, about 0.065 g, about 0.07 g, about 0.075 g, about 0.08 g, about 0.085 g, about 0.09 g, about 0.095 g, about 0.1 g, about 0.15 g, about 0.2 g, about 0.25 g, about 0.3 g, about 0.35 g, about 0.4 g, about 0.45 g, about 0.5 g, about 0.55 g, about 0.6 g, about 0.65 g, about 0.7 g, about 0.75 g, about 0.8 g, about 0.85 g, about 0.9 g, about 0.95 g, about 1 g, about 1.5 g, about 2 g, about 2.5 g, about 3 g, about 3.5 g, about 4 g, about 4.5 g, about 5 g, about 5.5 g, about 6 g, about 6.5 g, about 7 g, about 7.5 g, about 8 g, about 8.5 g, about 9 g, about 9.5 g, or about 10 g.

In some embodiments, the TILs can be administered in a single dose. Such administration can be by injection, e.g., intravenous injection. In some embodiments, the TILs can be administered in multiple doses. The dosage can be once, twice, three times, four times, five times, six times, or more than six times a year. The dosage can be once a month, once every two weeks, once a week, or once every 2 days. In some embodiments, the administration of the TILs can be continuous.

In one aspect, the present application provides a pharmaceutical composition. In some embodiments, the pharmaceutical composition can include the TILs of the present application and/or the composition of the present application, and a pharmaceutically acceptable carrier.

In one aspect, the present application provides a kit, which can include a T cell activator, a T cell growth factor, and/or feeder cells used in the method for culturing tumor-infiltrating lymphocytes (TILs) of the present application, and instructions describing the steps of the method for culturing tumor-infiltrating lymphocytes (TILs) of the present application. In one aspect, the present application provides a kit, which can include the TILs of the present application and/or the pharmaceutical composition of the present application.

In one aspect, the present application provides a method for affecting tumor cell growth, which can include administering to a subject the TILs of the present application and/or the pharmaceutical composition of the present application. In some embodiments, affecting tumor growth can include reducing the volume of the tumor to, for example, about 99%, about 95%, about 90%, about 80%, about 70%, about 60%, about 50%, about 40%, about 30%, about 20%, about 19%, about 18%, about 17%, about 16%, about 15%, about 14%, about 13%, about 12%, about 11%, about 10%, about 9%, about 8%, about 7%, about 6%, about 5%, about 4%, about 3%, about 2%, about 1%, about 0.5%, about 0.4%, about 0.3%, about 0.2% or about 0.1% of the volume before the administration.

In one aspect, the present application provides use of the TILs of the present application and/or the pharmaceutical composition of the present application for the manufacture of a medicament that can be used for preventing and/or treating tumors. In some embodiments, the tumors of the present application are selected from solid tumors. In some embodiments, the tumors of the present application are one or more of the tumors selected from the group consisting of melanoma, ovarian cancer, cervical cancer, lung cancer, bladder cancer, breast cancer, head and neck cancer, pancreatic cancer, liver cancer, stomach cancer, colorectal cancer, and kidney cancer.

In one aspect, the present application provides a method for preventing and/or treating tumors, which can include administering to a subject the TILs of the present application and/or the pharmaceutical composition of the present application. In some embodiments, the tumors of the present application are selected from solid tumors. In some embodiments, the tumors of the present application are one or more of the tumors selected from the group consisting of melanoma, ovarian cancer, cervical cancer, lung cancer, bladder cancer, breast cancer, head and neck cancer, pancreatic cancer, liver cancer, stomach cancer, colorectal cancer, and kidney cancer.

In one aspect, the present application provides a TIL of the present application and/or a pharmaceutical composition of the present application, which can be used for preventing and/or treating tumors. In some embodiments, the tumors of the present application are selected from solid tumors. In some embodiments, the tumors of the present application are one or more of the tumors selected from the group consisting of melanoma, ovarian cancer, cervical cancer, lung cancer, bladder cancer, breast cancer, head and neck cancer, pancreatic cancer, liver cancer, stomach cancer, colorectal cancer, and kidney cancer.

Without intending to be limited by any theory, the following examples are only intended to illustrate the fusion protein, the preparation method and use, etc. of the present application, and are not intended to limit the scope of the present invention.

Examples

**Example 1 Method for culturing tumor-infiltrating lymphocyte (TIL) cells**

Reception and preparation of feeder cells

1.1.1 Reception of apheresis blood

The information of the apheresis blood, batch numbers, and volumes were recorded, and the blood sample was rewarmed to room temperature.

1.1.2 Manual isolation and cryopreservation of PBMCs (peripheral blood mononuclear cells)

The blood bag was sterilized with 75% alcohol and transferred into a biosafety cabinet. After the blood bag cutting with one sterile scissor, the apheresis blood was transferred into 50 mL centrifuge tubes. The blood bag was washed with 20 mL PBS or normal saline that was injected with a 20 mL syringe. The washing solution was also transferred into the 50 mL centrifuge tubes. The liquid volume in each 50 mL centrifuge tube should might not exceed 30 mL. The apheresis blood within the tube was centrifuged at 3000 g for 10 minutes. During the centrifugation, 6-8 tubes of 50 mL centrifuge tubes should be prepared with pre-warmed lymphocyte separation solution (Ficoll, Tianjin Haoyang), 20 mL/tube. After the centrifugation, the upper layer of plasma was discarded, and the cell pellets were diluted with PBS or normal saline. The diluted blood cell mixture solution was slowly added dropwise onto the upper layer of the lymphocyte separation solution without destroying the interface. About 25 mL of samples was added for each tube, and should the final volume within each tube should be no more than 28 mL.

Centrifugation was carried out at temperature of 18-22°C and 500-600 g for 15-30 minutes using a horizontal rotor. After centrifugation, the resulting buffy coat will be at the interface of normal saline and the lymphocyte separation solution Ficoll. The upper layer of plasma and normal saline was aspirated off, and the middle buffy coat was transferred to another 50 mL sterile centrifuge tube with pipette. The collected buffy coat was diluted with PBS or normal saline and centrifuged at room temperature and 600 g for 10 minutes. After the centrifugation was completed, the supernatant was discarded. The cells were washed once with PBS or normal saline and centrifuged at room temperature and 500 g for 5 minutes.

If there were lots of red blood cells, the remanent red blood cells should might be lysed after centrifugation. A red blood cell lysis solution was added at a volume ratio of 1:2 to 1:3 of the cell pellets to the red blood cell lysis solution and mixed well. Red blood cells lysis at room temperature for 10 minutes, the centrifuge tubes were gently mixed for 2-3 times to ensure the lysis effect. After the lysis was completed, PBS or normal saline was added to wash the cells. After the lysis, the cells were washed twice, centrifuged at 400 g for 6 minutes and counted before the last centrifugation.

The supernatant was discarded, and the cells were resuspended in the basic medium with the cell density adjusted to about $2-3\times10^7$/mL, wherein the liquid level should be not higher than 1 cm, and the volume in each T225 culture flask should be less than 200 mL. The suspension was irradiated with X-rays at 50 Gy in the tiled state. The supernatant was discarded after centrifugation, and the cells were cryopreserved according to the counting results in about $1-2\times10^8$ cells/mL and 1-2 mL/tube. The cells were placed in a programmed cooling box and transferred to a -80°C freezer for cryopreservation.

1.1.3 Automatic isolation and cryopreservation of PBMCs

The tubing of the blood bag was connected to the input end of a cpro-separation kit (Cytiva) aseptically. If the blood volume was more than 120 mL, a pre-concentration step should be performed to concentrate the blood volume to less than 120 mL. A neatcell procedure should be used to isolate and wash PBMCs, wherein the washing solution was normal saline, and the intermediate volume was 20 mL; the resuspending solution was the basic culture medium, and 80 mL was added for each batch. After isolation, the PBMCs of each donor were one bag of 100 mL. When in the tiled state, the liquid level should be not higher than 1 cm, and the suspension was irradiated with X-rays at 50 Gy. Sampling and counting were carried out after irradiation. The PBMC suspensions of 3-5 donors were mixed according to the ratio of 0.5:1 to 1:2. Cells were collected and washed three times using a culture wash procedure, and the washing solution was normal saline; the intermediate volume and the final volume were set, so that the volume reached no less than 2 mL/$1\times10^9$ cells; an equal amount to 2-fold cryopreservation solution was added and mixed well. The cell density was adjusted to about $1\times10^7$ cells/mL to $2\times10^8$ cells/mL with a 1-fold cryopreservation solution. The suspension was divided into 20 mL/bag, cryopreserved in a programmed cooler, and stored in liquid nitrogen.

1.2 Reception and processing of tumor tissues

1.2.1 Reception of tissues

Tumor tissues and blood samples were received from donors. The sample information was checked and recorded, and corresponding sample labels were printed.

1.2.2 Tissue processing and culture

The sample tubes and blood collection tubes were sterilized with 75% alcohol and transferred into a biosafety cabinet. The PBMC cells in the blood samples were isolated and cryopreserved according to the above procedures for manual isolation and cryopreservation of PBMCs. A kind of culture flasks and bags with gas permeable surfaces, e.g.

G-Rex100 culture flasks (Wilson Wolf Manufacturing) were taken. 300 mL of rewarmed complete medium was added, which should optionally select X-vivo 15 medium or other commercially available T cell culture media, e.g. T cell culture media of Stem Cell, Lonza, Thermo, Miltenyi brands, etc., and into which essential amino acids and antibiotics should be added, and IL-2 was added at a concentration of 300-9000 IU/mL (e.g., it may be 1000-9000 IU/mL, e.g., it may be 6000 IU/mL). Several 10 cm culture dishes were taken, into which was added an appropriate amount of a medium. The tumor tissues were taken out from the sample tubes into the 10 cm culture dishes using sterile ophthalmic forceps. The amount of the culture medium was such that the tumor tissues were just immersed. The tissue morphology was observed and recorded. The tissues were washed and the culture dishes were replaced. The tissues were cut preliminarily using ophthalmic scissors and ophthalmic forceps to remove fatty tissues and necrotic tissues. Each tissue piece was further cut to a size of about 27 mm$^3$. Non-suspended tumor tissue pieces were taken. 20 mL syringes were, after removing the inner pistons, connected with the culture bags. About 1 g of the tissue pieces were transferred into the culture bags through the syringes using pipettes. The culture bags were placed in a carbon dioxide incubator to culture. The scissors and forceps were preliminary disinfected with 75% alcohol after cleaning, and sterilized after ultrasonic cleaning, to obtain the first TIL population.

1.3 First stage of in vitro expansion and harvest (preREP stage)

1.3.1 First stage of in vitro expansion

According to the cell growth status, the culture medium was replenished or replaced for half amount every 3-7 days to ensure the nutrition for the cells. A complete medium was used, which should optionally select X-vivo 15 medium or other commercially available T cell culture media, e.g. T cell culture media of Stem Cell, Lonza, Thermo, Miltenyi brands, etc., and into which essential amino acids and antibiotics should be added, and IL-2 was added at a concentration of 300-9000 IU/mL (e.g., it may be 1000-9000 IU/mL, e.g., it may be 6000 IU/mL), for example, 6000 IU/mL of IL-2. Sampling and counting were carried out on days 3-14, e.g. on days 3, 4, 5, 6, 7, 8, 9, 10, 11, 12, 13, or 14, of the first stage of the in vitro expansion. If the cell number was between $5 \times 10^5$ and $5 \times 10^8$, the cells entered the harvest step of the first stage of the in vitro expansion described below.

1.3.2 Harvest of the first stage of in vitro expansion

The cells at the end of the first stage of in vitro expansion were collected and centrifuged. The culture medium was discarded. The cells were washed once with PBS or normal saline to obtain the TILs subjected to the first stage of in vitro expansion (the second TIL population). Sampling and counting were carried out to leave about $5 \times 10^5$ to $2 \times 10^8$ cells to enter the step of the first stage of in vitro expansion described below; about $5 \times 10^5$ cells should be taken for quality control detection; the rest of the cells were added into an equal volume of 2-fold cryopreservation solution for cryopreservation.

1.4 Second stage of in vitro expansion (REP stage)

1.4.1 Activation of the TILs in the second stage of in vitro expansion

An amount of $5 \times 10^5$ to $2 \times 10^8$ cells subjected to the first stage of in vitro expansion were taken. A complete medium was used, which should optionally select X-vivo 15 medium or other commercially available T cell culture media, e.g. T cell culture media of Stem Cell, Lonza, Thermo, Miltenyi brands, etc., and into which essential amino acids and antibiotics should be added to adjust the cell density to $5 \times 10^5$ to $2 \times 10^6$ cells/mL. Into a suspension 24-well culture plate was added, at 1 mL/well, IL-2 at a concentration of 300-9000 IU/mL (for example, the concentration should be 1000-9000 IU/mL, e.g., it should be 6000 IU/mL). While adding IL-2 to each experimental group, the corresponding CD28 agonist was added according to the following formula:
Control group: adding the CD3 antibody into the culture medium simultaneously, for example, about 30 ng/mL of OKT3.
Mixed antibody group: adding the CD3 antibody and the CD28 antibody into the culture medium at the same time, for example, about 30 ng/mL of OKT3, and about 30 ng/mL of CD28 antibody.
Magnetic bead group: adding magnetic beads (Dynabeads with a diameter of about 1 to 10 $\mu$m, Thermo Fisher) comprising the CD3 antibody and the CD28 antibody into the culture medium at the same time, for example, the magnetic beads were added at a ratio of magnetic beads to TILs from about 1:2 to 2:1.
Nano-matrix group: adding transACT (with a diameter of about 100 to 500 nm, Miltenyi) comprising the CD3 antibody and the CD28 antibody into the culture medium at the same time, for example, transACT was added at a ratio of transACT to TILs from about 1: 100 to 1:2000.

1.4.2 Expanded culture in the second stage of in vitro expansion

For each of the above experimental groups, in the second stage of in vitro expansion, some time $T_n$ ($T_n$ should be from 0 hours to 14 days, e.g., 24 hours or 48 hours) after adding IL-2 and different forms of T cell activators, the mixed feeder cells from 1-5 donors were resuscitated; the activated TIL cells, tissue pieces, and feeder cells were transferred into G-Rex100 culture flasks or gas-permeable bags, into which the complete culture medium was supplemented. Sampling and counting were carried out every 1-3 days, and the culture medium was replenished or half-changed depending on the cell status until the total number of the cells was more than $1 \times 10^9$, or the culture time of the second stage of in vitro expansion reached 13 days, then the culture was terminated.

1.4.3 Harvest of tumor-infiltrating lymphocytes

The TILs subjected to the second stage of in vitro expansion (the third TIL population) were obtained by taking the cells subjected to the second stage of in vitro expansion, discarding the supernatant of the culture medium after centrifugation, and washing three times with PBS or normal saline or a compound electrolyte solution. Sampling and counting were carried out during the third washing. According to the counting results, the supernatant was discarded after the last centrifugation, and $3 \times 10^6$ cells were taken and sent for quality control detection; all the remaining cells were added to the cryopreservation solution, and the cell density was adjusted to $1\text{-}3 \times 10^8$ cells/mL for cryopreservation.

1.5 Application of tumor-infiltrating lymphocytes

Resuscitated therapeutic tumor-infiltrating lymphocytes should be administered to a subject by intravenous infusion.

**Example 2 Test of the proliferative ability of the TILs**

Cell counts were performed on the third TIL population obtained from the culture of the second stage of in vitro expansion of each experimental group in Example 1.

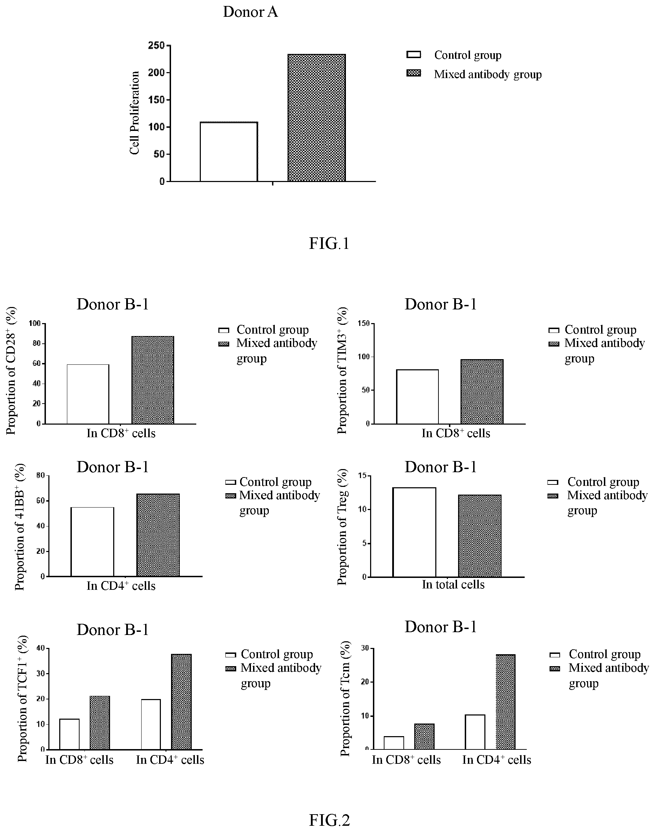

The analysis of the proliferative ability of the experimental groups added with different forms of the CD28 agonists and the control group is shown in FIG. 1. The values of the vertical coordinate in FIG. 1 indicate the expansion fold of the number of the TIL cells in the third TIL population obtained from the second stage of in vitro expansion for each experimental group, compared with that in the second TIL population before the start of the second stage of in vitro expansion. The results show that, for donor A, the proliferative ability of the TILs obtained from the second stage of in vitro expansion with the CD28 antibody added are stronger than those of the TILs cultured in the control group (without the addition of the CD28 antibody).

**Example 3 Flow cytometry test of the TILs**

The third TIL population obtained from the culture of the second stage of in vitro expansion for each experimental group in Example 1 was tested by flow cytometry.

Sources of test materials for the flow cytometry of the TILs

Transcription Factor Buffer Set, Manufacturer BD, Product Number 562574; V-bottomed 96-well plate, Manufacturer Corning, Product Number 3894; Flow tube, Manufacturer Corning, Product Number 352052.

The flow antibodies in this example were purchased from BD or Biolegend. $1 \times 10^5$ to $5 \times 10^5$ cell samples per group were added into flow tubes or V-bottom 96-well plates. Centrifugation was performed at 600 g for 3 minutes and the supernatant was discarded. Washing was carried out once using PBS, with 1 mL/tube for flow tubes, and 250 $\mu$L/well for 96-well plates, and the supernatant was discarded. A formulated antibody working solution was added for cell surface staining, in which the concentration of the antibody (BD or Biolegend) was 1:100 to 1:200, and the reactive detection dyes were contained in 1:10000. Staining was carried out with 100 $\mu$L/tube for flow tubes, and 50 $\mu$L/well for 96-well plates, and incubation was performed at 2-8°C in dark for 30 minutes. Formulation of reagents required for transcription factor staining during the staining process: Transcription Factor Buffer Set (BD) was used to dilute a 4× Fixation/Permeabilization Solution (BD) to produce 1× working solution A; double distilled water was used to dilute a 5× Perm/Wash Buffer (BD) to produce 1× working solution B. They were pre-cooled at 4°C ready for use. After the staining was completed, an appropriate amount of PBS was added to wash the cells twice (250 $\mu$L each time for 96-well plates, 1 mL each time for flow tubes). Centrifugation was performed at 600 g for 3 minutes. After the centrifugation, the supernatant was discarded. Cell fixation and permeabilization: the cells were sufficiently resuspended. An appropriate amount (100

μL/well for 96-well plates, and 1 mL/tube for flow tubes) of 1× working solution A was added to fix and permeabilize. Incubation was performed at 2-8°C in dark for 40-50 minutes. After the fixation and permeabilization were completed, 1× working solution B was added to wash the cells (250 μL each time for 96-well plates, and 2 mL each time for flow tubes). Centrifugation was performed at 2-8°C and 350 g for 6 minutes, and washing was carried out twice. 1× working solution B was used to formulate intracellular antibodies of which the concentration was 1:100 to 1:200, with 50 μL/well for 96-well plates, and 100 μL/tube for flow tubes. Staining was performed at 2-8°C in dark for 30 minutes. After the staining was completed, 1× working solution B was added to wash the cells (250 μL each time for 96-well plates, and 2 mL each time for flow tubes). Centrifugation was performed at 2-8°C and 350 g for 6 minutes, and washing was carried out twice. After the surface staining was completed, PBS was used to wash the cells once (250 μL each time for 96-well plates, 1 mL each time for flow tubes). Centrifugation was performed at room temperature and 600 g for 3 minutes. After the centrifugation, the supernatant was discarded. The cells were resuspended with 100-500 μL PBS for an on-board flow cytometry test.

The analysis of the flow cytometry results of the TILs cultured with different stimulants is shown in FIGs. 2-6.

FIG. 2 shows the ratio of T cell subpopulations of the TIL cells obtained from the culture of a mixed antibody group and a control group for donor B-1. The results show that the TILs obtained from the second stage of in vitro expansion with the CD28 antibody added, compared with the control group (without the addition of the CD28 antibody), have an improved ratio of T cell subpopulations. For example, a higher ratio of activated T cells (CD28$^+$ or 41BB$^+$), a lower ratio of regulatory T cells (Treg, e.g., CD4$^+$CD25$^+$Foxp3$^+$), a higher ratio of stem cell-like T cells (TCF1$^+$), and/or a higher ratio of central memory T cells (Tcm, e.g., CD45RA$^-$CCR7$^+$).

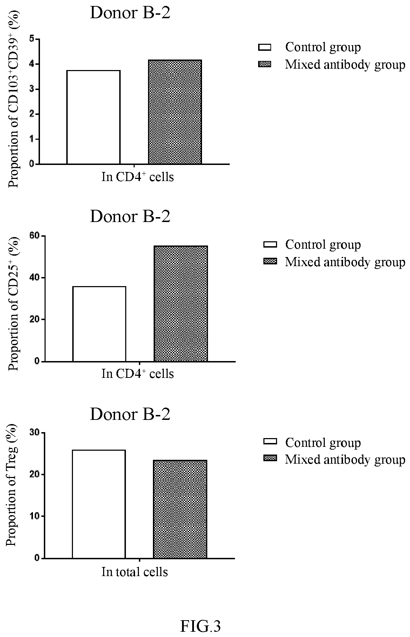

FIG. 3 shows the ratio of T cell subpopulations of the TIL cells obtained from the culture of a mixed antibody group and a control group for donor B-2. The results show that the TILs obtained from the second stage of in vitro expansion with the CD28 antibody added, compared with the control group (without the addition of the CD28 antibody), have an improved ratio of T cell subpopulations. For example, a higher ratio of tumor-specific T cells (CD103$^+$CD39$^+$), a higher ratio of activated T cells (CD25$^+$), and/or a lower ratio of regulatory T cells (Treg, e.g., CD4$^+$CD25$^+$Foxp3$^+$).

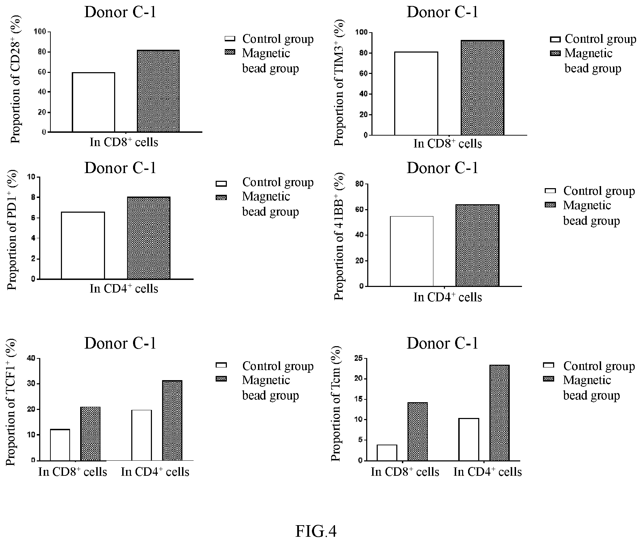

FIG. 4 shows the ratio of T cell subpopulations of the TIL cells obtained from the culture of a magnetic bead group and a control group for donor C-1. The results show that the TILs obtained from the second stage of in vitro expansion with the CD28 antibody added (for example, with the addition of magnetic beads including the CD3 antibody and the CD28 antibody), compared with the control group (without the addition of the CD28 antibody), have an improved ratio of T cell subpopulations. For example, a higher ratio of activated T cells (CD28$^+$, PD1$^+$ or 41BB$^+$), a higher ratio of stem cell-like T cells (TCF1$^+$), and/or a higher ratio of central memory T cells (Tcm, e.g., CD45RA$^-$CCR7$^+$).

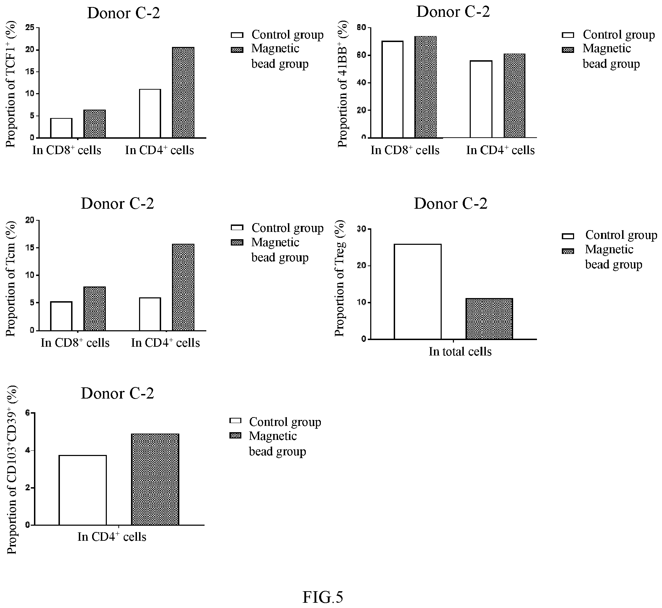

FIG. 5 shows the ratio of T cell subpopulations of the TIL cells obtained from the culture of a magnetic bead group and a control group for donor C-2. The results show that the TILs obtained from the second stage of in vitro expansion with the CD28 antibody added (for example, with the addition of magnetic beads including the CD3 antibody and the CD28 antibody), compared with the control group (without the addition of the CD28 antibody), have an improved ratio of T cell subpopulations. For example, a higher ratio of stem cell-like T cells (TCF1$^+$), a higher ratio of activated T cells (41BB$^+$), a higher ratio of central memory T cells (Tcm, e.g., CD45RA$^-$CCR7$^+$), a lower ratio of regulatory T cells (Treg, e.g., CD4$^+$CD25$^+$Foxp3$^+$), and/or a higher ratio of tumor-specific T cells (CD103$^+$CD39$^+$).

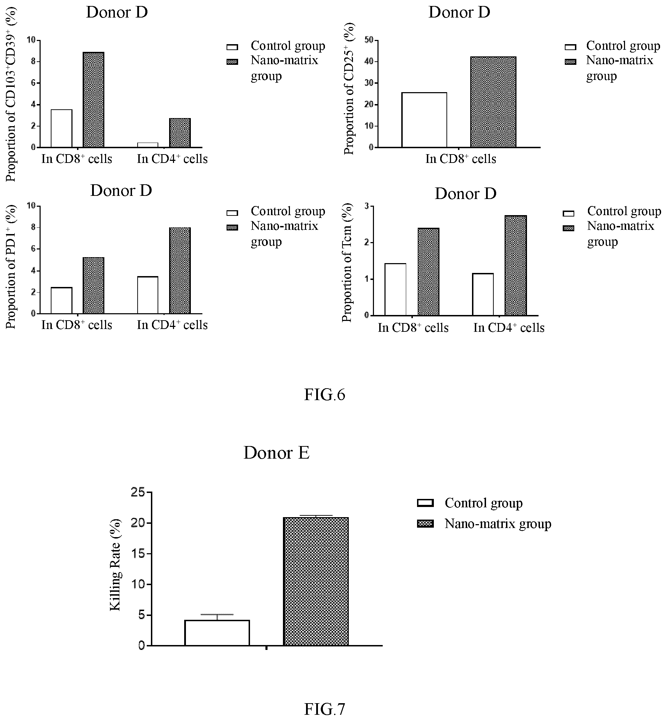

FIG. 6 shows the ratio of T cell subpopulations of the TIL cells obtained from the culture of a nano-matrix group and a control group for donor D. The results show that the TILs obtained from the second stage of in vitro expansion with the CD28 antibody added (for example, with the addition of transACT including the CD3 antibody and the CD28 antibody), compared with the control group (without the addition of the CD28 antibody), have an improved ratio of T cell subpopulations. For example, a higher ratio of tumor-specific T cells (CD103$^+$CD39$^+$), a higher ratio of activated T cells (CD25$^+$ or PD1$^+$), and/or a higher ratio of central memory T cells (Tcm, e.g., CD45RA$^-$CCR7$^+$).

**Example 4 Test of cell killing ability of the TILs**

The third TIL population obtained from the culture of the second stage of in vitro expansion for each experimental group in Example 1 was tested for its cell killing ability.

Cell preparation

The TILs obtained from each experimental group were prepared for the test and the target cells (e.g., Hela tumor cells) were prepared for the co-culture.

Test steps

Labeling the tumor cells with CFSE (5(6)-Carboxyfluorescein diacetate N-succinimidyl ester, Sigma, 21888-25MG-F): the tumor cells were washed with PBS and resuspended in 500 μL of PBS; CFSE was added to 500

μL of PBS and mixed with 500 μL resuspension of the tumor cells in PBS, to a final concentration of CFSE of 0.5 μmol/L. After incubation at 37°C for 6 minutes, a culture medium containing 10% FBS was added for washing. Centrifugation was performed at 600 g for 5 minutes. X-vivo 15 medium or other commercially available T cell culture media, e.g. T cell culture media of Stem Cell, Lonza, Thermo, Miltenyi brands, etc., were used to resuspend the tumor cells to a concentration of $1 \times 10^6$ cells/mL. The third TIL population obtained from the culture of the second stage of in vitro expansion of each experimental group was centrifuged at 600 g for 5 minutes, and the TIL cells were resuspended according to an effector-target ratio (the ratio of the TIL cells to tumor cells) of 3:1 (i.e., the concentration of the resuspended TIL cells was $3 \times 10^6$ cells/mL). Each 100 μL of the tumor cells and the TIL cells were added into a U-bottom 96-well plate (Corning), and three replicate wells were set for each group. At the same time, a control group containing only the tumor cells was set. The well plate was centrifuged at 200 g for 1 minute and incubated at 37°C for 4 hours to overnight.

After the incubation was completed, centrifugation was performed at 600 g for 3 minutes and the supernatant was discarded. Trypsin was added at 20 μL/well. Incubation was performed in an incubator at 37°C for 3-5 minutes to digest the tumor cells. After the digestion was completed, 180 μL of a culture medium containing 10% FBS was added to terminate the digestion. Dapi (Beyotime, C0060) was diluted at 1:100, and then the diluted Dapi was added at 20 μL/well. On-board flow cytometry test was performed.

$$\text{Killing rate}\% = \text{the number of Dapi}^+\text{CFSE}^+ \text{ cells/total CFSE}^+ \times 100\%.$$

FIG. 7 shows the cell killing ability of the TIL cells obtained from the culture of a nano-matrix group and a control group for donor E. The results show that the TILs obtained from the second stage of in vitro expansion with the CD28 antibody added (for example, with the addition of transACT including the CD3 antibody and the CD28 antibody), compared with the control group (without the addition of the CD28 antibody), have a higher cell killing ability.

**Example 5 Test of intracellular factor expression of the TILs**

The third TIL population obtained from the culture of the second stage of in vitro expansion for each experimental group in Example 1 was tested for its intracellular factor expression.

Experiment Preparation

Formulation of a culture medium required for the test of intracellular factor expression: T cell culture medium was taken, into which were added Golgistop (BD) at a volume ratio of 1:1000 and CD107a antibody (BD) at a volume ratio of 1:200.

Test steps

The third TIL population obtained from the culture of the second stage of in vitro expansion of each experimental group was centrifuged, and then resuspended to $5 \times 10^5$ cells/mL using 600 μL of the above culture medium required for the test of intracellular factor expression. The resuspension was then added into a 96-well plate and incubated in an incubator at 37°C overnight.

After the incubation was completed, the cells were washed once with PBS at 200 μL/well, and centrifuged at 600 g for 3 minutes, and the supernatant was discarded. An antibody-mixed working solution was formulated for staining of CD3/CD4/CD8 (BD) on the cell surface at 50 μL for each group, in which the antibody concentration was 1:100, and incubated at 2-8°C in dark for 30 minutes. After the staining was completed, the cells were washed, then fixed and permeabilized by adding the Fixation/Permeabilization Buffer (BD) at 100 μL/well, and incubated at 4°C in dark for 20 minutes. After the fixation and permeabilization were completed, $1 \times$BD Perm/Wash buffer (BD) was added for washing. The $1 \times$BD Perm/Wash buffer (BD) can be continually used to formulate the IPNγ antibody (BD) and the GZMB antibody (BD), of which the concentration was 1:100. Staining was performed at 50 μL/group, at 4°C in dark for 30 minutes. After the staining was completed, $1 \times$BD Perm/Wash buffer (BD) was added for washing, and PBS was used for resuspension, then on-board flow cytometry test was performed.

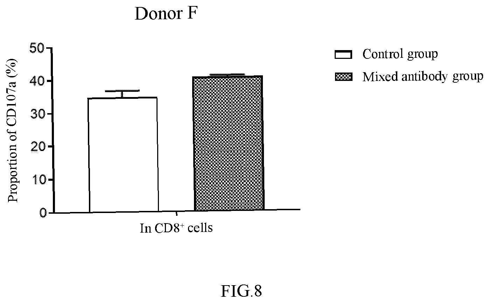

FIG. 8 shows the test results of the intracellular factor expression of the TIL cells obtained from the culture of a mixed antibody group and a control group for donor F. The results show that the TILs obtained from the second stage of in vitro expansion with the CD28 antibody added, compared with the control group (without the addition of the CD28 antibody), have a higher intracellular factor expression ability. For example, a higher CD107a expression ability.

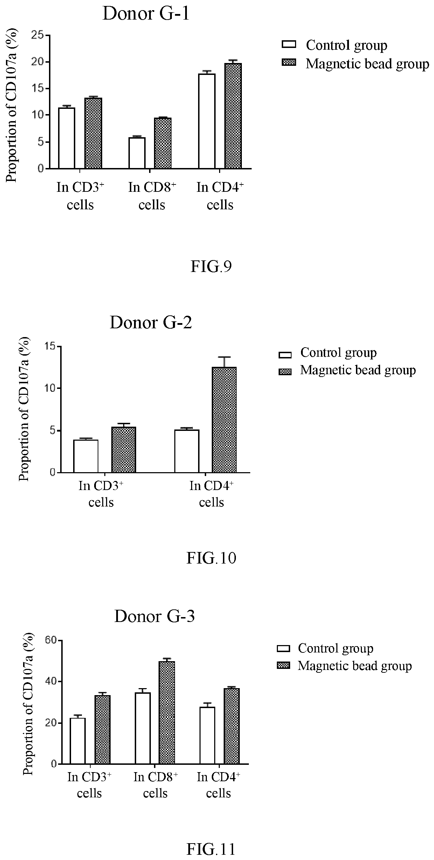

FIG. 9 shows the test results of the intracellular factor expression of the TIL cells obtained from the culture of a magnetic bead group and a control group for donor G-1. The results show that the TILs obtained from the second stage of in vitro expansion with the CD28 antibody added (for example, with the addition of magnetic beads including the CD3 antibody and the CD28 antibody), compared with the control group (without the addition of the CD28 antibody), have a higher intracellular factor expression ability. For example, a higher CD107a expression ability.

FIG. 10 shows the test results of the intracellular factor expression of the TIL cells obtained from the culture of a magnetic bead group and a control group for donor G-2. The results show that the TILs obtained from the second stage of in vitro expansion with the CD28 antibody added (for example, with the addition of magnetic beads including the CD3 antibody and the CD28 antibody), compared with the control group (without the addition of the CD28 antibody), have a higher intracellular factor expression ability. For example, a higher CD107a expression ability.

FIG. 11 shows the test results of the intracellular factor expression of the TIL cells obtained from the culture of a magnetic bead group and a control group for donor G-3. The results show that the TILs obtained from the second stage of in vitro expansion with the CD28 antibody added (for example, with the addition of magnetic beads including the CD3 antibody and the CD28 antibody), compared with the control group (without the addition of the CD28 antibody), have a higher intracellular factor expression ability. For example, a higher CD107a expression ability.

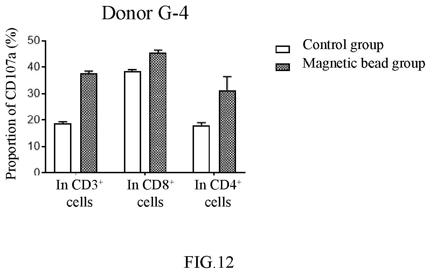

FIG. 12 shows the test results of the intracellular factor expression of the TIL cells obtained from the culture of a magnetic bead group and a control group for donor G-4. The results show that the TILs obtained from the second stage of in vitro expansion with the CD28 antibody added (for example, with the addition of magnetic beads including the CD3 antibody and the CD28 antibody), compared with the control group (without the addition of the CD28 antibody), have a higher intracellular factor expression ability. For example, a higher CD107a expression ability.

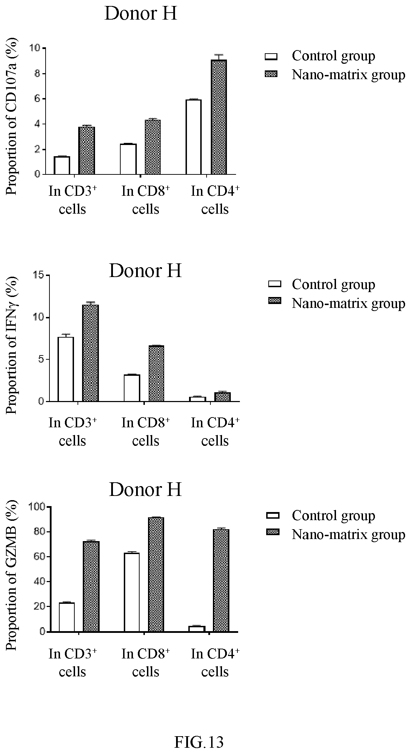

FIG. 13 shows the test results of the intracellular factor expression of the TIL cells obtained from the culture of a nano-matrix group and a control group for donor H. The results show that the TILs obtained from the second stage of in vitro expansion with the CD28 antibody added (for example, with the addition of transACT including the CD3 antibody and the CD28 antibody), compared with the control group (without the addition of the CD28 antibody), have a higher intracellular factor expression ability. For example, a higher CD107a expression ability, a higher IPN$\gamma$ expression ability, or a higher GZMB expression ability.

## Example 6 Test of cytokine secretion of the TILs

The third TIL population obtained from the culture of the second stage of in vitro expansion for each experimental group in Example 1 was tested for its cytokine secretion.

Preparation of standards

Human Th1/Th2/Th17 cytokine standard lyophilized powder (BD) was reconstituted with 2 mL of Assay Diluent (BD) (the concentration of each cytokine in the standard solution was 5000 pg/mL) and diluted in a gradient sequence of 1:2, 1:4, 1:8, 1:16, 1:32, 1:64, 1:128, 1:256, 1:512, 1:1024, then mixed with Capture Beads (BD) and PE Detection Reagent (BD), and transferred into 15 mL cone-bottom centrifuge tubes, which were labeled as "Standard Tubes". One tube containing only Assay Diluent was taken as the negative control.

Test steps

The third TIL population obtained from the culture of the second stage of in vitro expansion for each experimental group was diluted with Assay Diluent, added into well-plates at 10 $\mu$L/well, then mixed with each of the Capture Beads (BD) at 2 $\mu$L/Beads/well and mixed with PE Detection Reagent (BD) at 10 $\mu$L/well, and incubated at room temperature in dark for 3 hours.

After the incubation was completed, 200 $\mu$L of Wash Buffer (BD) was added into each well, and centrifuged at 500 g for 3 minutes. After the centrifugation was completed, 100 $\mu$L of Wash Buffer (BD) was added into each well for resuspension, thus performing the flow cytometry.

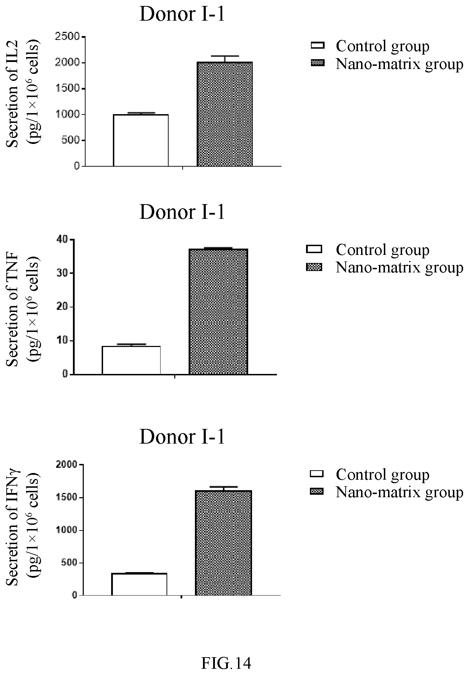

FIG. 14 shows the test results of the cytokine secretion of the TIL cells obtained from the culture of a nano-matrix group and a control group for donor I-1. The results show that the TILs obtained from the second stage of in vitro expansion with the CD28 antibody added (for example, with the addition of transACT including the CD3 antibody and the CD28 antibody), compared with the control group (without the addition of the CD28 antibody), have a higher cytokine secretion ability. For example, a higher IL-2 secretion ability, a higher TNF secretion ability, or a higher IPN$\gamma$ secretion ability.

The TILs obtained from each experimental group were incubated with tumor cells overnight by referring to the method of Example 4 (the test of cell killing ability of the TILs). After the incubation was completed, the supernatant was taken for the test of cytokine secretion according to the test steps of this embodiment.

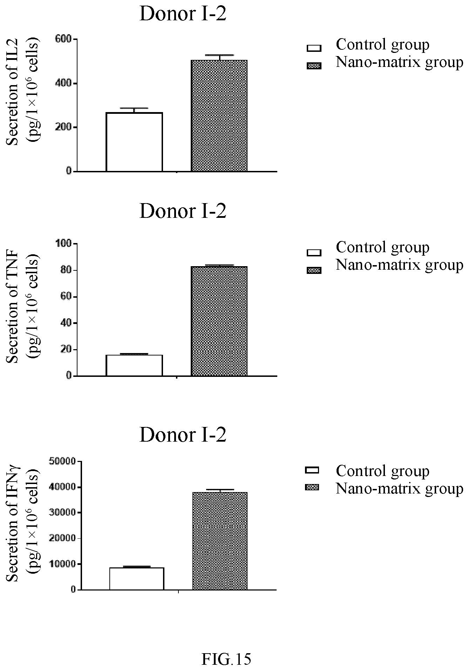

FIG. 15 shows the test results of the cytokine secretion of the TIL cells obtained from the culture of a nano-matrix group and a control group, after co-incubation with tumor cells, for donor I-2. The results show that the TILs obtained from the second stage of in vitro expansion with the CD28 antibody added (for example, with the addition of transACT including the CD3 antibody and the CD28 antibody), compared with the control group (without the addition of the CD28 antibody), have a higher cytokine secretion ability. For example, a higher IL-2 secretion ability, a higher TNF secretion ability, or a higher IPNγ secretion ability.

## Example 7 Test of gene knockout efficiency of the TILs

The TIL cells of each experimental group obtained after 48 hours of culture in the second stage of in vitro expansion in Example 1 were taken for the test of gene knockout efficiency.

sgRNA (its sequence was as set forth in SEQ ID NO: 33, GGAGAATGACGAGTGGACCC) was formulated with Nuclease-free water (Commercial source: Shanghai Youfan Biosciences Co., Ltd.; RT121-02), the concentration of which was adjusted to 50 μmol/L. 2 μL of gRNA was added into a PCR tube, with Nuclease-free water as the negative control, incubated in a PCR instrument at 95°C for 2 minutes, and then cooled at room temperature for 10 minutes.

According to the volume ratio of sgRNA:P3 Buffer:Cas9 nuclease = 2:2:1, into a PCR tube containing sgRNA were added P3 Buffer (commercial source: Lonza; V4XP-3032) and 61.7 μmol/L of Cas9 nuclease (commercial source: Suzhou Curegenetics Biotechnology Co., Ltd.; C01-2019-11-001) in turn. The PCR tube was placed in the PCR instrument, incubated at 25°C for 10 minutes to form RNP, which is kept at 4°C ready for use.

T cell culture medium was added at 1 mL/well and preheated in a $CO_2$ incubator. The TIL cells of each experimental group obtained after 48 hours of culture in the second stage of in vitro expansion in Example 1 were mixed well and then counted. $5 \times 10^5$ cells were taken from the samples of each experimental group, into which was added P3 Buffer (20 μL) to mix well. The cells were added into a new PCR tube and mixed with 5 μL of RNP. The mixture of cells and RNP was added into electroporation strips, and electroporated in an electroporator (Lonza) (human T cell stimulated (E0115)). After the electroporation procedures were completed, 180 μL of preheated T cell culture medium was immediately added, the whole volume was transferred into 24-well suspension plates and incubated in a $CO_2$ incubator. The cells were counted after 24 hours, into which were added feeder cells (irradiated PBMC cells) at a ratio of the TILs to feeder cells = 1:200, and continually incubated in the $CO_2$ incubator for 72 hours. After the incubation was completed, TIL cells of each experimental group were counted. $2 \times 10^5$ cells were taken from each experimental group, and centrifuged at 500 g for 3 minutes, the supernatant was then discarded.

Formulation of mixed antibodies for flow cytometry: Fixable Viability Dye eFluor 780 (Commercial source: eBioscience; 65-0865-18) was diluted 10000 fold in PBS. Into 100 μL of the diluted solution of Fixable Viability Dye eFluor 780 in PBS were respectively added 1 μL of TCR-aβ-APC (Commercial source: eBioscience; 17-9986-42), 1 μL of BB515 Mouse Anti-Hu CD8 (Commercial source: BD Pharmingen; 564526), and 1 μL of PE-Cy7 Mouse Anti-Hu CD4 (Commercial source: BD Pharmingen; 557852), and mixed well.

Into the TIL cell samples of each experimental group were added 100 μL of the above mixed antibodies for flow cytometry, mixed well and then incubated on ice for 30 minutes. After the incubation was completed, centrifugation was performed at 500 g for 3 minutes and the supernatant was then discarded. 200 μL of PBS was added for resuspension. The test was performed by a flow cytometer, and the TCRβ knockout efficiency was analyzed with a Flowjo software.

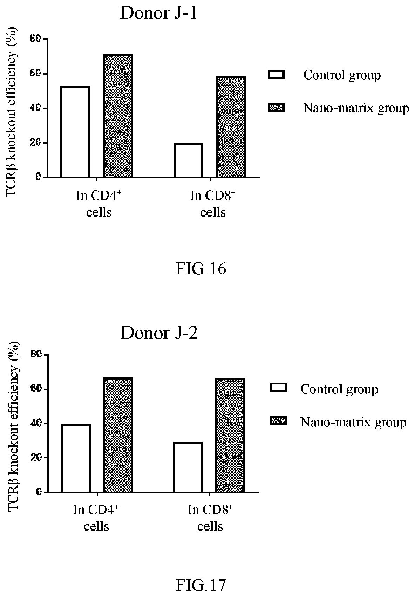

FIG. 16 shows the results of the gene knockout efficiency of the TIL cells obtained from the culture of a nano-matrix group and a control group, after co-incubation with tumor cells, for donor J-1. The results show that the TILs obtained from the second stage of in vitro expansion with the CD28 antibody added (for example, with the addition of transACT including the CD3 antibody and the CD28 antibody), compared with the control group (without the addition of the CD28 antibody), have an improved gene knockout efficiency ability. For example, an enhanced TCRβ gene knockout efficiency.

FIG. 17 shows the results of the gene knockout efficiency of the TIL cells obtained from the culture of a nano-matrix group and a control group, after co-incubation with tumor cells, for donor J-2. The results show that the TILs obtained from the second stage of in vitro expansion with the CD28 antibody added (for example, with the addition of transACT including the CD3 antibody and the CD28 antibody), compared with the control group (without the addition of the CD28 antibody), have an improved gene knockout efficiency ability. For example, an enhanced TCRβ gene knockout efficiency.

## Example 8 Test of proliferative ability of the TILs with the CD28 agonist added at the end of the REP stage

A first TIL population derived from tumor tissues and not expanded in vitro was obtained by referring to Example 1. The first TIL population was subjected to the first stage of in vitro expansion (preREP) in the same manner and the second stage of in vitro expansion (REP) in the same manner to obtain a third TIL population. The third TIL population was randomly divided into 3 groups. IL-2 was added into the T cell culture medium of each experimental group, while no T cell activators were added into the blank group. Into the group without the CD28 agonist added was added about 30 ng/mL of the CD3 antibody, and into the group with the CD28 agonist added were added the CD3 agonist and the CD28 agonist, for example, transACT was added at a ratio of transACT to TILs of about 1:100 to 1:2000. The TILs (the fourth cell population) obtained from 3 days of cultivation were tested for the proliferative ability of the TIL cells by a method for testing the cell viability using a CellTiter-Glo kit (Commercial source: Promega).

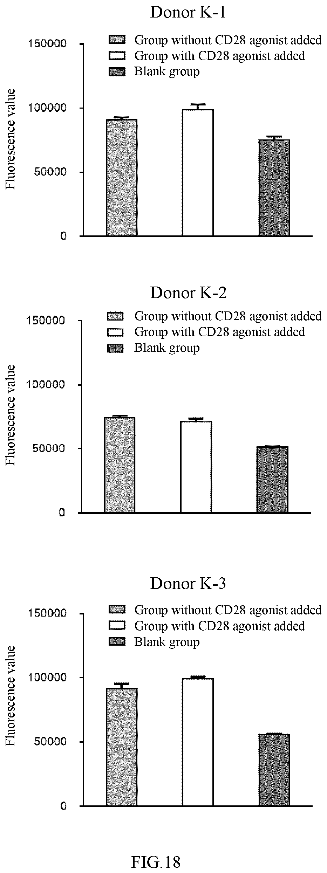

The analysis of the proliferative ability of the experimental groups that were expanded in vitro in different ways at the end of the REP stage is shown in FIG. 18. The fluorescence value of the ordinate in FIG. 18 reflects the proliferation ability of the TIL cells expanded in vitro in the third stage in different ways in each experimental group. The results show that, for donors K-1, K-2, and K-3, the proliferative ability of the TILs obtained with the CD28 agonist added in the third stage of in vitro expansion was comparable to that of the TILs obtained without the CD28 agonist added in the third stage of expansion.

**Example 9 Activation effects of different forms of CD28 agonists**

For each experimental group in Example 1 of which the culture in the second stage of in vitro expansion lasted for 7 days, 10 days or more than 10 days, the activation effects achieved by using different forms of CD28 agonists were tested. For example, the nano-sized CD28 agonists can be agonists including the CD3 antibody and the CD28 antibody, with a diameter of about 100 nm; for example, the nano-matrix A can be transACT (with a diameter of about 100 to 500 nm, Miltenyi), the nano-matrix B can be CytoSinct (with a diameter of about 100 nm, GENSCRIPT, for example, it can be added at a ratio of transACT to TILs of about 1:100 to 1:1000).

The analysis of the proliferative ability of experimental groups added with different forms of the CD28 agonists is shown in FIG. 19A. The values of the vertical coordinate in the figure indicate the expansion fold of the number of the TIL cells in the third TIL population obtained from the second stage of in vitro expansion for each experimental group, compared with that in the second TIL population before the start of the second stage of in vitro expansion. The results show that, different forms of nano-sized CD28 agonists can all have the effects of enhancing the proliferative ability of the TILs.

The analysis of the flow cytometry results of the experimental groups added with different forms of the CD28 agonists is shown in FIGs. 19B to 19L.

FIG. 19B shows that, different forms of nano-sized CD28 agonists can all have the ability of increasing the ratio of tumor-specific T cells (CD103$^+$CD39$^+$).

FIGs. 19C-19F show that, different forms of nano-sized CD28 agonists can all have the ability of reducing the ratio of exhausted T cells (CD39$^+$, PD1$^+$, LAG3$^+$, TIM3$^+$).

FIG. 19G show that, different forms of nano-sized CD28 agonists can all have the ability of reducing the ratio of regulatory T cells (Treg).

FIGs. 19H-19J show that, different forms of nano-sized CD28 agonists can all have the ability of increasing the ratio of activated T cells (CD25$^+$, 41BB$^+$, CD28$^+$).

FIG. 19K shows that, different forms of nano-sized CD28 agonists can all have the ability of increasing the ratio of stem cell-like T cells (CD69$^-$CD39$^-$).

FIG. 19L shows that, different forms of nano-sized CD28 agonists can all have the ability of increasing the ratio of central memory T cells (Tcm, e.g., CD45RO$^+$CD62L$^+$).

The test results of the intracellular factor expression of the experimental groups added with different forms of the CD28 agonists are shown in FIGs. 19M to 19N.

FIG. 19M shows the expression ratio of intracellular factors (CD 107a) as tested by flow cytometry with different forms of nano-sized CD28 agonists added in the culture of the second stage of in vitro expansion. The results show that, different forms of nano-sized CD28 agonists can all enhance the intracellular factor expression of the TIL cells.

FIG. 19N shows the expression ratio of intracellular factors (CD 107a) as tested by flow cytometry with different forms of nano-sized CD28 agonists added in the culture of the second stage of in vitro expansion, wherein each group of the TIL cells have been incubated with the CD3 antibody overnight before the flow cytometry. The results show that, different forms of nano-sized CD28 agonists can all enhance the intracellular factor expression of the TIL cells.

The test results of the cytokine secretion of the experimental groups added with different forms of the CD28 agonists are shown in FIG. 19O.

FIG. 19O shows the cytokine secretion ability of the TIL cells as verified by testing the cytokine secretion, with different forms of nano-sized CD28 agonists added in the culture of the second stage of in vitro expansion, wherein the TIL cells in the CD3 antibody stimulation group have been incubated with the CD3 antibody overnight before the cytokine detection. The results show that, different forms of nano-sized CD28 agonists can all have the ability of enhancing the cytokine secretion of the TIL cells.

The foregoing detailed description is provided in an illustrative and exemplary manner, and is not intended to limit the scope of the appended claims. Various modifications to the embodiments currently enumerated herein will be apparent to those of ordinary skill in the art, and remain within the scope of the appended claims and their equivalents.

EP 4 269 568 A1

**Claims**

1. A method for culturing tumor-infiltrating lymphocytes (TILs), comprising: subjecting TILs derived from tumor tissues and not expanded in vitro to at least one stage of in vitro expansion, and wherein the method comprises contacting the TILs with a CD28 agonist in at least one stage of the in vitro expansion.

2. The method according to claim 1, wherein the method comprises subjecting the TILs derived from tumor tissues and not expanded in vitro to a first stage of in vitro expansion and a second stage of in vitro expansion, and wherein in the second stage of in vitro expansion the method comprises contacting the TILs having subjected to the first stage of in vitro expansion with the CD28 agonist.

3. The method according to any one of claims 1 to 2, wherein the CD28 agonist comprises an anti-CD28 antibody and/or an antigen-binding fragment thereof.

4. The method according to any one of claims 2 to 3, wherein the second stage of in vitro expansion is carried out for at most about 13 days.

5. The method according to any one of claims 2 to 4, wherein the second stage of in vitro expansion is carried out for about 3 days to about 13 days.

6. The method according to any one of claims 1 to 5, wherein the method comprises co-culturing the TILs with feeder cells in at least one stage of the in vitro expansion.

7. The method according to claim 6, wherein the method comprises contacting the TILs with the CD28 agonist and co-culturing the TILs with the feeder cells in a single stage of the in vitro expansion.

8. The method according to any one of claims 6 to 7, wherein the method comprises contacting the TILs with the CD28 agonist for a certain period and then co-culturing the TILs with the feeder cells in a single stage of the in vitro expansion.

9. The method according to claim 8, wherein the certain period is at least about 2 hours.

10. The method according to any one of claims 8 to 9, wherein the certain period is about 6 hours to about 72 hours.

11. The method according to any one of claims 8 to 10, wherein the certain period is about 12 hours to about 48 hours.

12. The method according to any one of claims 8 to 10, wherein the certain period is about 6 hours, about 12 hours, about 24 hours, about 48 hours, or about 72 hours.

13. The method according to any one of claims 6 to 12, wherein the feeder cells comprise antigen-presenting cells.

14. The method according to any one of claims 6 to 13, wherein the feeder cells comprise one or more of the cells selected from the group consisting of peripheral mononuclear cells, dendritic cells, and artificial antigen-presenting cells.

15. The method according to any one of claims 6 to 14, wherein the feeder cells are peripheral mononuclear cells.

16. The method according to any one of claims 6 to 15, wherein the feeder cells are irradiated feeder cells.

17. The method according to any one of claims 6 to 16, wherein the co-culture of the TILs with the feeder cells comprises contacting the surfaces of the feeder cells with the surface of the TILs.

18. The method according to any one of claims 6 to 17, wherein the co-culture of the TILs with the feeder cells comprises adding the feeder cells into the cell culture medium of the TILs.

19. The method according to any one of claims 6 to 18, wherein the method comprises adding the feeder cells into the cell culture medium of the TILs at a proportion of the feeder cells to the TILs from about 40:1 to about 400:1.

20. The method according to any one of claims 1 to 19, wherein the method further comprises contacting the TILs with one or more T cell growth factors in at least one stage of the in vitro expansion.

21. The method according to claim 20, wherein the method comprises contacting the TILs with the CD28 agonist and the one or more T cell growth factors in a single stage of the in vitro expansion.

22. The method according to any one of claims 20 to 21, wherein the method comprises contacting the TILs with the CD28 agonist and the one or more T cell growth factors substantially at the same time in a single stage of the in vitro expansion.

23. The method according to any one of claims 20 to 22, wherein the T cell growth factors are one or more of the factors selected from the group consisting of IL-2, IL-7, IL-12, IL-15, IL-21, interferon gamma, and functionally active fragments thereof.

24. The method according to any one of claims 20 to 23, wherein the T cell growth factors comprise IL-2 and/or functionally active fragments thereof.

25. The method according to any one of claims 20 to 24, wherein the contact of the TILs with the one or more T cell growth factors comprises adding the T cell growth factors into the cell culture medium of the TILs.

26. The method according to any one of claims 20 to 25, wherein the initial concentration of the T cell growth factors in the cell culture medium of the TILs is at least about 300 IU/mL.

27. The method according to any one of claims 1 to 26, wherein the TILs that have been contacted with the CD28 agonist in at least one stage of the in vitro expansion show improved expansion effects, compared to the corresponding TILs that have not been contacted with the CD28 agonist in the in vitro expansion stage.

28. The method according to claim 27, wherein the improved expansion effects comprise one or more of the properties selected from the group consisting of an increased number of TIL cells, an improved proportion of T cell subpopulations, an enhanced cytokine secretion ability, and an enhanced tumor cell killing ability.

29. The method according to claim 28, wherein the improved proportion of T cell subpopulations comprises one or more of the properties selected from the group consisting of an increased proportion of central memory T cells, a reduced proportion of regulatory T cells, an increased proportion of activated T cells, an increased proportion of tumor-specific T cells, and an increased proportion of stem cell-like T cells.

30. The method according to any one of claims 1 to 29, wherein the TILs that have been contacted with the CD28 agonist in at least one stage of the in vitro expansion show an improved gene editing effect, compared to the TILs that have not been contacted with the CD28 agonist in the in vitro expansion stage.

31. The method according to claim 30, wherein the improved gene editing effect comprises an enhanced gene knockout efficiency.

32. The method according to any one of claims 1 to 31, wherein the method further comprises contacting the TILs with one or more other T cell activators other than the CD28 agonist in at least one stage of the in vitro expansion.

33. The method according to claim 32, wherein the method comprises contacting the TILs with the CD28 agonist and the one or more other T cell activators in a single stage of the in vitro expansion.

34. The method according to any one of claims 32 to 33, wherein the method comprises contacting the TILs with the CD28 agonist and the one or more other T cell activators substantially at the same time in a single stage of the in vitro expansion.

35. The method according to any one of claims 32 to 34, wherein the other T cell activators comprise one or more of the molecules selected from the group consisting of CD80, CD86, B7-H3, 4-1BBL, CD27, CD30, CD134, B7h, CD40, LIGHT, and functionally active fragments thereof.

36. The method according to any one of claims 32 to 35, wherein the other T cell activators comprise agonists of one or more of the targets selected from the group consisting of CD3, HVEM, CD40L, OX40, and 4-1BB.

37. The method according to any one of claims 32 to 36, wherein the other T cell activators comprise a CD3 agonist.

38. The method according to any one of claims 32 to 37, wherein the other T cell activators comprise an anti-CD3 antibody and/or an antigen-binding fragment thereof.

39. The method according to any one of claims 32 to 38, wherein the contact of the TILs with the CD28 agonist and the one or more other T cell activators comprises one or more of the means selected from the group consisting of (1) adding the CD28 agonist and the other T cell activators into the cell culture medium of the TILs; (2) adding engineering cells expressing the CD28 agonist and the other T cell activators into the cell culture medium of the TILs; (3) adding a solid-phase medium comprising the CD28 agonist and the other T cell activators into the cell culture medium of the TILs.

40. The method according to any one of claims 32 to 39, wherein the initial concentration of the other T cell activators in the cell culture medium of the TILs is at least about 30 ng/mL.

41. The method according to any one of claims 32 to 40, wherein the initial concentration of the other T cell activators in the cell culture medium of the TILs is about 30 ng/mL to about 300 ng/mL.

42. The method according to any one of claims 39 to 41, wherein the diameter of the solid-phase medium is about 500 nm to about 10 $\mu$m.

43. The method according to any one of claims 39 to 41, wherein the diameter of the solid-phase medium is about 1 nm to about 500 nm.

44. The method according to any one of claims 42 to 43, wherein the diameter of the solid-phase medium is measured by a transmission electron microscope.

45. The method according to any one of claims 39 to 44, wherein the solid-phase medium comprises a polymer.

46. The method according to any one of claims 39 to 45, wherein the solid-phase medium comprises at least about 25 $\mu$g of the CD28 agonist and the other T cell activators per mg.

47. The method according to any one of claims 39 to 46, wherein the method comprises adding the solid-phase medium comprising the CD28 agonist and the other T cell activators into the cell culture medium of the TILs at a proportion of the solid-phase medium to the TILs from about 2:1 to about 1:2.

48. The method according to any one of claims 39 to 46, wherein the method comprises adding the solid-phase medium comprising the CD28 agonist and the other T cell activators into the cell culture medium of the TILs at a proportion of the solid-phase medium to the TILs from about 1:100 to about 1:2000.

49. The method according to any one of claims 1 to 48, wherein the TILs derived from tumor tissues and not expanded in vitro are TILs derived from fragments of the tumor tissues.

50. The method according to claim 49, wherein the fragments have a volume of about 1 mm$^3$ to about 27 mm$^3$.

51. A method for culturing tumor-infiltrating lymphocytes (TILs), comprising:

(A) contacting a first TIL population derived from tumor tissues and not expanded in vitro with one or more T cell growth factors; wherein, a second TIL cell population is obtained through step (A);
(B) contacting the second TIL population with a CD28 agonist.

52. The method according to claim 51, wherein the CD28 agonist comprises an anti-CD28 antibody and/or an antigen-binding fragment thereof.

53. The method according to any one of claims 51 to 52, wherein the step (B) is carried out for at most about 13 days.

54. The method according to any one of claims 51 to 53, wherein the step (B) is carried out for about 3 days to about 13 days.

55. The method according to any one of claims 51 to 54, wherein the method further comprises co-culturing the TILs with feeder cells in the step (A) and/or the step (B).

56. The method according to claim 55, wherein the method comprises co-culturing the second TIL population with the feeder cells in the step (B).

57. The method according to any one of claims 55 to 56, wherein the method comprises in the step (B), contacting the second TIL population with the CD28 agonist for a certain period, and then co-culturing said TIL population with the feeder cells.

58. The method according to claim 57, wherein the certain period is at least about 2 hours.

59. The method according to any one of claims 57 to 58, wherein the certain period is about 6 hours to about 72 hours.

60. The method according to any one of claims 57 to 59, wherein the certain period is about 12 hours to about 48 hours.

61. The method according to any one of claims 57 to 59, wherein the certain period is about 6 hours, about 12 hours, about 24 hours, about 48 hours, or about 72 hours.

62. The method according to any one of claims 55 to 61, wherein the feeder cells comprise antigen-presenting cells.

63. The method according to any one of claims 55 to 62, wherein the feeder cells comprise one or more of the cells selected from the group consisting of peripheral mononuclear cells, dendritic cells, and artificial antigen-presenting cells.

64. The method according to any one of claims 55 to 63, wherein the feeder cells are peripheral mononuclear cells.

65. The method according to any one of claims 55 to 64, wherein the feeder cells are irradiated feeder cells.

66. The method according to any one of claims 55 to 65, wherein the co-culture of the TILs with the feeder cells comprises contacting the surface of the feeder cells with the surface of the TILs.

67. The method according to any one of claims 55 to 66, wherein the co-culture of the TILs with the feeder cells comprises adding the feeder cells into the cell culture medium of the TILs.

68. The method according to any one of claims 55 to 67, wherein the method comprises adding the feeder cells into the cell culture medium of the TILs at a proportion of the feeder cells to the TILs from about 40:1 to about 400:1.

69. The method according to any one of claims 51 to 68, wherein the method further comprises contacting the TILs with one or more T cell growth factors in the step (A) and/or the step (B).

70. The method according to claim 69, wherein the method comprises contacting the second TIL population with the one or more T cell growth factors in the step (B).

71. The method according to any one of claims 69 to 70, wherein the method comprises contacting the second TIL population with the CD28 agonist and the one or more T cell growth factors substantially at the same time in the step (B).

72. The method according to any one of claims 69 to 71, wherein the T cell growth factors are one or more of the factors selected from the group consisting of IL-2, IL-7, IL-12, IL-15, IL-21, interferon gamma, and functionally active fragments thereof.

73. The method according to any one of claims 69 to 72, wherein the T cell growth factors comprise IL-2 and/or functionally active fragments thereof.

74. The method according to any one of claims 69 to 73, wherein the contact of the TILs with the one or more T cell growth factors comprises adding the T cell growth factors into the cell culture medium of the TILs.

75. The method according to any one of claims 69 to 74, wherein the initial concentration of the T cell growth factors in the cell culture medium of the TILs is at least about 300 IU/mL.

76. The method according to any one of claims 51 to 75, wherein the TILs that have been contacted with the CD28 agonist in the step (B) show improved expansion effects, compared to the corresponding TILs that have not been contacted with the CD28 agonist in the step (B).

77. The method according to claim 76, wherein the improved expansion effects comprise one or more of the properties selected from the group consisting of an increased number of TIL cells, an improved proportion of T cell subpopulations, an enhanced cytokine secretion ability, and an enhanced tumor cell killing ability.

78. The method according to claim 77, wherein the improved proportion of T cell subpopulations comprises one or more of the properties selected from the group consisting of an increased proportion of central memory T cells, a reduced proportion of regulatory T cells, an increased proportion of activated T cells, an increased proportion of tumor-specific T cells, and an increased proportion of stem cell-like T cells.

79. The method according to any one of claims 51 to 78, wherein the TILs that have been contacted with the CD28 agonist in the step (B) show an improved gene editing effect, compared to the corresponding TILs that have not been contacted with the CD28 agonist in the step (B).

80. The method according to claim 79, wherein the improved gene editing effect comprises an enhanced gene knockout efficiency.

81. The method according to any one of claims 51 to 80, wherein the method further comprises contacting the TILs with one or more other T cell activators other than the CD28 agonist in the step (A) and/or the step (B).

82. The method according to claim 81, wherein the method comprises contacting the second TIL population with the CD28 agonist and the one or more other T cell activators in the step (B).

83. The method according to any one of claims 81 to 82, wherein the method comprises contacting the second TIL population with the CD28 agonist and the one or more other T cell activators substantially at the same time in the step (B).

84. The method according to any one of claims 81 to 83, wherein the other T cell activators comprise one or more of the molecules selected from the group consisting of CD80, CD86, B7-H3, 4-1BBL, CD27, CD30, CD134, B7h, CD40, LIGHT, and functionally active fragments thereof.

85. The method according to any one of claims 81 to 84, wherein the other T cell activators comprise agonists of one or more of the targets selected from the group consisting of CD3, HVEM, CD40L, OX40, and 4-1BB.

86. The method according to any one of claims 81 to 85, wherein the other T cell activators comprise a CD3 agonist.

87. The method according to any one of claims 81 to 86, wherein the other T cell activators comprise an anti-CD3 antibody and/or an antigen-binding fragment thereof.

88. The method according to any one of claims 81 to 87, wherein the contact of the TILs with the CD28 agonist and with the one or more other T cell activators comprises one or more of the means selected from the group consisting of (1) adding the CD28 agonist and the other T cell activators into the cell culture medium of the TILs; (2) adding engineering cells expressing the CD28 agonist and the other T cell activators into the cell culture medium of the TILs; (3) adding a solid-phase medium comprising the CD28 agonist and the other T cell activators into the cell culture medium of the TILs.

89. The method according to any one of claims 81 to 88, wherein the initial concentration of the other T cell activators in the cell culture medium of the TILs is at least about 30 ng/mL.

90. The method according to any one of claims 81 to 89, wherein the initial concentration of the other T cell activators in the cell culture medium of the TILs is about 30 ng/mL to about 300 ng/mL.

91. The method according to any one of claims 88 to 90, wherein the diameter of the solid-phase medium is about 500 nm to about 10 $\mu$m.

92. The method according to any one of claims 88 to 90, wherein the diameter of the solid-phase medium is about 1 nm to about 500 nm.

93. The method according to any one of claims 91 to 92, wherein the diameter of the solid-phase medium is measured by a transmission electron microscope.

94. The method according to any one of claims 88 to 93, wherein the solid-phase medium comprises a polymer.

95. The method according to any one of claims 88 to 94, wherein the solid-phase medium comprises at least about 25 $\mu$g of the CD28 agonist and the other T cell activators per mg.

96. The method according to any one of claims 88 to 95, wherein the method comprises adding the solid-phase medium comprising the CD28 agonist and the other T cell activators into the cell culture medium of the TILs at a proportion of the solid-phase medium to the TILs from about 2:1 to about 1:2.

97. The method according to any one of claims 88 to 95, wherein the method comprises adding the solid-phase medium comprising the CD28 agonist and the other T cell activators into the cell culture medium of the TILs at a proportion of the solid-phase medium to the TILs from about 1:100 to about 1:2000.

98. The method according to any one of claims 51 to 97, wherein the TILs derived from tumor tissues and not expanded in vitro are TILs derived from fragments of the tumor tissues.

99. The method according to claim 98, wherein the fragments have a volume of about 1 mm$^3$ to about 27 mm$^3$.

100. A tumor-infiltrating lymphocyte (TIL), wherein the TIL is obtainable by the method of any one of claims 1 to 99.

101. A composition, comprising the TIL of claim 100.

102. A pharmaceutical composition, comprising the TIL of claim 100 and/or the composition of claim 101, and optionally a pharmaceutically acceptable carrier.

103. A method for affecting tumor cell growth, comprising administering to a subject the TIL of claim 100 and/or the pharmaceutical composition of claim 102.

104. Use of the TIL as defined in claim 100 and/or the pharmaceutical composition as defined in claim 102 for the manufacture of a medicament for preventing and/or treating tumors.

105. The use according to claim 104, wherein the tumors are solid tumors.

106. The use according to any one of claims 104 to 105, wherein the tumors are one or more of the tumors selected from the group consisting of melanoma, ovarian cancer, cervical cancer, lung cancer, bladder cancer, breast cancer, head and neck cancer, pancreatic cancer, liver cancer, stomach cancer, colorectal cancer, and kidney cancer.

Donor A

FIG.1

FIG.2

## Donor B-2

☐ Control group
▨ Mixed antibody group

## Donor B-2

☐ Control group
▨ Mixed antibody group

## Donor B-2

☐ Control group
▨ Mixed antibody group

FIG.3

FIG.4

FIG.5

FIG.6

FIG.7

Donor F

FIG.8

FIG.9

FIG.10

FIG.11

Donor G-4

FIG.12

FIG.13

FIG.14

FIG.15

FIG.16

FIG.17

FIG.18

FIG.19A

FIG.19B

Donor 713

Donor 713

Donor 001

FIG.19C

Donor 713

Donor 001

FIG.19D

Donor 001

Donor 001

Donor 713

FIG.19E

Donor 713

Donor 001

FIG.19F

Donor 001

FIG.19G

FIG.19H

FIG.19I

FIG.19J

FIG.19K

FIG.19L

FIG.19M

Donor 001

Donor 713

FIG.19N

Donor 001

Donor 001

FIG.19O

## INTERNATIONAL SEARCH REPORT

| International application No. |
|---|
| **PCT/CN2021/140841** |

**A. CLASSIFICATION OF SUBJECT MATTER**

C12N 5/0783(2010.01)i; A61K 35/17(2015.01)i; C12N 5/09(2010.01)i; A61P 35/00(2006.01)i

According to International Patent Classification (IPC) or to both national classification and IPC

**B. FIELDS SEARCHED**

Minimum documentation searched (classification system followed by classification symbols)

C12N; A61K; A61P

Documentation searched other than minimum documentation to the extent that such documents are included in the fields searched

Electronic data base consulted during the international search (name of data base and, where practicable, search terms used)

CNABS, VEN, CNKI, CNTXT, USTXT, EPTXT, PUBMED, ISI WEB, EMBL-EBI, STNext: 苏州沙砾生物科技有限公司, 上海沙砾生物科技有限公司, 珠海拓域生物科技有限公司, 珠海沙砾生物科技有限公司, 刘雅容, 赵佩佩, 肿瘤浸润淋巴 细胞, tumor infiltrating lymphocytes, TIL, CD28, 激动剂, 抗体, antibody, 饲养细胞, feeder cell, IL-2, 培养基, culture, medium

**C. DOCUMENTS CONSIDERED TO BE RELEVANT**

| Category* | Citation of document, with indication, where appropriate, of the relevant passages | Relevant to claim No. |
|---|---|---|
| X | CN 111801415 A (LUDWIG INSTITUTE FOR CANCER RESEARCH LTD) 20 October 2020 (2020-10-20) claims 1-124, and description, paragraph 111 | 1-106 |
| X | WO 2020172202 A1 (MYST THERAPEUTICS, INC.) 27 August 2020 (2020-08-27) claims 1-112 | 1-106 |
| X | ZOCCHI, M. R. et al. "Signalling in Human Tumour Infiltrating Lymphocytes: the CD28 Molecule is Functional and is Physically Associated with the CD45R0 Molecule" *EUROPEAN JOURNAL OF CANCER*, Vol. 28A, No. 4/5, 31 May 1992 (1992-05-31), pp. 749-754 | 1-106 |
| X | BALDAN, V. et al. "Efficient and reproducible generation of tumour-infiltrating lymphocytes for renal cell carcinoma" *BRITISH JOURNAL OF CANCER*, Vol. 112, 17 March 2015 (2015-03-17), pp. 1510-1518 | 1-106 |

☑ Further documents are listed in the continuation of Box C.  ☑ See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 March 2022** | **23 March 2022** |

| Name and mailing address of the ISA/CN | Authorized officer |
|---|---|
| **China National Intellectual Property Administration (ISA/ CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2021/140841**

**C. DOCUMENTS CONSIDERED TO BE RELEVANT**

| Category* | Citation of document, with indication, where appropriate, of the relevant passages | Relevant to claim No. |
|---|---|---|
| X | 江珊珊等 (JIANG, Shanshan et al.). "肝癌肿瘤浸润淋巴细胞(TIL)体外扩增及特性研究 (In Vitro Culturing and Characteristics of Tumor-infiltrating Lymphocyte of Hepatocellular Carcinoma)" 中国细胞生物学学报 (Chinese Journal of Cell Biology), Vol. 36, No. 7, 31 July 2014 (2014-07-31), pp. 970-975 | 1-106 |
| A | WO 2020232029 A1 (IOVANCE BIOTHERAPEUTICS, INC.) 19 November 2020 (2020-11-19) entire document | 1-106 |
| A | US 2017246277 A1 (THE JOHNS HOPKINS UNIVERSITY) 31 August 2017 (2017-08-31) entire document | 1-106 |
| A | SANTOIEMMA, P. P. et al. "Tumor infiltrating lymphocytes in ovarian cancer" CANCER BIOLOGY & THERAPY, Vol. 16, No. 6, 29 May 2015 (2015-05-29), pp. 807-820 | 1-106 |

## INTERNATIONAL SEARCH REPORT

| International application No. |
|---|
| **PCT/CN2021/140841** |

**Box No. I      Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

**INTERNATIONAL SEARCH REPORT**

International application No.

**PCT/CN2021/140841**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **103**
   because they relate to subject matter not required to be searched by this Authority, namely:

   [1] The subject matter of claim 10 relates to a disease treatment method, and thus said claim does not comply with PCT Rule 39.1(iv). The present report is formed on the basis of amending the subject matter to be a pharmaceutical use.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2021/140841**

| Patent document cited in search report | | | Publication date (day/month/year) | Patent family member(s) | | | Publication date (day/month/year) |
|---|---|---|---|---|---|---|---|
| CN | 111801415 | A | 20 October 2020 | AU | 2018361561 | A1 | 07 May 2020 |
| | | | | WO | 2019086711 | A1 | 09 May 2019 |
| | | | | JP | 2021501596 | A | 21 January 2021 |
| | | | | CA | 3081479 | A1 | 09 May 2019 |
| | | | | US | 2020338125 | A1 | 29 October 2020 |
| | | | | EP | 3707245 | A1 | 16 September 2020 |
| WO | 2020172202 | A1 | 27 August 2020 | CN | 113710270 | A | 26 November 2021 |
| | | | | EP | 3927370 | A1 | 29 December 2021 |
| | | | | KR | 20210152464 | A | 15 December 2021 |
| | | | | CA | 3130578 | A1 | 27 August 2020 |
| | | | | IL | 285631 | D0 | 30 September 2021 |
| | | | | AU | 2020226498 | A1 | 14 October 2021 |
| WO | 2020232029 | A1 | 19 November 2020 | None | | | |
| US | 2017246277 | A1 | 31 August 2017 | CA | 3017170 | A1 | 24 March 2016 |
| | | | | CA | 3017170 | C | 23 March 2021 |
| | | | | AU | 2019202508 | A1 | 02 May 2019 |
| | | | | AU | 2019202508 | B2 | 01 July 2021 |
| | | | | CA | 2961749 | A1 | 24 March 2016 |
| | | | | CA | 2961749 | C | 12 January 2021 |
| | | | | IL | 251261 | D0 | 29 May 2017 |
| | | | | IL | 251261 | A | 31 August 2020 |
| | | | | AU | 2015317712 | A1 | 27 April 2017 |
| | | | | AU | 2015317712 | B2 | 17 January 2019 |
| | | | | KR | 20210032011 | A | 23 March 2021 |
| | | | | JP | 2017529080 | A | 05 October 2017 |
| | | | | SG | 11201702191 Y | A | 27 April 2017 |
| | | | | KR | 20200055808 | A | 21 May 2020 |
| | | | | KR | 102229421 | B1 | 19 March 2021 |
| | | | | CN | 107002038 | A | 01 August 2017 |
| | | | | JP | 2021006051 | A | 21 January 2021 |
| | | | | US | 2021252121 | A1 | 19 August 2021 |
| | | | | WO | 2016044530 | A1 | 24 March 2016 |
| | | | | KR | 20170078619 | A | 07 July 2017 |
| | | | | MX | 2017003625 | A | 11 October 2017 |
| | | | | EP | 3194577 | A1 | 26 July 2017 |
| | | | | EP | 3194577 | A4 | 04 April 2018 |
| | | | | US | 2018043003 | A1 | 15 February 2018 |
| | | | | US | 10098939 | B2 | 16 October 2018 |
| | | | | US | 10987412 | B2 | 27 April 2021 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SAMBROOK et al.** Molecular Cloning: A Laboratory Manual. Cold Spring Harbor Laboratory Press, 1989
- **KABAT E.A. et al.** *Sequences of proteins of immunological interest.,* 1991
- **CHOTHIA et al.** Canonical Structures For the Hypervariable Regions of Immunoglobulins. *J. Mol. Biol.,* 1987, vol. 196, 901
- **MACCALLUM et al.** Antibody-Antigen Interactions: Contact Analysis and Binding Site Topography. *J. Mol. Biol.,* 1996, vol. 262, 732